# EUROPEAN PATENT APPLICATION

(11) **EP 4 596 700 A1**
(43) Date of publication of application: **06.08.2025**
(21) Application number: 23871001.6
(22) Date of filing: 28.09.2023
(51) Int. Cl.: C12N 15/867, C07K 16/22

(54) **NUCLEIC ACID CONSTRUCT AND USE THEREOF**

(30) Priority: 29.09.2022 CN 202211201733
(71) Applicant: Kanglin Biotech (Hangzhou) Co., Ltd., Hangzhou, Zhejiang 310018 (CN)
(72) Inventor: WU, Haoquan, Hangzhou, Zhejiang 310018 (CN); CHEN, Mengjiao, Hangzhou, Zhejiang 310018 (CN); SU, Lingling, Hangzhou, Zhejiang 310018 (CN)
(74) Representative: Maiwald GmbH
(86) International application number: PCT/CN2023/122450
(87) International publication number: WO 2024/067776

(57) **Abstract**

Provided herein are a nucleic acid construct encoding an anti-VEGF fusion protein, such as an expression vector, as well as recombinant virus such as a recombinant lentivirus and a recombinant adeno-associated virus prepared by the construct. Also provided herein are uses of the fusion protein, the nucleic acid construct, and the recombinant virus in the treatment of age-related macular degeneration (AMD), such as wet AMD.

## Description

This application claims priority to Chinese Patent Application No. 202211201733.2, filed on September 29, 2022, the entire contents of which are incorporated herein by reference.

### Technical Field

The present application relates to the field of gene therapy technology. Specifically related are nucleic acid constructs for age-related macular degeneration (AMD) such as wet AMD.

### Background of the Invention

Age-related macular degeneration (AMD), also known as age-associated macular degeneration, is an age-related, progressive, and irreversible central vision loss disease and is the leading cause of blindness among people over 60 years old worldwide. Advanced AMD is clinically classified into two primary categories: dry and wet. Among them, dry AMD is mainly characterized by the deposition of subchoroidal drusen and the formation of geographic atrophy. Wet AMD is characterized by choroidal neovascularization (CNV) and subretinal cavity exudation. Neovascular AMD (nAMD), which accounts for only 10% of all AMD, but the number of patients blinded by AMD accounts for 90% of all patients blinded by AMD.

Vascular endothelial growth factor (VEGF) is a key factor which regulates angiogenesis and vascular leakage and plays an important role in the formation of choroidal neovascularization. VEGF belongs to a member of the platelet-derived growth factor (PDGF) superfamily. VEGF family members include VEGF-A, VEGF-B, VEGF-C, VEGF-D and placental-like growth factor (PLGF). Among them, VEGF-A promotes the activation of a large number of downstream proteins after binding to its receptor VEGFR-2, leading to the growth, differentiation and proliferation of vascular endothelial cells, as well as an increase in vascular permeability. In addition, VEGF-A is also an important inflammatory cell chemokine. Pathologic expression of VEGF has now been shown to be one of the important pathogenic mechanisms of nAMD.

A large number of clinical trials and practices have proved that intravitreal injection of anti-VEGF drugs can effectively stabilize and improve patients' vision, with remarkable short-term efficacy, and it becomes the first-line treatment regimen for nAMD patients. Current anti-VEGF therapeutic drugs include ranibizumab (Genentech, Inc.), Eylea (aflibercept; Regeneron Pharmaceuticals, Inc.), Beovu (brolucizumab; Novartis, Inc.) and Compexip (Conbercept; Chengdu Kanghong Biotechnology Co., Ltd.) etc.

However, there are still some practical limitations to anti-VEGF therapy, including the high cost of drugs, the need for frequent intravitreal injections, the possibility of complications, incomplete responses and/or the inability of some patients to maintain a response. Therefore, the search for more effective and durable treatments plays an important role in reducing the burden of treatment on society and individuals.

In addition, most of the anti-VEGF drugs currently commercially available or in the clinical trial stage bind VEGF at a ratio of 1:1 or even 2:1, and their therapeutic effects are limited. Therefore, there is an urgent need to develop a more durable anti-VEGF drug that can bind VEGF at a higher binding ratio.

### Summary of the Invention

To address the above problems, the present application achieves continuous expression of high levels of anti-VEGF molecules in vision cells by comprising nucleotide sequences encoding fused multiple anti-VEGF molecules in same expression vector, and then transducing viral particles comprising the expression vector into human vision cells. The technical solutions of the present application is able to block VEGF/VEGFR binding more efficiently, thereby effectively treating age-related macular degeneration.

Accordingly, in the first aspect, the present application provides a nucleic acid construct such as an expression vectorwhich comprises one or more nucleotide sequences encoding a VEGF-binding region, such as a first nucleotide sequence encoding a first VEGF-binding region and/or a second nucleotide sequence encoding a second VEGF-binding region, wherein the first VEGF-binding region and/or the second VEGF-binding region are each independently selected from an antigen-binding fragment derived from an anti-VEGF antibody and a VEGF-binding fragment derived from a VEGF receptor (VEGFR) which comprises domain 2 and domain 3 (D2/3) of the VEGFR.

The present application provides a nucleic acid construct such as an expression vector which comprises the first nucleotide sequence encoding the first VEGF-binding region and/or the second nucleotide sequence encoding the second VEGF-binding region, wherein the first VEGF-binding region and the second VEGF-binding region are each independently selected from an antigen-binding fragment derived from an anti-VEGF antibody and a VEGF-binding fragment derived from a VEGF receptor (VEGFR) which comprises domain 2 and domain 3 (D2/3) of the VEGFR.

In some embodiments, the antigen-binding fragment derived from the anti-VEGF antibody may be selected from scFv, Fab, Fab', F(ab')2, Fd, Fd', Fv, ds-scFv and dAb.

In some embodiments, the first nucleotide sequence and the second nucleotide sequence are connected by a nucleotide sequence encoding a linker.

In some embodiments, the first VEGF-binding region is the antigen-binding fragment derived from the anti-VEGF antibody (e.g., scFv), and the second VEGF-binding region is an antigen-binding fragment derived from another different anti-VEGF antibody (e.g., scFv).

In some embodiments, the first VEGF-binding region is the antigen-binding fragment derived from the anti-VEGF antibody (e.g., scFv), and the second VEGF-binding region is the VEGF-binding fragment derived from the VEGFR.

In some embodiments of the above-described nucleic acid constructs, the nucleic acid construct further comprises the third nucleotide sequence encoding the third VEGF-binding region, said third VEGF-binding region is selected from the antigen-binding fragment derived from the anti-VEGF antibody (e.g., scFv) and/or the VEGF-binding fragment derived from the VEGFR which comprises the D2/3 of the VEGFR.

In some preferred embodiments, the first nucleotide sequence, the second nucleotide sequence and the third nucleotide sequence are connected by a nucleotide sequence encoding a linker.

In some embodiments, the first VEGF-binding region is the antigen-binding fragment derived from the anti-VEGF antibody (e.g., scFv), the second VEGF-binding region is an antigen-binding fragment derived from another different anti-VEGF antibody (e.g., scFv), and the third VEGF-binding region is the VEGF-binding fragment derived from the VEGFR.

In some embodiments, the nucleic acid construct of the present application comprises, from the 5' end to the 3' end, the following structures:
(1) scFv1;
(2) D2/3;
(3) D2/3-linker-scFv1;
(4) scFv1-linker-scFv2;
(5) scFv1-linker-D2/3;
(6) scFv1-linker-scFv2-linker-D2/3;
(7) scFv1-linker-D2/3-linker-scFv2; or
(8) D2/3-linker-scFv1-linker-scFv2,
wherein the scFv1 is a nucleotide sequence encoding the scFv derived from the anti-VEGF antibody, the scFv2 is a nucleotide sequence encoding the scFv derived from another different anti-VEGF antibody, and the D2/3 is a nucleotide sequence encoding the VEGF-binding fragment derived from the VEGFR nucleotide sequence which comprises the D2/3 of the VEGFR, the linker is a nucleotide sequence encoding the linker, wherein each of the linkers may be same or different.

In some embodiments, the nucleic acid construct of the present application may further comprise a nucleotide sequence encoding the Fc region of the antibody.

In some embodiments, the Fc region may be the Fc region of an antibody isotype selected from IgA, IgG, IgM, IgD and IgE. In some embodiments, the Fc region may be the Fc region of an antibody subtype selected from IgG1, IgG2, IgG3 and IgG4. In one preferred embodiment, the Fc region is the Fc region of human IgG1.

In some preferred embodiments, the nucleic acid construct of the present application comprises, from the 5' end to the 3' end, the following structures:
(1) scFv1-linker-Fc;
(2) D2/3-linker-Fc;
(3) D2/3-linker-scFv1-Fc;
(4) scFv1-linker-D2/3-Fc;
(5) scFv1-linker-scFv2-Fc;
(6) scFv1-linker-scFv2-linker- D2/3-Fc;
(7) scFv1-linker-D2/3-linker-scFv2-Fc; or
(8) D2/3-linker-scFv1-linker-scFv2-Fe,
wherein the scFv1 is a nucleotide sequence encoding the scFv derived from the anti-VEGF antibody, the scFv2 is a nucleotide sequence encoding the scFv derived from another different anti-VEGF antibody, and the D2/3 is a nucleotide sequence encoding the VEGF-binding fragment derived from the VEGFR nucleotide sequence comprises the D2/3 of the VEGFR, the linker is a nucleotide sequence encoding the linker, wherein each of the linkers may be same or different, the Fc is a nucleotide sequence encoding the Fc region of the antibody.

In embodiments of the nucleic acid constructs of the present application, the anti-VEGF antibody may be any antibody that specifically binds VEGF. In some preferred embodiments, the anti-VEGF antibody is selected from Brolucizumab, Ranibizumab or Aflibercept.

In some preferred embodiments, the nucleic acid construct of the present application comprises, from the 5' end to the 3' end, the following structures:
(1) D2/3;
(2) D2/3-Fc;
(3) BrolscFv;
(4) BrolscFv-Fc;
(5) RaniScFv;
(6) RaniScFv-Fc;
(7) D2/3-linker-RaniScFv;
(8) RaniScFv-linker-D2/3;
(9) BrolscFv-linker-RaniScFv-Fc;
(10) BrolscFv-linker-RaniScFv;
(11) RaniScFv-linker-BrolscFv;
(12) RaniScFv-linker- BrolscFv-Fc;
(13) D2/3-linker- RaniScFv-Fc;
(14) RaniScFv-linker-D2/3-Fc;
(15) D2/3-linker-BrolscFv-linker-RaniScFv-Fc;
(16) D2/3-linker-BrolscFv-linker-RaniScFv;
(17) BrolscFv-linker-D2/3-linker-RaniScFv;
(18) BrolscFv-linker-D2/3-linker-RaniScFv-Fc;
(19) RaniScFv-linker-BrolscFv-linker-D2/3;
(20) RaniScFv-linker-BrolscFv-linker-D2/3-Fc;
(21) BrolscFv-linker-D2/3;
(22) BrolscFv-linker-D2/3-Fc;
(23) D2/3-linker-BrolscFv; or
(24) BrolscFv-linker-D2/3,
wherein the BrolscFv is a nucleotide sequence encoding the scFv derived from Brolucizumab, the RaniScFv is a nucleotide sequence encoding the scFv derived from Ranibizumab, and the D2/3 is a nucleotide sequence encoding the VEGF-binding fragment derived from the VEGFR which comprises the D2/3 of the VEGFR, the linker is a nucleotide sequence encoding the linker, wherein each of the linkers may be same or different, the Fc is a nucleotide sequence encoding the Fc region of the antibody.

In some embodiments, the nucleic acid constructs of the present application may further comprise a nucleotide sequence encoding N-terminal signal sequence at 5' end. In some embodiments, the N-terminal signal sequence has the amino acid sequence as set forth in SEQ ID NO: 45.

Accordingly, in some embodiments, the nucleic acid construct of the present application comprises, from the 5' end to the 3' end, the following structures:
(1) signal-scFv1-linker-Fc;
(2) signal-D2/3-linker-Fc;
(3) signal-D2/3-linker-scFv1-Fc;
(4) signal-scFv1-linker-D2/3-Fc;
(5) signal-scFv1-linker-scFv2-Fc;
(6) signal-scFv1-linker-scFv2 -linker- D2/3-Fc;
(7) signal-scFv1-linker-D2/3-linker-scFv2-Fc; or
(8) signal-D2/3-linker-scFv1-linker-scFv2-Fc,
wherein the signal is a nucleotide sequence encoding the signal sequence at the 5' end, the scFv1 is a nucleotide sequence encoding the scFv derived from the anti-VEGF antibody, the scFv2 is a nucleotide sequence encoding the scFv derived from another different anti-VEGF antibody, and the D2/3 is a nucleotide sequence encoding the VEGF-binding fragment derived from the VEGFR nucleotide sequence which comprises the D2/3 of the VEGFR, the linker is a nucleotide sequence encoding the linker, wherein each of the linkers may be same or different, and the Fc is a nucleotide sequence encoding the Fc region of the antibody.

In some preferred embodiments, the nucleic acid construct of the present application comprises, from the 5' end to the 3' end, the following structures:
(1) signal-D2/3;
(2) signal-D2/3-Fc;
(3) signal-BrolscFv;
(4) signal-BrolscFv-Fc;
(5) signal-RaniScFv;
(6) signal-RaniScFv-Fc;
(7) signal-D2/3-linker-RaniScFv;
(8) signal-RaniScFv-linker-D2/3;
(9) signal-BrolscFv-linker-RaniScFv-Fc;
(10) signal-BrolscFv-linker-RaniScFv;
(11) signal-RaniScFv-linker-BrolscFv;
(12) signal-RaniScFv-linker- BrolscFv-Fc;
(13) signal-D2/3-linker-RaniScFv-Fc;
(14) signal-RaniScFv-linker-D2/3-Fc;
(15) signal-D2/3-linker-BrolscFv-linker-RaniScFv-Fc;
(16) signal-D2/3-linker-BrolscFv-linker-RaniScFv;
(17) signal-BrolscFv-linker-D2/3-linker-RaniScFv;
(18) signal-BrolscFv-linker-D2/3-linker-RaniScFv-Fc;
(19) signal-RaniScFv-linker-BrolscFv-linker-D2/3;
(20) signal-RaniScFv-linker-BrolscFv-linker-D2/3-Fc;
(21) signal-BrolscFv-linker-D2/3;
(22) signal-BrolscFv-linker-D2/3-Fc;
(23) signal-D2/3-linker-BrolscFv; or
(24) signal-BrolscFv-linker-D2/3,
wherein the signal is a nucleotide sequence encoding the signal sequence at the 5' end, the BrolscFv is a nucleotide sequence encoding the scFv derived from Brolucizumab, the RaniScFv is a nucleotide sequence encoding the scFv derived from Ranibizumab, and the D2/3 is a nucleotide sequence encoding the VEGF-binding fragment derived from the VEGFR which comprises the D2/3 of the VEGFR, the linker is a nucleotide sequence encoding the linker, wherein each of the linkers may be same or different, the Fc is a nucleotide sequence encoding the Fc region of the antibody.

In some embodiments, the scFv derived from Brolucizumab comprises the amino acid sequence as set forth in SEQ ID NO: 1, or the amino acid sequence having at least 80%, at least 85%, at least 88%, at least 90%, at least 92%, at least 94%, at least 96%, at least 98% or at least 99% sequence identity to SEQ ID NO: 1. In some embodiments, the nucleotide sequence encoding the scFv derived from Brolucizumab in the nucleic acid construct of the present application has the nucleotide sequence as set forth in SEQ ID NO: 2, or the nucleotide sequence having at least 80%, at least 85%, at least 88%, at least 90%, at least 92%, at least 94%, at least 96%, at least 98% or at least 99% sequence identity to SEQ ID NO: 2.

In some embodiments, the scFv derived from Ranibizumab comprises the amino acid sequence as set forth in SEQ ID NO: 3, or the amino acid sequence having at least 80%, at least 85%, at least 88%, at least 90%, at least 92%, at least 94%, at least 96%, at least 98% or at least 99% sequence identity to SEQ ID NO: 3. In some embodiments, the nucleotide sequence encoding the scFv derived from Ranibizumab in the nucleic acid construct of the present application comprises the nucleotide sequence as set forth in SEQ ID NO: 4, or the nucleotide sequence having at least 80%, at least 85%, at least 88%, at least 90%, at least 92%, at least 94%, at least 96%, at least 98% or at least 99% sequence identity to SEQ ID NO: 4.

In some embodiments, the VEGF binding fragment derived from the VEGFR comprises the amino acid sequence as set forth in SEQ ID NO: 5, or the amino acid sequence having at least 80%, at least 85%, at least 88%, at least 90%, at least 92%, at least 94%, at least 96%, at least 98% or at least 99% sequence identity to SEQ ID NO: 5. In some embodiments, the nucleotide sequence encoding the VEGF fragment derived from the VEGFR in the nucleic acid construct of the present application comprises the nucleotide sequence as set forth in SEQ ID NO: 6, or the nucleotide sequence having at least 80%, at least 85%, at least 88%, at least 90%, at least 92%, at least 94%, at least 96%, at least 98% or at least 99% sequence identity to SEQ ID NO: 6.

In some embodiments, the Fc region of the antibody comprises the amino acid sequence as set forth in SEQ ID NO: 34, or the amino acid sequence having at least 80%, at least 85%, at least 88%, at least 90%, at least 92%, at least 94%, at least 96%, at least 98% or at least 99% sequence identity to SEQ ID NO: 34. In some embodiments, the nucleotide sequence encoding the Fc region of the antibody contained in the nucleic acid construct comprises the nucleotide sequence as set forth in SEQ ID NO: 35, or the nucleotide sequence having at least 80%, at least 85%, at least 88%, at least 90%, at least 92%, at least 94%, at least 96%, at least 98% or at least 99% sequence identity to SEQ ID NO: 35.

In the case where the nucleic acid construct of the present application comprises one or more linkers, the linkers may be same or different. In some embodiments, the linker is a cleavage linker. In other embodiments, the linker is a non-cleavage linker. In some embodiments, the linker is a flexible linker. In other embodiments, the linker is a rigid linker.

In some preferred embodiments, the linkers are each independently selected from(GS)ₙ, (GGS)ₙ, (GGGS)ₙ, (GGGGS)ₙ, (Gly)₈, (Gly)₆ and (EAAAK)ₙ, wherein n is 1-10, preferably n is 2-8, and more preferably n is 4 or 5.

In some specific embodiments, the expression vector of the present application comprises, from the 5' end to the 3' end, the following structure: D2/3-linker-RaniScFv, wherein the RaniScFv is a nucleotide sequence encoding the scFv derived from Ranibizumab, the D2/3 is a nucleotide sequence encoding the VEGF binding fragment derived from the VEGFR which comprises the D2/3 of the VEGFR, the linker is a nucleotide sequence encoding the linker.

In some embodiments, the expression vector of the present application encodes a fusion protein which comprises the amino acid sequence as set forth in SEQ ID NO: 13, or the amino acid sequence having at least 80%, at least 85%, at least 88%, at least 90%, at least 92%, at least 94%, at least 96%, at least 98% or at least 99% sequence identity to SEQ ID NO: 13. In some embodiments, the expression vector of the present application comprises the nucleotide sequence as set forth in SEQ ID NO: 14, or the nucleotide sequence having at least 80%, at least 85%, at least 88%, at least 90%, at least 92%, at least 94%, at least 96%, at least 98% or at least 99% sequence identity to SEQ ID NO: 14.

In some specific embodiments, the nucleic acid construct of the present application comprises, from the 5' end to the 3' end, the following structure: BrolscFv-linker-RaniScFv, wherein the BrolscFv is a nucleotide sequence encoding the scFv derived from Brolucizumab, the RaniScFv is a nucleotide sequence encoding the scFv derived from Ranibizumab, the linker is a nucleotide sequence encoding the linker, Fc is a nucleotide sequence encoding the Fc region of the antibody.

In some embodiments, the expression vector of the present application encodes a fusion protein which comprises the amino acid sequence as set forth in SEQ ID NO: 7, or the amino acid sequence having at least 80%, at least 85%, at least 88%, at least 90%, at least 92%, at least 94%, at least 96%, at least 98% or at least 99% sequence identity to SEQ ID NO: 7. In some embodiments, the expression vector of the present application comprises the nucleotide sequence as set forth in SEQ ID NO: 8, or the nucleotide sequence having at least 80%, at least 85%, at least 88%, at least 90%, at least 92%, at least 94%, at least 96%, at least 98% or at least 99% sequence identity to SEQ ID NO: 8.

In some specific embodiments, the expression vector of the present application comprises, from the 5' end to the 3' end, the following structure: BrolscFv-linker-RaniScFv, wherein the BrolscFv is a nucleotide sequence encoding the scFv derived from Brolucizumab, the RaniScFv is a nucleotide sequence encoding the scFv derived from Ranibizumab, and the linker is a nucleotide sequence encoding the linker.

In some embodiments, the expression vector of the present application encodes a fusion protein which comprises the amino acid sequence as set forth in SEQ ID NO: 9, or the amino acid sequence having at least 80%, at least 85%, at least 88%, at least 90%, at least 92%, at least 94%, at least 96%, at least 98% or at least 99% sequence identity to SEQ ID NO: 9. In some embodiments, the expression vector of the present application comprises the nucleotide sequence as set forth in SEQ ID NO: 10, or the nucleotide sequence having at least 80%, at least 85%, at least 88%, at least 90%, at least 92%, at least 94%, at least 96%, at least 98% or at least 99% sequence identity to SEQ ID NO: 10.

In some specific embodiments, the expression vector of the present application comprise, from the 5' end to the 3' end, the following structure: D2/3-linker-RaniScFv-Fc, wherein the RaniScFv is a nucleotide sequence encoding the scFv derived from Ranibizumab, the D2/3 is a nucleotide sequence encoding the VEGF binding fragment derived from the VEGFR which comprises the D2/3 of the VEGFR, the linker is a nucleotide sequence encoding the linker, the Fc is a nucleotide sequence encoding the Fc region of the antibody.

In some embodiments, the expression vector of the present application encodes a fusion protein which comprises the amino acid sequence as set forth in SEQ ID NO: 11, or the amino acid sequence having at least 80%, at least 85%, at least 88%, at least 90%, at least 92%, at least 94%, at least 96%, at least 98% or at least 99% sequence identity to SEQ ID NO: 11. In some embodiments, the expression vector of the present application comprises the nucleotide sequence as set forth in SEQ ID NO: 12, or the nucleotide sequence having at least 80%, at least 85%, at least 88%, at least 90%, at least 92%, at least 94%, at least 96%, at least 98% or at least 99% sequence identity to SEQ ID NO: 12.

In some embodiments, the expression vector of the present application comprises, from the 5' end to the 3' end, the following structure: D2/3-linker-BrolscFv-linker-RaniScFv-Fc, wherein the BrolscFv is a nucleotide sequence encoding the scFv derived from Brolucizumab, the RaniScFv is a nucleotide sequence encoding the scFv derived from Ranibizumab, and the D2/3 is a nucleotide sequence encoding the VEGF-binding fragment derived from the VEGFR which comprises the D2/3 of the VEGFR, the linker is a nucleotide sequence encoding the linker, wherein each of the linkers may be same or different, the Fc is a nucleotide sequence encoding the Fc region of the antibody.

In some embodiments, the expression vector of the present application encodes a fusion protein which comprises the amino acid sequence as set forth in SEQ ID NO: 15, or the amino acid sequence having at least 80%, at least 85%, at least 88%, at least 90%, at least 92%, at least 94%, at least 96%, at least 98% or at least 99% sequence identity to SEQ ID NO: 15. In some embodiments, the expression vector of the present application comprises the nucleotide sequence as set forth in SEQ ID NO: 16, or the nucleotide sequence having at least 80%, at least 85%, at least 88%, at least 90%, at least 92%, at least 94%, at least 96%, at least 98% or at least 99% sequence identity to SEQ ID NO: 16.

In some specific embodiments, the expression vector of the present application comprises, from the 5' end to the 3' end, the following structure: D2/3-linker-BrolscFv-linker-RaniScFv, wherein the BrolscFv is a nucleotide sequence encoding the scFv derived from Brolucizumab, the RaniScFv is a nucleotide sequence encoding the scFv derived from Ranibizumab, and the D2/3 is a nucleotide sequence encoding the VEGF-binding fragment derived from the VEGFR which comprises the D2/3 of the VEGFR, the linker is a nucleotide sequence encoding the linker, wherein each of the linkers may be same or different.

In some embodiments, the expression vector of the present application encodes a fusion protein, said fusion protein comprises the amino acid sequence as set forth in SEQ ID NO: 17, or the amino acid sequence having at least 80%, at least 85%, at least 88%, at least 90%, at least 92%, at least 94%, at least 96%, at least 98% or at least 99% sequence identity to SEQ ID NO: 17. In some embodiments, the expression vector of the present application comprises the nucleotide sequence as set forth in SEQ ID NO: 18, or the nucleotide sequence having at least 80%, at least 85%, at least 88%, at least 90%, at least 92%, at least 94%, at least 96%, at least 98% or at least 99% sequence identity to SEQ ID NO: 18.

In some specific embodiments, the expression vector of the present application comprises, from the 5' end to the 3' end, the following structure: D2/3-linker-BrolscFv, wherein the BrolscFv is a nucleotide sequence encoding the scFv derived from Brolucizumab, the D2/3 is a nucleotide sequence encoding the VEGF-binding fragment derived from the VEGFR which comprises the D2/3 of the VEGFR, and the linker is a nucleotide sequence encoding the linker.

In some embodiments, the expression vector of the present application encodes a fusion protein which comprises the amino acid sequence as set forth in SEQ ID NO: 19, or the amino acid sequence having at least 80%, at least 85%, at least 88%, at least 90%, at least 92%, at least 94%, at least 96%, at least 98% or at least 99% sequence identity to SEQ ID NO: 19. In some embodiments, the expression vector of the present application comprises the nucleotide sequence as set forth in SEQ ID NO: 20, or the nucleotide sequence having at least 80%, at least 85%, at least 88%, at least 90%, at least 92%, at least 94%, at least 96%, at least 98% or at least 99% sequence identity to SEQ ID NO: 20.

In some specific embodiments, the expression vector of the present application comprises, from the 5' end to the 3' end, the following structure: BrolscFv-linker-D2/3, wherein the BrolscFv is a nucleotide sequence encoding the scFv derived from Brolucizumab, the D2/3 is a nucleotide sequence encoding the VEGF-binding fragment derived from the VEGFR which comprises the D2/3 of the VEGFR, and the linker is a nucleotide sequence encoding the linker.

In some embodiments, the expression vector of the present application encodes a fusion protein which comprises the amino acid sequence as set forth in SEQ ID NO: 21, or the amino acid sequence having at least 80%, at least 85%, at least 88%, at least 90%, at least 92%, at least 94%, at least 96%, at least 98% or at least 99% sequence identity to SEQ ID NO: 21. In some embodiments, the expression vector of the present application comprises the nucleotide sequence as set forth in SEQ ID NO: 22, or the nucleotide sequence having at least 80%, at least 85%, at least 88%, at least 90%, at least 92%, at least 94%, at least 96%, at least 98% or at least 99% sequence identity to SEQ ID NO: 22.

In some embodiments, the expression vector of the present application further comprises a promoter sequence for the expression of the nucleotide sequence. In some embodiments, the promoter is selected from CBH, CMV, CAGG, CAG, RPE65 and VMD2 promoter. In a preferred embodiment, the promoter is CAGG.

In some embodiments, the CBH promoter has the amino acid sequence as set forth in SEQ ID NO: 28.

In some embodiments, the CMV promoter has the amino acid sequence as set forth in SEQ ID NO: 29.

In some embodiments, the CAGG promoter has the amino acid sequence as set forth in SEQ ID NO: 30.

In some embodiments, the CAG promoter has the amino acid sequence as set forth in SEQ ID NO: 31.

In some embodiments, the RPE65 promoter has the amino acid sequence as set forth in SEQ ID NO: 32.

In some embodiments, the VMD2 promoter has the amino acid sequence as set forth in SEQ ID NO: 33.

In some embodiments, the expression vector of the present application is a viral vector. In some embodiments, the expression vector of the present application is a viral vectors that is capable of stable expression in host cells.

In some embodiments, the expression vector of the present application is selected from a bovine papillomavirus vectors, an EBV virus vector, a retroviral vector (e.g., a lentiviral vector) and an adeno-associated virus vector.

In some embodiments, the expression vector of the present application is a lentiviral vector. In some preferred embodiments, the lentiviral vector further comprises one or more elements selected from a chimeric LTR promoter, an HIV-1 packaging signal (ψ), a central polypurine tract (cPPT), a Rev response element (RRE), a polypurine tract (PPT), a woodchuck hepatitis B virus post-transcriptional regulatory element (WPRE), a polyadenylation signal of SV40 virus (SV40 pA signal), a replication origin of SV40 virus (SV40 ori) and a self-inactivating long terminal repeats.

In other embodiments, the expression vector of the present application is an adeno-associated virus (AAV) vector. In some preferred embodiments, the adeno-associated viral vector further comprises one or more elements selected from the polyadenylation signal of SV40 virus (SV40SL) and the inverted terminal repeats (ITR).

In the second aspect, the present application provides a viral particle. The viral particle of the present application comprises the expression vector as described in the first aspect of the present application. In some preferred embodiments, the viral particle of the present application is a lentiviral particle or an adeno-associated viral particle.

In the third aspect, the present application provides a cell, which comprises the expression vector as described in the first aspect of the present application.

In some embodiments, the cell of the present application comprises the adeno-associated viral expression vector as described in the present application, and further comprises a serotype plasmid and/or helper plasmid for assembling the adeno-associated viral expression vector into the viral particle.

In some other embodiments, the cell of the present application comprises the lentiviral expression vector as described in the present application, and further comprises an envelope plasmid and/or a packaging plasmid for assembling the lentiviral expression vector into the lentiviral particle.

In the fourth aspect, the present application provides a cell, which has been infected with the viral particle as described in the second aspect of the present application. In some embodiments, the cell is a retinal cell. In a preferred embodiment, the cell is a human retinal pigment epithelial cell.

In the fifth aspect, the present application provides a vector system comprising the expression vector as described herein, and optionally one or more additional vectors such as plasmids. In some embodiments, the vector system further comprises a host cell, preferably the host cell is a 293T cell.

In some embodiments, the vector system is a lentiviral vector system. In some embodiments, the lentiviral vector system comprises a lentiviral expression vector and a helper plasmid. In some embodiments, the helper plasmid comprises one or more nucleotide sequences encoding gag and pol proteins and optionally nucleotide sequences encoding other viral packaging components. In some embodiments, the helper plasmid may comprise a packaging plasmid and/or an envelope plasmid. In some embodiments, the lentiviral vector system further comprises a host cell, whichmay be a lentivirus-producing cell, for example may be a 293T cell.

In some embodiments, the vector system is an adeno-associated virus (AAV) vector system. In some embodiments, the AAV vector system comprises an AAV expression vector as well as a serotype plasmid and/or a helper plasmid. In some embodiments, the helper plasmid comprises a nucleotide sequence encoding a viral packaging component. In some embodiments, the serotype plasmid is selected from serotypes AAV1, AAV2, AAV3, AAV4, AAV5, AAV6, AAV7, AAV8 and AAV9. In some embodiments, the AAV vector system further comprises a host cell, whichmay be a cell that produces an adeno-associated virus, for example may be a 293T cell.

In the sixth aspect, the present application provides a composition (e.g., a pharmaceutical composition) comprising the expression vector as described in the first aspect of the present application, the viral particle as described in the second aspect of the present application, the cell as described in the third or fourth aspect of the present application, or the vector system as described in the fifth aspect of the present application. Optionally, the compositions of the present application further comprise pharmaceutically acceptable carriers and/or excipients.

In the seventh aspect, the present application provides a system for intraocular delivery comprising: the expression vector as described in the first aspect of the present application, the viral particle as described in the second aspect of the present application, the cell as described in the third or fourth aspect of the present application, the vector system as described in the fifth aspect of the present application or the composition as described in the sixth aspect of the present application; and b) a device for intraocular delivery.

In some embodiments, the device for intraocular delivery is selected from a device for intravitreal delivery, a device for subretinal cavity delivery and a device for suprachoroidal cavity delivery.

In the eighth aspect, the present application provides a method for treating age-related macular degeneration in a subject, including the step of administering the expression vector as described in the first aspect of the application, the viral particle as described in the second aspect of the application, the cell as described in the third aspect or the fourth aspect of the application, the vector system as described in the fifth aspect of the application or the composition as described in the sixth aspect of the application into the eye of the subject. In some embodiments, the age-related macular degeneration is wet macular degeneration.

In some preferred embodiments, the expression vector, the viral particle, the cell, the vector system, or the composition is administered to the intravitreal and/or subretinal cavity and/or suprachoroidal cavity of the subject.

### Description of Drawings

FIG. 1 illustrates the mapping of KL-Kan-BB, a third-generation, replication-defective, self-inactivating pseudo-enveloped lentiviral vector based on HIV-1.
FIG. 2 illustrates a schematic diagram of constructs based on the lentiviral vector of KL-Kan-BB carrying coding sequences for different fusion proteins or a single anti-VEGF agent.
FIG. 3 illustrates the mapping of the adeno-associated viral vector pAAV-MCS.
FIG. 4 illustrates a schematic diagram of constructs based on the adeno-associated viral vector pAAV-MCS carrying coding sequences for different fusion proteins or a single anti-VEGF agent.
FIG. 5 illustrates a schematic diagram of the molecular structure of the expected mature fusion proteins of fusion proteins 1 to fusion proteins 8 of the present application after infection and expression in cells by lentivirus or AAV.
FIG. 6 illustrates the corresponding anti-VEGF proteins expressed by the cells after transfection of the cells with the lentiviral expression vectors pKL-Kan-BB-CAGG-Fusion Protein 3, pKL-Kan-BB-CAGG-Fusion Protein 4 and pKL-Kan-BB-CAGG-Fusion Protein 7, as detected by Coomassie Brilliant Blue staining.
FIG. 7 illustrates the protein loading information when the expression of anti-VEGF protein wasdetected by Western Blot.
FIG. 8 illustrates the corresponding anti-VEGF proteins expressed by the cells after transfection of the cells with the lentiviral expression vectors pKL-Kan-BB-CAGG-Fusion Protein 1 to pKL-Kan-BB-CAGG-Fusion Protein 8, pKL-Kan-BB-CAGG-Ranibizumab or pKL-Kan-BB-CAGG-Aflibercept, as detected by Western Blot.
FIG. 9 illustrates the binding of the corresponding anti-VEGF proteins to VEGF expressed by the cells after transfection of the cells with the lentiviral expression vectors pKL-Kan-BB-CAGG-Fusion Protein 1 to pKL-Kan-BB-CAGG-Fusion Protein 8, pKL-Kan-BB-CAGG-Ranibizumab or pKL-Kan-BB-CAGG-Aflibercept, as detected by Western Blot.
FIG. 10 illustrates the levels of corresponding anti-VEGF proteins expressed by cells after transduction of the cells with the lentiviral particles containing pKL-Kan-BB-CAGG-Fusion Protein 1 to pKL-Kan-BB-CAGG-Fusion Protein 8, pKL-Kan-BB-CAGG-Ranibizumab or pKL-Kan-BB-CAGG-Aflibercept constructs, respectively.
FIG. 11 illustrates the levels of corresponding anti-VEGF proteins expressed by cells after transduction of the cells with the adeno-associated viral particles containing pAAV-MCS-CAGG-Fusion Protein 1 to pAAV-MCS-CAGG-Fusion Protein 8, pAAV-MCS-CAGG-Ranibizumab or pAAV-MCS-CAGG-Aflibercept constructs, respectively.
FIG. 12 illustrates the levels of corresponding anti-VEGF proteins expressed by cells after transduction of the cells with the adeno-associated viral particles containing pAAV-MCS-CAGG-Fusion Protein 4, pAAV-MCS-CAGG-Ranibizumab or pAAV-MCS-CAGG-Aflibercept constructs, respectively.
FIG. 13 illustrates the expression levels of target proteins within the 4w after injection of AAV dilutions, adeno-associated viral particles containing pAAV-MCS-CAGG-Fusion Protein 4 and pAAV-MCS-CAGG-Aflibercept constructs, respectively, into the rat vitreous.
FIG. 14 illustrates the zoning of the eye by the FFA angiographic scoring method.
FIG. 15 illustrates the FFA angiography results of the left and right eyes 2 weeks after modeling and dosing to Dutch rabbits.
FIG. 16 illustrates the content of target proteins in the aqueous fluid of the right eye collected at different time points after modeling and dosing to Dutch rabbits.

### Detailed Description of the Invention

Unless otherwise defined, all technical and scientific terms used herein have the same meaning as commonly understood by one of ordinary skill in the art to which this disclosure belongs.

In order to more readily understand the present disclosure, certain terms are first defined below. The following terms and additional definitions of other terms are set forth throughout the specification.

As used in this specification and the appended claims, the singular forms "a", "an" and "the" include plural referents unless the context clearly indicates otherwise.

Herein, the terms "comprising", "having" , "including" and "containing" should be construed as open-ended terms (i.e., meaning "including but not limited to ").

As used herein, "and/or" means and includes any and all possible combinations of one or more items of the associated listed items. For example, a composition comprises A and/or B may be construed as a composition comprises A, a composition comprises B or a composition comprises A and B.

All numerical designations, such as pH, temperature, time, concentration, and molecular weight, including ranges, are approximation and are changed (+) or ( -) in increments of 1.0 or 0.1 as appropriate, or optionally in changes of +/ -15%, 10%, 5%, 2%. It should be understood that all numerical designations are preceded by the term "about". It should also be understood that the reagents described herein are exemplary only and that equivalents thereof are known in the art. When referring to measurable values such as amounts or concentrations, the term "about" as used herein is meant to include a variation within 20%, 10%, 5%, 1%, 0.5% or 0.1% of the specified amount.

As used herein, the terms "nucleic acid", "nucleic acid molecule", "nucleic acid sequence", "nucleotide sequence" and "polynucleotide" are used interchangeably and refer to the polymerized form of nucleotides (ribonucleotides or deoxyribonucleotides) of any length. Accordingly, the terms include, but are not limited to, single-strand, double-strand, or doublestranded DNA or RNA, genomic DNA, cDNA, DNA-RNA hybrids or polymers comprising, consisting of or consisting essentially of purine and pyrimidine bases, or other natural, chemically or biochemically modified, non-natural or derived nucleotide bases.

The terms "protein", "peptide", "polypeptide" and "amino acid sequence" are used interchangeably and, in their broadest sense, refer to the polymerized form of two or more amino acid subunits, amino acid analogs or peptide mimetics. "Protein", "peptide", "polypeptide" and "amino acid sequence" contain at least two amino acids, and there is no limitation on the maximum number of amino acids. The term "amino acid" as used herein refers to natural and/or non-natural or synthetic amino acids, including D and L optical isomers and amino acid analogs.

As used herein, "expression" refers to the process by which a nucleic acid sequence is transcribed into mRNA and/or the transcribed mRNA is subsequently translated into a peptide, polypeptide, amino acid sequence or protein. If the nucleic acid sequence is from genomic DNA, the expression may include splicing the mRNA in eukaryotic cells.

The term "encoding" when applied to a nucleic acid sequence refers to a nucleic acid sequence that "encodes" a polypeptide if it can be transcribed to produce mRNA and/or translated to produce a polypeptide in its native state or when manipulated by methods well known to those skilled in the art. The antisense strand is the complement of such a nucleic acid and the coding sequence can be deduced from it.

"Homology" or "identity" refers to sequence similarity between two polypeptides or between two nucleic acid sequences. The percentage of identity may be determined by comparing positions in each sequence, said sequences may be aligned for comparison purposes. When the positions in the sequences being compared are occupied by same bases or amino acids, the molecules are identical at that position. The degree of identity between sequences depends on the number of shared matching positions. "Unrelated" or "non-homologous" sequences share less than 40% identity, less than 25% identity with one of the sequences of the present application. By importing a nucleic acid sequence or an amino acid sequence into ClustalW (available from https://genome.jp/tools-bin/clustalw/) and using that ClustalW, alignments and percentage of sequence identity of the nucleic acid sequecnes or the amino acid sequences provided herein can be determined.

The term "promoter" as used herein refers to an expression control sequence that controls the initiation and rate of transcription of a gene or a transgene. The promoter may be, for example, constitutive, inducible, repressive or tissue-specific. The promoter can contain genetic elements that regulate the binding of proteins and molecules such as RNA polymerase and transcription factors.

Non-limiting examples of promoters include pol I promoter, pol II promoter, pol III promoter, T7 promoter, U6 promoter, H1 promoter, retroviral Rous sarcoma virus LTR promoter, cytomegalovirus (CMV) promoter, SV40 promoter, dihydrofolate reductase promoter, beta-actin promoter, elongation factor 1α short (EFS ) promoter, β-glucuronidase (GUSB) promoter, cytomegalovirus (CMV) immediate early (IE) enhancer and/or promoter, chicken β-actin (CBA) promoter or derivatives thereof such as CAG promoter, CB promoter, (human) elongation factor 1α-subunit (EF1α) promoter, ubiquitin C (UBC) promoter, prion promoter, neuron-specific enolase (NSE), neurofilament light chain (NFL) promoter, neurofilament heavy chain (NFH) promoter, platelet-derived growth factor (PDGF) promoter, platelet-derived growth factor B-chain (PDGF-β) promoter, synaptic protein (Syn) promoter, synaptic protein 1 (Syn1) promoter, methyl-CpG binding protein 2 (MeCP2) promoter, Ca2+/calmodulin-dependent protein kinase II (CaMKII) promoter, metabotropic glutamate receptor 2 (mGluR2) promoter, neurofilament light chain (NFL) promoter, neurofilament heavy chain (NFH) promoter, β-globin minigene nβ2 promoter, pro-enkephalin (PPE) promoter, enkephalin (Enk) promoter, excitatory amino acid transport protein 2 (EAAT2) promoter, glial fibrillary acidic protein (GFAP) promoter, myelin basic protein (MBP) promoter or functional fragments thereof.

It is known in the art that the nucleotide sequences of these promoters can be modified to increase or decrease the efficiency of mRNA transcription. Promoters of synthetic origin can be used for ubiquitous or tissue-specific expression. In addition, promoters of viral origin, some of which are described above, may be used in the methods disclosed herein, such as CMV, HIV, adenovirus and AAV promoters. In embodiments, the use of promoters together with enhancers can increase transcription efficiency. Non-limiting examples of enhancers include interstitial retinol binding protein (IRBP) enhancers, RSV enhancers or CMV enhancers.

As used herein, "poly A signal", "polyadenylation signal" or "polyadenylation signal" refers to the RNA sequence that is located immediately downstream of the 3' coding region and is recognized by an RNA cleavage complex that cleaves the 3' end sequence of the newly transcribed RNA by RNA polymerase (e.g., Pol II) so that polyadenylation can occur. Polyadenylate polymerase then adds and extends the polyA tail by adding the adenosine monophosphate unit from ATP to the de novo cleaved 3' end of the RNA. The poly A signal sequence recognized by the RNA cleavage complex varies among different groups of eukaryotes, with most human polyadenylation sites containing AAUAAA sequence, although this sequence is less common in plant and fungal mRNAs. All of these sequence motifs recognized by the RNA cleavage complex to enable RNA cleavage and subsequent polyadenylation are within the range of the poly A signal sequence.

Exemplary polyadenylation signals include the bovine growth hormone polyadenylation signal (bGH poly A), the small poly A signal (SPA), the human growth hormone polyadenylation signal (hGH poly A), the polyadenylation signal of SV40 virus (SV40 poly A), the rabbit beta globin poly A sequence (rBG poly A) or variants thereof.

As used herein, the terms "patient" and "subject" are used interchangeably and in their conventional meanings, and refer to an organism suffering from, or susceptible to, the condition that can be prevented or treated by administering the viral vector or the viral particle or the composition of the present application, and include human and non-human animals.

In one embodiment, the subjects are non-human animals (for example, chimpanzees and other ape and monkey species; farm animals such as cattle, sheep, pigs, goats, and horses; domestic mammals such as dogs and cats; laboratory animals including rodents such as mice, rats, and guinea pigs; birds, including domestic, wild and game birds such as chickens, turkeys and other gallinaceous birds, ducks, geese, and the like). In one embodiment, the subjects are mammals. In one embodiment, the subjects are human.

As used herein, the term "administration" is intended to denote the delivery of a substance to a subject, such as an animal or a human. Administration may be performed at a single dose, continuously or intermittently throughout the course of treatment. Methods for determining the most effective mode of administration and dosage are known to those of skill in the art and will vary with factors such as the composition used for treatment, the therapeutic purpose and the age, health, or gender of the subject being treated. In some embodiments, single or multiple administrations may be made, with dosage levels and patterns selected by a physician or, in the case of pets and other animals, by a veterinarian.

As used herein, the term "treating" includes: (1) suppressing a condition, disease, or disorder, i.e., preventing, reducing or delaying the progression of a disease or its recurrence or the development of at least one clinical or subclinical symptom thereof; or (2) alleviating a disease, i.e., causing regression of at least one of the conditions, diseases or disorders, or clinical or subclinical symptoms thereof.

As used herein, the term "improvement" refers to the improvement in a symptom associated with a disease and may refer to the improvement in at least one parameter that measures or quantifies the symptom.

As used herein, the term "preventing" a condition, disease, or disorder includes: preventing, delaying or reducing the incidence and/or likelihood of occurrence of at least one clinical or subclinical symptom of a developing condition, disease, or disorder in a subject who may suffer from, or be susceptible to, the condition, disease, or disorder but not yet experience or exhibit the clinical or subclinical symptoms of the condition, disease or disorder.

As used herein, the term "vector" refers to a macromolecule or a series of macromolecules encapsulating a polynucleotide that facilitates the delivery of the polynucleotide into a target cell in vitro or in vivo. Classifications of vectors include, but are not limited to, plasmids, viral vectors, liposomes and other gene delivery vectors. The polynucleotide to be delivered is sometimes referred to as a "transgene" and includes, but is not limited to, the coding sequence of certain proteins or synthetic polypeptides that may enhance, inhibit, impair, protect, trigger or prevent certain biological functions and physiological functions, the coding sequence of interest in vaccine development (e.g., polynucleotides that express proteins, polypeptides or peptides that are suitable for triggering immune responses in mammals), the coding sequences of RNAi components (e.g., shRNAs, siRNAs, antisense oligonucleotides), or optional markers.

As used herein, "viral vector" means a vector capable of being packaged to recombinantly produce viral particles, which contains polynucleotides that are to be delivered in vivo, ex vivo or in vitro into a host cell. Examples of the viral vectors include retroviral vectors, AAV vectors, lentiviral vectors, adenoviral vectors, alphaviral vectors, and the like.

As used herein, the terms "transduction", "transfection" and "transformation" refer to the process by which a heterologous polynucleotide is delivered to a host cell to undergo transcription and translation to produce a polypeptide product, including the introduction of a heterologous polynucleotide into a host cell using a recombinant virus.

As used herein, the term "infection" refers to the process by which a virus or viral particle comprising a polynucleotide component delivers a polynucleotide to a cell and produces its RNA and protein products, or it may also refer to the process of replication of the virus in a host cell.

"Multiplicity of infection" (abbreviated as MOI) is used herein to refer to the ratio of the number of viruses added to cells when infecting the cells with the virus.

The term "virus copy number (VCN)" is used herein to refer to: the viral vector genome integrated into a target cell, which measures the genetic dose of the transgene present in the genetically modified cell.

The term viral "titer" is used herein to refer to the number of infectious viral vector particles per unit volume of fluid. Specifically referred to herein the number of viral vector particles with defined transduction capacity per milliliter of viral vector product, that is, transduction units (TU) per milliliter (TU/ml).

The term viral "transduction unit (TU)" is used herein to refer to the viral vector particle that is capable of infecting and integrating into the genome of a cell.

The term "expression cassette" refers to a sequence that has the potential to encode a protein.

As used herein, the lentiviral vector includes viral vector systems that are modified from the HIV-1 virus to efficiently introduce target genes (or RNAi) into animal and human primary cells or cell lines. The lentiviral vector genome is positive-stranded RNA. After entering the cell, the genome is reversed into DNA by the reverse transcriptase carried by the lentiviral vector in the cytoplasm to form a DNA pre-integration complex. After entering the cell nucleus, the DNA is integrated into the cell genome. The integrated DNA transcribes mRNA and returns to the cytoplasm to express the target protein.

In addition, lentiviral vectors can efficiently infect and integrate into non-dividing cells. The above characteristics make lentiviral vectors distinct from other viral vectors, such as non-integrated adenoviral vectors, adeno-associated viral vectors with low integration rates and traditional retroviral vectors that only integrate dividing cells. A large number of literature studies have shown that the tissues or cells withlong-term expression of target genes mediated by lentiviral vectors include brain, liver, muscle, retina, hematopoietic stem cells, bone marrow mesenchymal stem cells, macrophages, etc. Lentiviral vectors do not express any HIV-1 proteins, have low immunogenicity, have no cellular immune response at the injection site and have low humoral immune response, which does not affect the second injection of the viral vector.

"Adeno-associated virus" or "AAV" belongs to the genus Dependoparvovirus, family Parvoviridae. The AAV is single-stranded DNA virus that composed of a viral genome and capsid. The AAV genome comprises the rep and cap genes flanked by two end terminal inverted repeats (ITRs). Among them, the Rep protein encoded by the rep gene is related to the replication and packaging of the virus, and the cap gene encodes the capsid protein of the virus. The "ITR" or "Inverted Terminal Repeat" can form a T-shaped palindrome structure that is involved in the replication and packaging process of AAV, which is often essential for completing the lytic and latent life cycles of AAV. AAV itself is replication-deficient and can only be latent in the host cell in the absence of a helper virus. Therefore, the production of AAV vectors usually requires helper plasmids (helper) to provide key genes involved in AAV replication as well as serotype plasmids to provide capsid proteins encoding AAV particles.

There are more than a dozen common serotypes and hundreds of variants of AAV that have currently been found, the main difference between them lies in the cap gene that encodes the capsid protein. The serotypes of AAV are primarily determined by the structure of the capsid protein, and these serotypes include, but are not limited to, AAV1, AAV2, AAV3, AAV4, AAV5, AAV6, AAV7, AAV8, AAV9, AAV10, AAV11, AAV12, AAV13, AAVrh10, AAV-DJ, AAV-DJ/B, AAV PHP.B, AAV PHP.eB, AAVPHP.S, AAVrh74.

As used herein, "AAV vector" and "recombinant AAV (rAAV) vector" are used interchangeably and refer to the vector comprising one or more heterologous nucleic acid sequences and one or more ITRs. When AAV vectors are present in host cells, with the help of helper plasmids and serotype plasmids, AAV vectors carrying target genes can be replicated and packaged into infectious viral particles.

The term "pharmaceutically acceptable" refers to a carrier, excipient or diluent that is suitable for use in contact with human and animal tissues without excessive toxicity, irritation, allergic response or other problems or complications, commensurate with a reasonable benefit/risk ratio, within the scope of sound medical judgment.

Exemplary carriers for use in the compositions of the present application include saline, buffered saline, glucose and water. Exemplary excipients for use in the compositions of the present application include fillers, binders, disintegrants, coating agents, adsorbents, anti-adhesion agents, flow aids, preservatives, antioxidants, flavoring agents, coloring agents, sweeteners, solvents, co-solvents, buffers, chelating agents, viscosity imparting agents, surfactants, diluents, wetting agents, carriers, diluents, preservatives, emulsifiers, stabilizers and tension modifiers. It is known to those skilled in the art to select suitable excipients for the preparation of the compositions of the present application. Typically, the choice of a suitable excipient depends in particular on the active agent used, the disease to be treated and the desired dosage form of the composition.

The viral vectors, the cells, the delivery systems or the viral particles of the present application are delivered intraocularly into a subject. In some embodiments, delivery may be by subretinal cavity delivery, suprachoroidal cavity delivery or intravitreal delivery.

Methods of subretinal cavity delivery are known in the art. See, for example, WO 2009/105690, which is incorporated herein by reference. Typically, the expression vectors described in the present application can be delivered in the form of intraocular (subretinal) injectable compositions under direct observation with a surgical microscope.

The steps used for intravitreal injection are known in the art (see, e.g., Peyman, G. A et al. (2009) Retina 29(7):875-912, and Fagan, X. J and Al-Qureshi, S. (2013) Clin. Experiment. Ophthalmol. 41(5):500-7). Briefly, subjects with intravitreal injections may be prepared for steps such as pupil dilation, disinfection of the eye, and administration of anesthetic. Any suitable mydriatic agents known in the art may be used to dilate the pupil. Adequate mydriasis can be confirmed prior to treatment. Sterilization may be achieved by antisepticeye treatment such as an iodine-containing solution such as povidone-iodine. Similar protocols can also be used to clean the eyelids, eyelashes and any other organizations nearby (e.g., skin). Any suitable anesthetic may be used in any appropriate concentration, such as lidocaine or proparacaine. The anesthetic may be administered by any methods known in the art, including, but not limited to, topical drops (drop), gels (gel) or jellies (jelly), and subconjunctival administration of the anesthetic. A sterilized eyelid speculum may be used to clear eyelashes from said area prior to injection. The site of injection can be labeled with a syringe. The site of injection can be selected based on the patient's lens. For example, the site of injection may be 3-3.5 mm from the (corneal) limbus in the patient with a pseudophakic eye or aphakia eye, and 3 .5-4 mm from the (corneal) limbus in the patient with a phakic eye.

The following provides a more detailed explanation of the present disclosure in conjunction with the accompanying drawings and examples. The following examples are used only to illustrate the present disclosure and are not intended to limit the scope of the present disclosure. Experimental methods for which specific conditions are not indicated in the examples are manipulated in accordance with conventional conditions known in the art or in accordance with conditions recommended by the manufacturer.

### Examples

### Example 1. Design of lentiviral vectors and adeno-associated viral vectors comprising anti-VEGF fusion proteins

Two or three VEGF binding regions were designed based on the sequences of Ranibizumab, Aflibercept (VEGFR D2/3-Fc) and Brolucizumab, and each of the VEGF binding region was linked by (G₄S)₅ as a linker to form a fusion protein. The structures of the fusion protein constructs are shown in Table 1.

**Table 1. Structure of constructs encoding fusion proteins that bind VEGF**

| Construct name | Name of the fusion protein | Structure of the fusion protein | Amino acid sequence of the fusion protein | Nucleotide sequences encoding the fusion protein |
|---|---|---|---|---|
| Construct 1 | Fusion protein 1 | BrolscFv -(G₄S)₅- RaniScFv-Fc | SEQ ID NO: 7 | SEQ ID NO: 8 |
| Construct 2 | Fusion protein 2 | BrolscFv-(G₄S)₅-RaniScFv | SEQ ID NO: 9 | SEQ ID NO: 10 |
| Construct 3 | Fusion protein 3 | D2/3-(G₄S)₅-RaniScFv -Fc | SEQ ID NO: 11 | SEQ ID NO: 12 |
| Construct 4 | Fusion protein 4 | D2/3-(G₄S)₅-RaniScFv | SEQ ID NO: 13 | SEQ ID NO: 14 |
| Construct 5 | Fusion protein 5 | D2/3-(G₄S)₅-BrolscFv -(G₄S)₅-RaniScFv -Fc | SEQ ID NO: 15 | SEQ ID NO: 16 |
| Construct 6 | Fusion protein 6 | D2/3-(G₄S)₅-BrolscFv-(G₄S)₅-RaniScFv | SEQ ID NO: 17 | SEQ ID NO: 18 |
| Construct 7 | Fusion protein 7 | D2/3-(G₄S)₅-BrolscFv | SEQ ID NO: 19 | SEQ ID NO: 20 |
| Construct 8 | Fusion protein 8 | BrolscFv-(G₄S)₅-D2/3 | SEQ ID NO: 21 | SEQ ID NO: 22 |

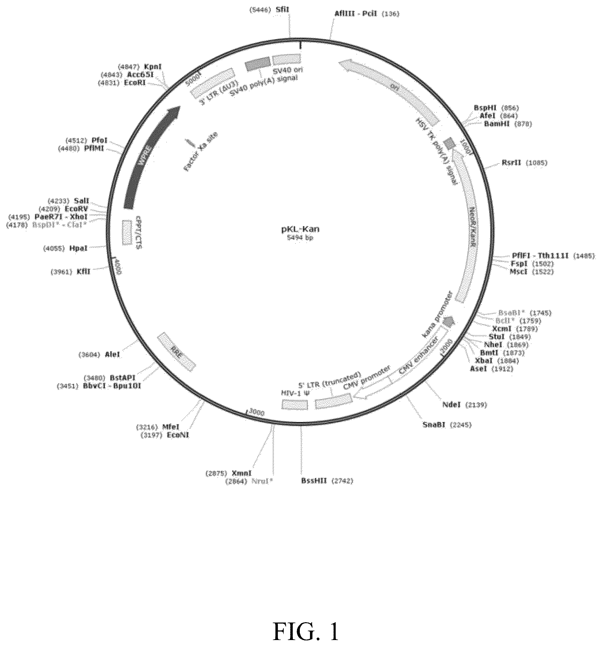

The CAGG promoter was selected as the promoter for the expression of these sequences. These gene expression cassettes were designed to be inserted into the third-generation replication-deficient self-inactivating pseudoenveloped lentiviral vector KL-Kan-BB based on HIV-1. The nucleotide sequence of this lentiviral vector backbone is as set forth in SEQ ID NO: 23. As shown in Figure 1, the lentiviral vector comprises a chimeric LTR promoter, an HIV-1 packaging signal (ψ), a central polypurine region (cPPT), a Rev response element (RRE), a polypurine fragment (PPT), a woodchuck hepatitis B virus post transcriptional regulatory element (WPRE), an polyadenylation signal of SV40 virus (SV40 pA signal), an replication origin of SV40 virus (SV40 ori) and a self-inactivating long end terminal repeats.

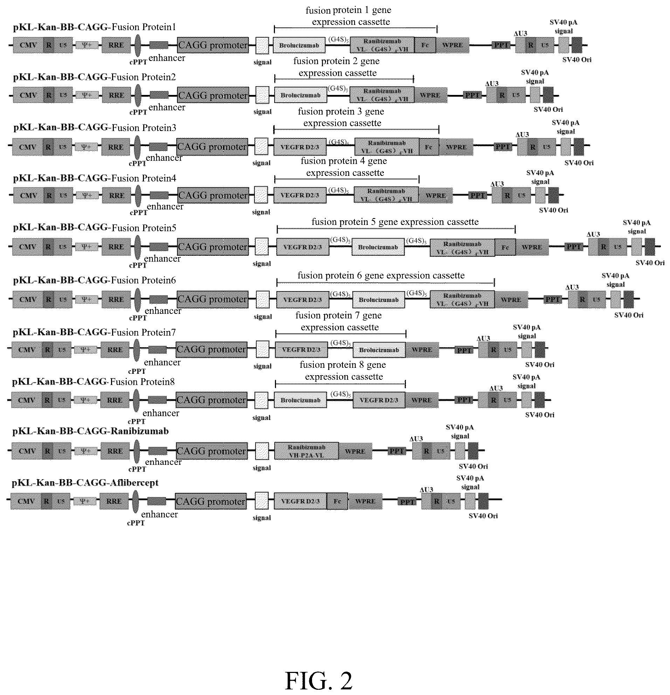

A variety of lentiviral vectors were constructed, as shown in Figure 2, which respectively comprise nucleotide sequences encoding fusion proteins that bind to VEGF (fusion protein 1 to fusion protein 8), or comprise only nucleotide sequences encoding the scFv derived from Ranibizumab, or nucleotide sequences encoding VEGF binding fragments derived from a VEGFR, namely VEGFR D2/3 and Fc.

These lentiviral vectors carrying the coding sequence of one of fusion protein 1 to fusion protein 8 were named as pKL-Kan-BB-CAGG-Fusion Protein 1 to pKL-Kan-BB-CAGG-Fusion Protein 8, respectively. The lentiviral vectors carrying only the nucleotide sequence encoding the scFv derived from Ranibizumab and the nucleotide sequence encoding the VEGF binding fragment derived from the VEGFR (i.e. VEGFR D2/3 and Fc), were named as pKL-Kan-BB-CAGG-Ranibizumab and pKL-Kan-BB-CAGG-Aflibercept, respectively.

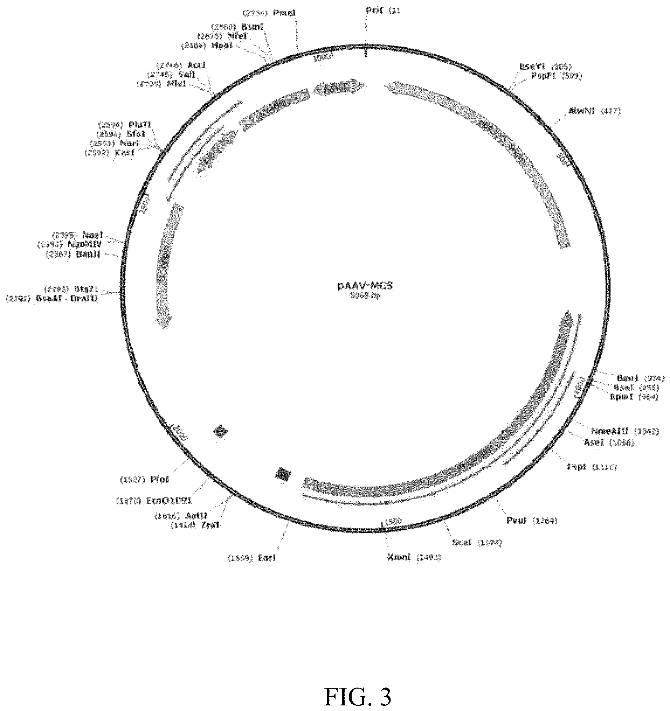

These gene expression cassettes were also designed to be inserted into the adeno-associated virus vector pAAV-MCS. The nucleotide sequence of the adeno-associated virus vector pAAV-MCS backbone is as set forth in SEQ ID NO: 24. As shown in Figure 3, the AAV vector comprises the polyadenylation signal of the SV40 virus (SV40SL), the inverted terminal repeats (ITR) and other elements.

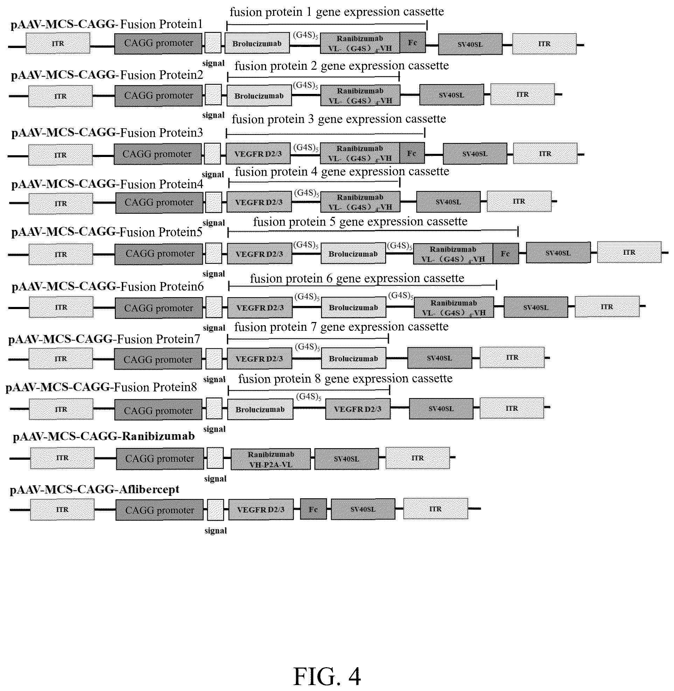

A variety of AAV vectors were constructed, as shown in Figure 4, which respectively comprise nucleotide sequences encoding fusion proteins that bind to VEGF (fusion protein 1 to fusion protein 8), or comprise only nucleotide sequences encoding the scFv derived from Ranibizumab, or nucleotide sequences encoding VEGF binding fragments derived from a VEGFR, namely VEGFR D2/3 and Fc. These AAV vectors carrying the coding sequence of one of fusion proteins 1 to fusion protein 8 were named as pAAV-MCS-CAGG-Fusion Protein 1 to pAAV-MCS-CAGG-Fusion Protein 8, respectively. The AAV vectors carrying only the nucleotide sequence encoding the scFv derived from Ranibizumab and the nucleotide sequence encoding the VEGF binding fragment derived from the VEGFR (, i.e. VEGFR D2/3 and Fc), were named as pAAV-MCS-CAGG-Ranibizumab and pAAV-MCS-CAGG-Aflibercept, respectively.

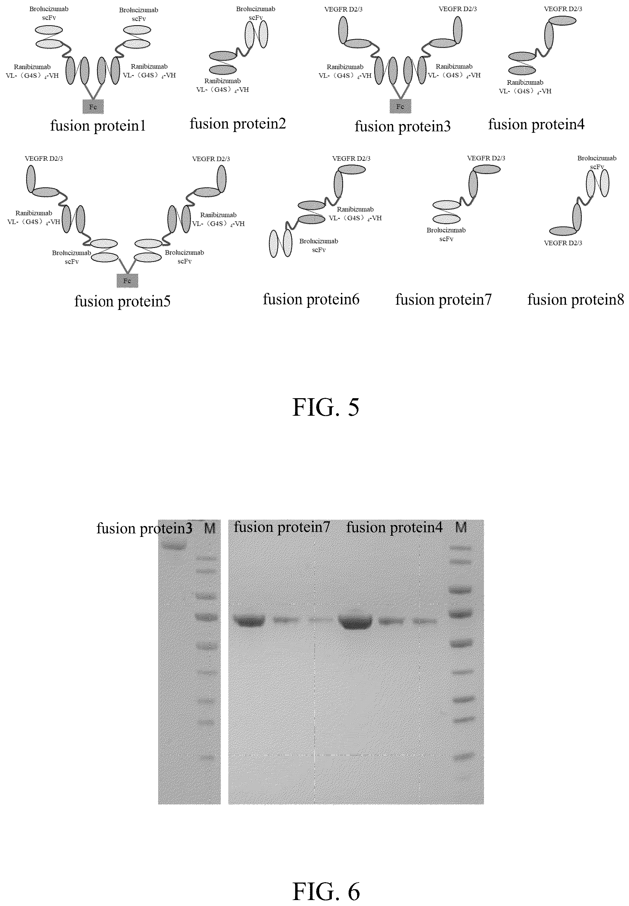

The molecular structures of the expected mature VEGF-binding fusion proteins after these gene expression vectors are infected and expressed in cells by lentivirus or AAV are as shown in Figure 5.

### Example 2. Construction of lentiviral vectors comprising anti-VEGF molecules

The gene expression cassette containing the fusion protein encoding VEGF designed in Example 1 and the promoter CAGG were cloned into a lentiviral vector. The lentiviral vector backbone is the third generation lentiviral backbone - pKL-Kan-BB (nucleotide sequence is SEQ ID NO: 23) from Kanglin Biotechnology (Hangzhou) Co., Ltd (see Figure 1).

The gene expression cassette construct 1 containing the nucleotide sequence encoding the fusion protein 1 binding to VEGF designed in Example 1 (comprising CAGG promoter) was synthesized via Nanjing GenScript Biotech Co., Ltd., and then cloning was performedbetween the multiple cloning sites XhoI/Sall on the lentiviral expression vector pKL-Kan-BB by the homologous recombination method well known in the art. After cloning, sequencing was performed to confirm the sequence information, and the constructed lentiviral expression vector was named as pKL-Kan-BB-CAGG-Fusion Protein 1.

The gene expression cassette constructs 2 to 8 containing the nucleotide sequences encoding the fusion proteins 2 to 8 binding to VEGF designed in Example 1 were synthesized via Nanjing GenScript Biotech Co., Ltd., and then replacement cloning was performedbetween the multiple cloning sites AgeI/SalI on the lentiviral expression vector pKL-Kan-BB-CAGG-Fusion Protein 1 by the homologous recombination method well known in the art. After cloning, sequencing was performed to confirm the sequence information, and the constructed lentiviral expression vectors were named as pKL-Kan-BB-CAGG-Fusion Proteins 2 to 8, respectively.

The nucleic acid coding sequence of the scFv derived from Ranibizumab designed in Example 1 was synthesized via Nanjing GenScript Biotech Co., Ltd., and then replacement cloning was performed respectivelybetween the multiple cloning sites AgeI/SalI on the lentiviral expression vector pKL-Kan-BB-CAGG-scFv1 by the homologous recombination method well known in the art. After cloning, the sequence information was confirmed by sequencing and named as pKL-Kan-BB-CAGG-Ranibizumab.

The nucleic acid sequence encoding the VEGF binding fragment derived from the VEGFR (i.e. VEGFR D2/3 and Fc) designed in Example 1 was synthesized via Nanjing GenScript Biotech Co., Ltd., and then replacement cloning was performedbetween the multiple cloning sites AgeI/SalI on the lentiviral expression vector pKL-Kan-BB-CAGG-scFv1 by the homologous recombination method well known in the art. After cloning, the sequence information was confirmed by sequencing and named as pKL-Kan-BB-CAGG-Aflibercept.

### Example 3: Construction of AAV vectors comprising genes for anti-VEGF molecules

The gene expression cassette containing the nucleotide sequence encoding the fusion protein binding to VEGF designed in Example 1 and the promoter CAGG were cloned into a AAV vector. The AAV vector backbone is from Kanglin Biotechnology (Hangzhou) Co., Ltd (nucleotide sequence is SEQ ID NO: 24) (see Figure 3).

The gene expression cassette construct 1 containing the nucleotide sequence encoding the fusion protein 1 binding to VEGF designed in Example 1 was synthesized via Nanjing GenScript Biotech Co., Ltd., and then cloning was performedbetween the multiple cloning sites XhoI/Sall on the adeno-associated virus expression vector pAAV-MCS by the homologous recombination method well known in the art. After cloning, the sequence information was confirmed by sequencing and named as pAAV-MCS-CAGG-Fusion Protein 1.

The gene expression cassette constructs 2 to 8 containing the nucleotide sequences encoding the fusion proteins 2 to 8 binding to VEGF designed in Example 1 were synthesized via Nanjing GenScript Biotech Co., Ltd., and then replacement cloning was performed respectivelybetween the multiple cloning sites AgeI/SalI of the adeno-associated virus expression vector pAAV-MCS-CAGG-Fusion Protein 1 by the homologous recombination method well known in the art. After cloning, the sequence information was confirmed by sequencing and named as pAAV-MCS-CAGG-Fusion Proteins 2 to 8, respectively.

The nucleic acid coding sequence of the scFv derived from Ranibizumab designed in Example 1 was synthesized via Nanjing GenScript Biotech Co., Ltd., and then replacement cloning was performed between the multiple cloning sites AgeI/SalI of the adeno-associated virus expression vector pAAV-MCS-CAGG-Fusion protein 1 by the homologous recombination method well known in the art. After cloning, the sequence information was confirmed by sequencing and named as pAAV-MCS-CAGG-Ranibizumab.

The nucleic acid sequence encoding the VEGF binding fragment derived from the VEGFR (i.e. VEGFR D2/3 and Fc) designed in Example 1 was synthesized via Nanjing GenScript Biotech Co., Ltd., and then replacement cloning was performed between the multiple cloning sites AgeI/SalI of the adeno-associated virus expression vector pAAV-MCS-CAGG-Fusion protein 1 by the homologous recombination method well known in the art. After cloning, the sequence information was confirmed by sequencing and named as pAAV-MCS-CAGG-Aflibercept.

### Example 4: Identification of proteins containing genes for anti-VEGF molecules

One of the lentiviral expression vectorscontaining the nucleotide sequence encoding the fusion protein binding to VEGF constructed in Example 2 (pKL-Kan-BB-CAGG-Fusion Protein 1 to pKL-Kan-BB-CAGG-Fusion Protein 8) or the lentiviral expression vector containing the scFv derived from Ranibizumab or the VEGF binding fragment derived from the VEGFR (i.e., VEGFR D2/3 and Fc)(pKL-Kan-BB-CAGG-Ranibizumab or pKL-Kan-BB-CAGG-Aflibercept) were transiently transfected into 293T cells (purchased from the American Type Culture Collection (ATCC), with the accession number CRL-3216). Expression of anti-VEGF molecular gene proteins was performed in this 293T cell line. The transfection method was PEI cationic polymer-mediated transient transfection of eukaryotic cells, and the PEI cationic polymer was PEI-Max transfection reagent purchased from Polysciences (purchased from Polysciences, Cat. No. 24765-1). The transfection operation was carried out with reference to the manufacturer's recommended standardized operation, and the scale of transfection was a 6-well plate for cell culture.

The protein supernatant was harvested 48 hours after completion of transfection. Firstly, cell debris was removed by centrifugation at 4000 rpm for 5 minutes at room temperature on the benchtop swing-bucket centrifuge, and the supernatant was collected and frozen at -80°C for later use.

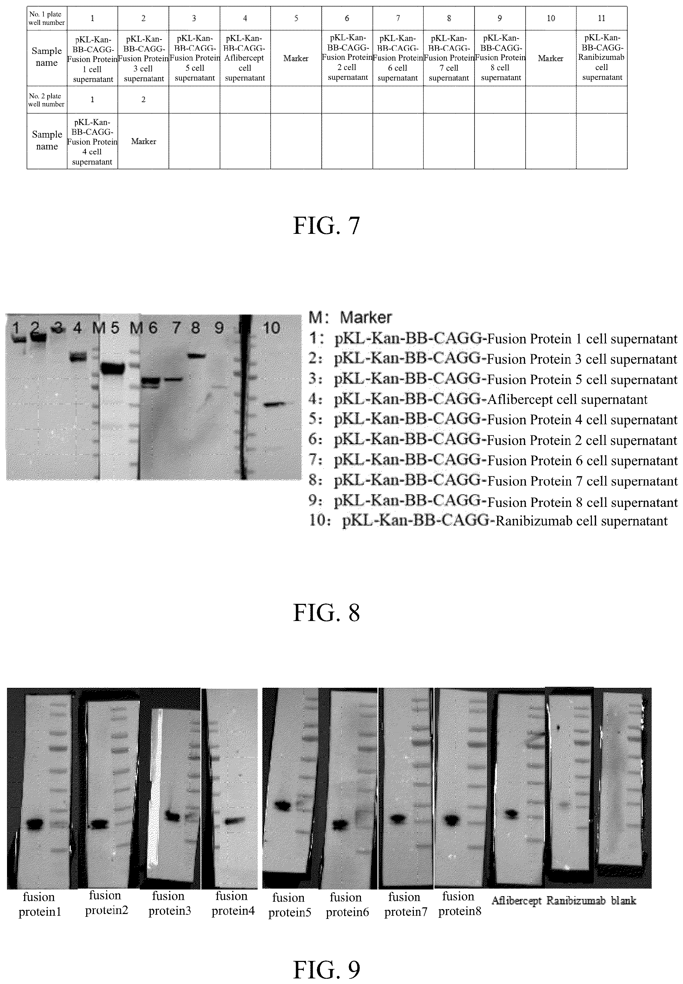

The following two Western Blot identifications of the cell supernatant ware performed by the methods well known in the art:
1. Identification was performed directly using cell supernatant loading, the specific operations were as follows:
   (1) SDS-PAGE gel running
      1 ug fusion protein 3; 5 ug, 1 ug, 0.5 ug fusion protein 4 and fusion protein 7 were taken for protein gel running, respectively. Coomassie Brilliant Blue staining was performed after finishing the gel running, and decolorization was performed overnight after finishing the staining. The results are shown in Figure 6.
   (2) Western Blot Validation
      (1) 40 uL of cell supernatant +10 uL Loading Buffer were taken; diluted fusion protein 3, fusion protein 4, fusion protein 7 samples were taken and mixed well with Loading Buffer, and 20uL of each was loaded, the loading information is shown in Figure 7.
         2) Blocked with 5% skim milk and washed for three times.
         3) The membrane was trimmed according to the marker imprint on the membrane, and different membranes were incubated with different antibodies. Specifically, channels 1-4 were incubated with goat anti-human IgG (Fc) (1:10000); channels 6-10 were incubated with ProteinL-HRP (1:10000); channel 12 was incubated with goat anti-human IgG (Fab) (1:5000) secondary antibody; washed for three times.
         4) Exposure.

The results of the experiment (Fig. 8) showed that the sizes of the proteins obtained from the cell transient transfection were all as expected and were the correct bands.

2. Identification of cell supernatant by loading VEGF165 protein.
1) 10uL VEGF165 protein + 190uL DPBS + 50uL Loading Buffer were taken for sample loading, and the VEGF165 protein used is recombinant human VEGF165 protein (Novoprotein Technology Co., Ltd.; Cat. No. C083), loaded 20 uL of samples were loaded into each well at intervals with the Marker.
2) Blocked with 5% skim milk and washed for three times.
3) The membranes were trimmed according to the marker imprints on the membranes, and each membrane was incubated with 2.5% skim milk containing different cell supernatants as the primary antibody and washed for three times.
4) For the bands whose primary antibodies were the cell supernatants containing pKL-Kan-BB-CAGG-Fusion Protein 1, pKL-Kan-BB-CAGG-Fusion Protein 3, pKL-Kan-BB-CAGG-Fusion Protein 5, and pKL-Kan-BB-CAGG-Aflibercept, goat anti-human IgG (Fc) (1:10000) secondary antibody was used for incubation; for the bands whose primary antibodies were the cell supernatants containing pKL-Kan-BB-CAGG-Fusion Protein 2, pKL-Kan-BB-CAGG-Fusion Protein 4, pKL-Kan-BB-CAGG-Fusion Protein 6, pKL-Kan-BB-CAGG-Fusion Protein 7, and pKL-Kan-BB-CAGG-Fusion Protein 8, Protein L-HRP (1:10000) secondary antibody was used for incubation; for the band whose primary antibodywas the cell supernatant containing pKL-Kan-BB-CAGG-Ranibizumab, goat anti-human IgG (Fab) (1:5000) secondary antibody was used for incubation; washed for three times.
5) Exposure.

The experimental results (Figure 9) showed that all the proteins obtained from cellular transient transfection could bind to VEGF165 protein.

### Example 5: Viral packaging of lentivirus vectors comprising genes for anti-VEGF molecules

One of the lentiviral expression vectorscomprising the nucleotide sequences encoding the fusion proteins binding to VEGF constructed in Example 2 (pKL-Kan-BB-CAGG-Fusion Protein 1 to pKL-Kan-BB-CAGG-Fusion Protein 8) or the lentiviral expression vectorcomprising the scFv derived from Ranibizumab or the VEGF binding fragment derived from the VEGFR( i.e. VEGFR D2/3 and Fc) (pKL-Kan-BB-CAGG-Ranibizumab or pKL-Kan-BB-CAGG-Aflibercept), as well as an envelope plasmid (pKL-Kan-Vsvg, whose nucleotide sequence is as set forth in SEQ ID NO: 27) and a packaging plasmid (pKL-Kan-pLP2, whose nucleotide sequence is as set forth in SEQ ID NO: 25; and pKL-Kan- pLP1, whose nucleotide sequence is as set forth in SEQ ID NO: 26) were used for simultaneous co-transfection of 293T cells (purchased from the American Type Culture Collection (ATCC), with the accession number CRL-3216). Packaging of lentiviral vectors for gene therapy with anti-VEGF molecules was performed in this 293T cell line. The transfection method was PEI cationic polymer-mediated transient transfection of eukaryotic cells, and the PEI cationic polymer was PEI-Max transfection reagent purchased from Polysciences (purchased from Polysciences, Cat. No. 24765-1), the transfection operation was carried out with reference to the manufacturer's recommended standardized operation, and the scale of transfection was 10 cm² cell culture dish.

Lentiviral vectors were harvested 48 hours after completion of transfection. Firstly, cell debris was removed by centrifugatione at 4000 rpm for 5 minutes at room temperature to remove cell debris on the benchtop swing-bucket centrifuge , and the supernatant was then centrifuged at 10000 g for 4 hours at 4°C to obtain viral particle pellet. After removing the supernatant, 1 mL of DMEM complete medium was added to the viral particle pellet, and the viral particles were resuspended with a micro-sampler, and the prepared virus resuspension was dispensed and frozen at -80°C for later use.

The infection titer of the prepared viral resuspension was first determined, i.e., the number of infectious viral vector particles per unit volume of liquid - TU/ml. The principle is that the human CD4+ T cell line (MT4 cell line, purchased from Shanghai Suer Biotechnology Co., Ltd.) was pre-plated in a 96-well plate (1E5 cells/well), and then the gradiently diluted virus resuspension to be tested was added to the 96-well plate to infect MT4 cells, and then the lentiviral vector copy number was detected in target cells by quantitative PCR, the infection efficiency of lentivirus infecting target cells was calculated by substituting the known titer into the standard curve of EGFP reporter gene infection titer/vector copy number.

The standard curve for the infection efficiency of the reporter gene lentiviral vector EGFP (the entiviral vector packaged as pCCL-sin-EF1α-WPRE-EGFP according to the above method) includes measuring the percentage of GFP signal by infection flow cytometry and detecting the VCN of MT4 cells after lentivirus infection by quantitative PCR. Based on the results of flow cytometry GFP expression and the VCN results of qPCR, the linear relationship between transduction efficiency and VCN was plotted, and fitted according to the linear relationship to obtain the relationship equation between the two.

Lentivirus biological titer (TU/mL) = number of cells inoculated (cells) * transduction efficiency (%) * dilution multiple of the lentivirus sample to be tested / volume of lentivirus inoculated during cell infection (mL)

The primer probe sequences used for quantitative PCR are as shown below.

The LTR primer probes amplify a sequence on the 5' LTR gene contained in lentiviruses:
LTR forward primer: CTGTTGTGTGACTCTGGTAACT (SEQ ID NO: 36)
LTR probe: 5'-AAATCTCTAGCAGTGGCGCCCG-3' (SEQ ID NO: 37)
LTR reverse primer: TTCGCTTTCAAGTCCCTGTT (SEQ ID NO: 38).
HK primer probes amplify a sequence of albumin (ALB) contained in the MT4 cell genome:
   HK forward primer: 5'-GCTGTCATCTCTTGTGGGCTGT -3' (SEQ ID NO: 39)
   HK probe: 5' -CCTGTCATGCCCACACAAATCTCTCC -3' (SEQ ID NO: 40)
   HK reverse primer: 5'-ACTCATGGGAGCTGCTGGTTC -3' (SEQ ID NO: 41).

The 5' end of the LTR probe carries a FAM fluorescent group and the 3' end carries an MGB fluorescent group. The 5' end of the HK probe carries a CY5 fluorescent group and the 3' end carries a BHQ2 fluorescent group.

The results of each lentivirus titer are as shown in Table 2.

**Table 2 Titers of lentiviral vectors comprising nucleotide sequences encoding proteins that bind VEGF**

| Lentiviral vector | Titer TU/mL |
|---|---|
| pKL-Kan-BB-CAGG-Ranibizumab | 4.26E+08 |
| pKL-Kan-BB-CAGG-Aflibercept | 6.44E+08 |
| pKL-Kan-BB-CAGG-Fusion Protein 1 | 7.33E+08 |
| pKL-Kan-BB-CAGG-Fusion Protein 2 | 1.50E+09 |
| pKL-Kan-BB-CAGG-Fusion Protein 3 | 4.91E+08 |
| pKL-Kan-BB-CAGG-Fusion Protein 5 | 3.35E+08 |
| pKL-Kan-BB-CAGG-Fusion Protein 4 | 6.00E+08 |
| pKL-Kan-BB-CAGG-Fusion Protein 6 | 4.42E+08 |
| pKL-Kan-BB-CAGG-Fusion Protein 7 | 1.33E+09 |
| pKL-Kan-BB-CAGG-Fusion Protein 8 | 2.79E+09 |

### Example 6: Viral packaging of AAV vectors comprising genes for anti-VEGF molecules

One of the AAV expression vector constructed in Example 3 containing the nucleotide sequence encoding the fusion protein that binds VEGF (pAAV-MCS-CAGG-Fusion Protein 1 to pAAV-MCS-CAGG-Fusion Protein 8) orthe AAV expression vectors comprising nucleotide sequences encoding the scFv derived from Ranibizumab or the VEGF-binding fragments derived from the VEGFR( i.e., VEGFR D2/3 and Fc) (pAAV-MCS-CAGG-Ranibizumab or pAAV-MCS-CAGG-Aflibercept), as well as an AAV serotype plasmid (pAAV-RC2, purchased from CELL BIOLABS, INC., Item No. VPK-422) and a helper plasmid (pAAV-Helper, purchased from CELL BIOLABS, INC.; Item No. VPK-402) were used for simultaneous co-transfection of 293T cells, and packaging of AAV vectors for gene therapy with anti-VEGF molecules was performed in this 293T cell line. The transfection method was PEI cationic polymer-mediated transient transfection of eukaryotic cells, and the PEI cationic polymer was PEI-Max transfection reagent purchased from Polysciences (purchased from Polysciences, Cat. No. 24765-1), the transfection operation was carried out with reference to the manufacturer's recommended standardized operation, and the scale of transfection was 15 cm² cell culture dish.

72 hours after transfection was completed, the cell supernatant and cells were collected into a 50 mL centrifuge tube. First, the cells and the supernatant were separated by centrifugation at 4200 rpm for 10 minutes at room temperature on a benchtop swing-bucket centrifuge. The supernatant was transferred to a new 50 mL centrifuge tube, then added with MgCl₂ and ribozyme, mixed well, and set aside for later use. The remaining cells were added with ribozyme and cell lysis buffer, incubated at room temperature for 1 h, and then centrifuged at 10,000 g for 10 minutes at room temperature. The supernatant was transferred to the original cell supernatant, mixed well, and concentrated by affinity purification. A 5 mL chromatography column was packed with affinity filler (POROS AAVX Affinity Resin, Thermo, Cat. No. A36743) and equilibrated with equilibration buffer (20 mmol/L PB, 0.15 mol/L NaCl, pH 7.4 ± 0.2). After equilibration, the sample was loaded, and the equilibration solution was used to equilibrate for another 5 column volumes after completing the sample loading. The AAV sample of about 8 mL was eluted with elution buffer (0.1 mol/L glycine, 0.15 mol/L NaCl, pH 2 ± 0.1), and concentrated and replaced to 1 mL with a 100KD ultrafiltration tube (Milipore, Catalog No. UFC8100), the AAV solvent was 180 mM NaCl, 10 mM PB, 0.001% F-68 after the solution was replaced. The samples were dispensed after purification and frozen at -80°C for later use.

The physical titer of the AAV viral vector was calculated according to the quantitative PCR results and using AAV standards as reference by methods well known in the art. The AAV standard was rAAV2 of known titer, which contained the gene CMV-EGFP (purchased from ATCC, Cat. No. VR-1616). The AAV standard was lysed with AAV lysis buffer and serially diluted as the standard. The sample AAV was also lysed with lysis buffer and diluted to the range contained in the standard curve based onthe estimated titer, the titer was determined by qPCR and converted to Vg/mL. The detailed steps were as follows:
1. Preparation of standards: 20 uL of aliquoted rAAV2 virus was taken, and the virus samples were lysised at a ratio of virus volume to lysis buffer (QuickExtract DNA Extraction Solution, manufacturer: Lucigen, Cat. No. QE09050) volume = 1:9. After mixing evenly and centrifuging briefly, the following procedure was performed using a gene thermal cycler: 56 °C, 60 min; 95 °C, 10 min; the lysed sample was used as the stock solution, diluted 5 times as the first point of the standard curve, and then diluted 5 times gradiently. The standard curve included 5 points (6.56E+08vg/mL, 1.312E+08vg/mL, 2.624E+07vg/mL, 5.248E+06vg/mL), ans was prepared in advance and frozen in aliquots;
2. Preparation of samples: 20 uL of AAV virus sample was taken and lysed at a ratio of virus volume to lysis buffer volume = 1:9. After mixing evenly and centrifuging briefly, the following procedure was performed using gene amplifier: 56 °C for 60 min; 95 °C for 10 min. Based on the estimated titer, the lysed sample was pre-diluted with purified water (e.g., 10-fold, 100-fold, 1000-fold, etc.) to ensure the diluted sample titer fall within the range of the standard curve;
3. Detection: The AAV common region ITR sequence was detected by qPCR method. The primer probe sequences used are as listed below, the reaction system is shown in Table 3, the reaction procedure is shown in Table 4.

**Table 3 qPCR reaction system (Roche LC480)**

| Ingredient | Volume added (uL) | Note |
|---|---|---|
| 2× Premix | 10 | (Accurate Biology, AG, 11704) |
| Primer Probe Mix | 3.6 | 1.2 uL each |
| Molecular grade water | 0.4 | N/A |
| Template | 6.0 | Diluted virus lysate sample |

**Table 4 qPCR reaction procedure**

| Reaction temperature (°C) | Time | Number of cycles |
|---|---|---|
| 95 | 30 s | 1 |
| 95 | 5 s | 40 |
| 55 | 30 s | |

4. Calculation: the sample titer was calculated based on the sample titer obtained from the standard curve * pre-dilution multiple * 10 (i.e. 10-fold dilution when lysis).

The primer probe sequences used for quantitative PCR are:
ITR forward primer: GGAACCCCTAGTGATGGAGTT (SEQ ID NO: 42)
ITR probe: 5'- CACTCCCTCTCTGCGCGC -3' (SEQ ID NO: 43)
ITR reverse primer: CGGCCTCAGTGAGCGA (SEQ ID NO: 44)
where the 5' end of the ITR probe carries a FAM fluorescent group and the 3' end carries a BHQ1 fluorescent group;

The results of each AAV virus titer are shown in Table 5.

**Table 5 Titers of AAV vectors comprising nucleotide sequences encoding proteins that bind VEGF**

| AAV vectors | Titer Vg/mL |
|---|---|
| AAV-MCS-CAGG-Ranibizumab | 3.09E+13 |
| AAV-MCS-CAGG-Aflibercept | 1.15E+13 |
| AAV-MCS-CAGG-Fusion Protein 1 | 1.32E+13 |
| AAV-MCS-CAGG-Fusion Protein 2 | 1.10E+13 |
| AAV-MCS-CAGG-Fusion Protein 3 | 5.00E+12 |
| AAV-MCS-CAGG-Fusion Protein 4 | 2.13E+13 |
| AAV-MCS-CAGG-Fusion Protein 5 | 2.54E+12 |
| AAV-MCS-CAGG-Fusion Protein 6 | 5.08E+12 |
| AAV-MCS-CAGG-Fusion Protein 7 | 7.51E+11 |
| AAV-MCS-CAGG-Fusion Protein 8 | 5.99E+11 |

### Example 7: Detection of protein expression efficiency and activity after transduction of cells with lentiviral particles containing nucleotide sequences encoding proteins that bind VEGF

Human retinal pigment epithelial cell line ARPE-19 cells were seeded in 48-well cell culture plates, with 5.00E+04 cells per well. After two hours, ARPE-19 cells were infected with lentiviral particles containing different constructs at gradients of MOIs 10, 15, 20, and 40, respectively. The medium was replaced after overnight transduction, and the cell culture supernatant was collected 72 hours and stored at -80°C for detecting protein expression levels in VEGF-luciferase reporter gene cell lines.

The collected cells were washed with PBS and then centrifuged at 4200 rpm for 5 min to collect the cells, resuspended in 20 uL of quick extraction solution (QuickExtract^{™} DNA Extraction Solutionr, purchased from Lucigen, Cat. No. QE09050), and the procedure shown in Table 6 was run with a PCR instrument to lyse the cells and extract total DNA.

**Table 6 Cell lysis procedure**

| Temperature | Time |
|---|---|
| 65°C | 15min |
| 68°C | 15min |
| 95°C | 10min |

The infected lentivirus vector copy number (VCN) of ARPE-19 cells was calculated by quantitative PCR data through methods well known in the art. The primer probe sequences are shown in Example 5, and the specific operations are as follows:
1. After cell lysis, the sample was diluted 10 times with pure water (2 uL+18 uL), and a single-copy cell lysate sample was simultaneously set up (the sample is 293T cells that have been confirmed by sequencing to integrate only one copy of the lentiviral vector).
2. The double-probe qPCR method was used for detection. The LTR gene was a universal sequence for lentivirus, and HK was a universal housekeeping reference gene for human cells, the number of lentiviral vector copies/cell in the starting template could be calculated by the Ct value analysis of the reactions of two pairs of primer probes. The reaction system is shown in Table 7 and the reaction procedure is shown in Table 4.

**Table 7 Preparation of Probe-based qPCR reaction system (20 ul reaction system)**

| **Ingredient** | **Volume added (uL)** | **Note** |
|---|---|---|
| 2x qPCR mix | 10 | (Accurate Biology, AG, 11704) |
| Sterile water | 0.4 | N/A |
| HK Forward primer (10 uM) | 0.8 | N/A |
| HK Reverse primer (10 uM) | 0.8 | N/A |
| HK Probe (2.5 uM) | 0.7 | Cy5 |
| LTR Forward primer (10 uM) | 0.8 | N/A |
| LTR Reverse primer (10 uM) | 0.8 | N/A |
| LTR Probe (2.5 uM) | 0.7 | FAM |
| Cell lysate template (1x) | 5.0 | the stock solution is diluted 10 times |

3. Data analysis: qPCR detection was completed with dual-probe method. The Ct value of LTR (set as Ctl) and the Ct value of HK (set as Cth) can be obtained simultaneously for each sample (well). Assuming that the total number of lentiviral vector copies in the original sample to be tested was x, and the total number of cells was y (for diploid organisms, the number of internal reference genes was 2y).

According to the definition of VCN, it can be seen: ① VCN=x/y;
According to the definition of Ct value, under the ideal condition that the amplification efficiency of two pairs of primers was the same, the following equation holds: ② x*(2^Ctl)=2y*(2^Cth).

By combining the formula ①, ②, we had VCN=2*(2^Cth)/(2^Ctl);
Assuming that the VCN of the single-copy DNA reference was VCN0. To reduce the errors caused by differences in reagents, instruments, operators, etc., the final VCN also needed to be corrected by the VCN of the single-copy reference: VCN=[2*(2^Cth)/(2^Ctl)]/ VCN0.

According to the VCN test results, samples with similar VCN were selected to detect the anti-VEGF protein expression level. The VEGF-luciferase reporter gene cell line GloResponseTM NFAT-Re-luc2P HEK293 Cell line (Promega) was used for detection method. The cell line is in the presence of VEGF protein, and KDR (VEGF receptor) binds to the VEGF , releases energy and prompts the cells to express firefly luciferase protein, and then the fluorescence level is detected with a luciferase detection kit. Anti-VEGF protein may bind to VEGF protein, thereby blocking the VEGF from binding to cells and producing luciferase signals. The VEGF165 protein used was recombinant human VEGF165 protein (Novoprotein Technology Co., Ltd.; Item No. C083). The detection kit used was the firefly luciferasereporter gene detection kit (Beyotime Biotechnology; Cat. No. RG006). The specific experimental steps were as follows:
1. VEGF165 protein was diluted to the appropriate concentration using 1% FBS+DMEM medium;
2. The supernatant of the human retinal pigment epithelial cell line ARPE-19 cells collected as described above was subjected to a gradient dilution and mixed well with an equal volume of the diluted VEGF165 solution, and then incubated for 30 min at room temperature;
3. VEGF-luciferase reporter gene cell line cells were digested by trypsin (TrypLE^{™} Express Enzyme, Manufacturer: Thermo, Cat. No. 12604039) and counted, and the concentration was adjusted to 5E5 cells/mL using 1% FBS+DMEM medium;
4. 80 uL of VEGF-luciferase reporter gene cell line cells (4E4 cells/well) and 20 uL of the mixture in Step 2 were added to each well of a 96-well plate;
5. After 6h of incubation at 37°C, the fluorescence values were detected using a luciferase kit.

Depending on the different binding effects of different anti-VEGF proteins with VEGF, the intensity of the fluorescence level that can be detected was also different. That is, the higher the amount of anti-VEGF protein binding to VEGF, the lower the cell luciferase level.

Viral vector samples with similar VCNs were compared. Samples pKL-Kan-BB-Ranibizumab, pKL-Kan-BB-CAGG-Aflibercept, pKL-Kan-BB-CAGG-Fusion Protein 1 to pKL-Kan-BB-CAGG-Fusion Protein 6 were compared in the same batch, and pKL-Kan-BB-CAGG-Fusion Protein 4 and pKL-Kan-BB-CAGG-Fusion Protein 7 were compared in the same batch, pKL-Kan-BB-CAGG-Fusion Protein 7 and pKL-Kan-BB-CAGG-Fusion Protein 8 were compared in the same batch.

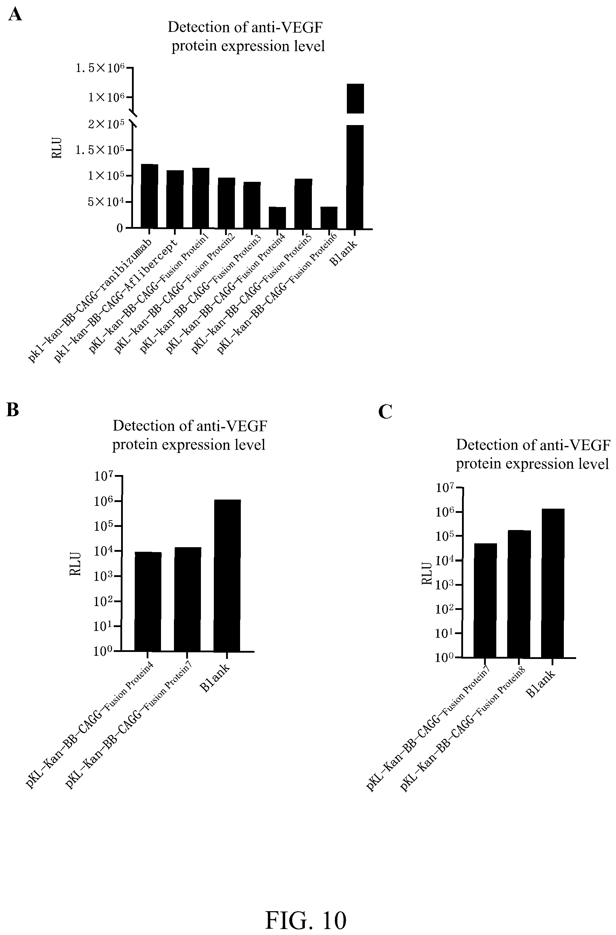

The data results of the activity level of anti-VEGF protein binding to VEGF in the supernatant of ARPE-19 infected with viral particles under similar VCN conditions are shown in Tables 8-1, 8-2, 8-3 and Figure 10.

**Table 8-1. Activity level of VEGF-binding proteins binding to VEGF in the supernatant (the supernatant was diluted 10 times and added to cell wells)**

| | VCN | RLU |
|---|---|---|
| pKL-kan-BB-CAGG-Ranibizumab | 5.66 | 1.23E+05 |
| pKL-kan -BB-CAGG-Aflibercept | 5.00 | 1.11E+05 |
| pKL-kan-BB -CAGG-Fusion Protein 1 | 4.96 | 1.16E+05 |
| pKL-kan-BB -CAGG-Fusion Protein 2 | 3.73 | 9.82E+04 |
| pKL-kan-BB -CAGG-Fusion Protein 3 | 5.13 | 1.36E+05 |
| pKL-kan-BB -CAGG-Fusion Protein 4 | 5.70 | 4.21E+04 |
| pKL-kan-BB -CAGG-Fusion Protein 5 | 4.83 | 9.58E+04 |
| pKL-kan-BB -CAGG-Fusion Protein 6 | 5.24 | 4.23E+04 |
| Blank | N/A | 1.26E6 |

| | | |
|---|---|---|
| Note: In the table, Blank referred to the cell supernatant without anti-VEGF protein (the cell supernatant not infected with the virus), and the rest were consistent with the experimental group, served as blank controls. The same below. | | |

**Table 8-2 Activity level of VEGF-binding proteins binding to VEGF in the supernatant (the supernatant was diluted 5 times and added to cell wells)**

| | VCN | RLU |
|---|---|---|
| pKL-Kan-BB-CAGG-Fusion Protein 4 | 4.17 | 9.30E+03 |
| pKL-Kan-BB-CAGG-Fusion Protein 7 | 4.14 | 1.44E+04 |
| Blank | N/A | 1.24E+06 |

**Table 8-3 Activity level of VEGF-binding proteins binding to VEGF in the supernatant (the supernatant was diluted 5 times and added to cell wells)**

| | VCN | RLU |
|---|---|---|
| pKL-Kan-BB-CAGG-Fusion Protein 7 | 1.70 | 5.09E+04 |
| pKL-Kan-BB-CAGG-Fusion Protein 8 | 1.86 | 1.81E+05 |
| Blank | N/A | 1.42E+06 |

The data from three batches of experiments showed that under similar VCN conditions, the anti-VEGF fusion proteins of the present application exhibited excellent neutralization effects, especially pAAV-MCS-CAGG-Fusion Protein 4, pAAV-MCS-CAGG-Fusion Protein 6 and pAAV-MCS-CAGG-Fusion Protein 7. The design of the fusion protein expression cassette of the present application significantly improved the expression level of the anti-VEGF protein in the culture supernatant of cells transduced by the lentivirus.

### Example 8: Detection of protein expression efficiency and activity after transduction of ARPE-19 cells with AAV viral particles containing nucleotide sequences encoding proteins that bind to VEGF

ARPE-19 cells were seeded in 48-well cell culture plates with 5.00E+04 cells per well. Two hours later, ARPE-19 cells were infected with AAV viral particles of different constructs at a MOI of 75,000. The medium was replaced after overnight transduction. Cell culture supernatant was collected after 72 hours and frozen at -80°C. It was used in VEGF-luciferase reporter gene cell lines to detect protein expression levels. For details, see Example 7.

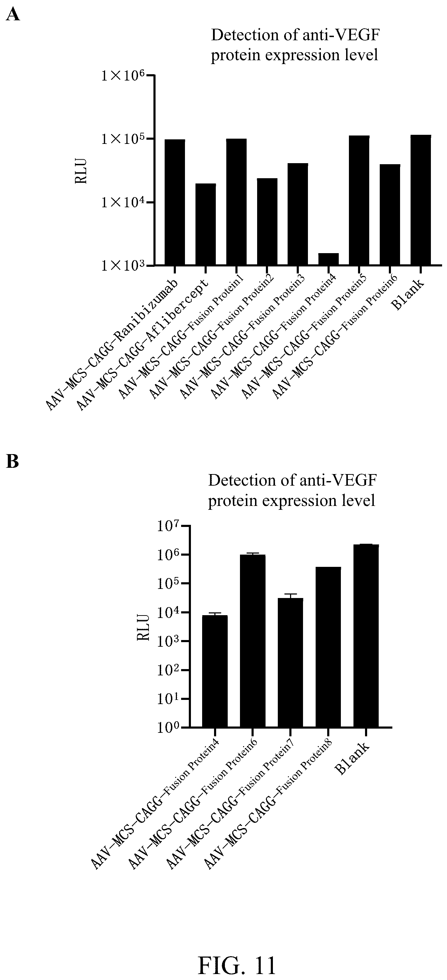

The AAV viral vectors of AAV-MCS-CAGG-Fusion Protein 1 to AAV-MCS-CAGG-Fusion Protein 6, as well as AAV-MCS-CAGG-Ranibizumab and AAV-MCS-CAGG-Aflibercept were compared in the same batch, AAV-MCS-CAGG-Fusion Protein 4, AAV-MCS-CAGG-Fusion Protein 6, AAV-MCS-CAGG-Fusion Protein 7, AAV-MCS-CAGG-Fusion Protein 8 were compared in the same batch. The data on the activity level of anti-VEGF protein binding to VEGF in the supernatant obtained by inoculating the ARPE-19 with different viral particles at the same MOI are shown in Table 9-1, Table 9-2, and Figure 11.

**Table 9-1 Activity level of anti-VEGF protein binding to VEGF in the supernatant (supernatant stock solution was added to cell wells for reaction)**

| Sample name | RLU | |
|---|---|---|
| AAV-MCS-CAGG-Ranibizumab | 7.82E+04 | 1.18E+05 |
| AAV-MCS-CAGG-Aflibercept | 1.95E+04 | 1.99E+04 |
| AAV-MCS-CAGG-Fusion Protein 1 | 1.14E+05 | 8.67E+04 |
| AAV-MCS-CAGG-Fusion Protein 2 | 3.06E+04 | 1.69E+04 |
| AAV-MCS-CAGG-Fusion Protein 3 | 4.16E+04 | 4.06E+04 |
| AAV-MCS-CAGG-Fusion Protein 4 | 1.41E+03 | 1.73E+03 |
| AAV-MCS-CAGG-Fusion Protein 5 | 1.25E+05 | 1.03E+05 |
| AAV-MCS-CAGG-Fusion Protein 6 | 4.15E+04 | 3.80E+04 |
| Blank | 1.16E+05 | 1.16E+05 |

**Table 9-2 Activity level of anti-VEGF proteins binding to VEGF in the supernatant (supernatant stock solution was added to cell wells for reaction)**

| Sample name | RLU | |
|---|---|---|
| AAV-MCS-CAGG-Fusion Protein 4 | 9.11E+03 | 6.69E+03 |
| AAV-MCS-CAGG-Fusion Protein 6 | 8.93E+05 | 1.10E+06 |
| AAV-MCS-CAGG-Fusion Protein 7 | 2.40E+04 | 3.97E+04 |
| AAV-MCS-CAGG-Fusion Protein 8 | 3.79E+05 | 3.79E+05 |
| Blank | 2.15E+06 | 2.30E+06 |

The data from the two experiments showed that under the same MOI (7.5E+04) condition, the anti-VEGF fusion protein of the present application showed excellent neutralization effect, especially pAAV-MCS-CAGG-Fusion Protein 4 and pAAV-MCS-CAGG-Fusion Protein 7. The design of the fusion protein expression cassette of the present application significantly improved the activity level of the anti-VEGF protein binding to VEGFR in the culture supernatant of cells transduced by the AAV virus.

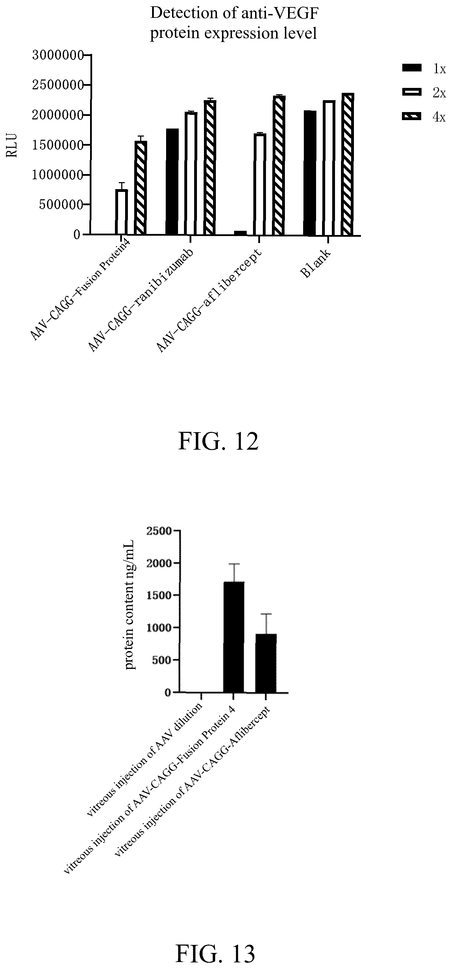

In addition, under the same MOI condition, the expression levels of AAV-CAGG-Fusion Protein 4, AAV-CAGG-Ranibizumab and AAV-CAGG-Aflibercept were compared according to the different dilution multiple of the supernatant to reach the same level of RLU. As shown in Table 10 and Figure 12.

**Table 10 Detection of anti-VEGF protein expression level**

| | MOI | RLU (1x) | RLU (2x) | RLU (4x) |
|---|---|---|---|---|
| AAV-CAGG-Fusion Protein 4 | 7.50E+04 | 1.41E+04 | 8.39E+05 | 1.63E+06 |
| | | 1.22E+04 | 6.89E+05 | 1.52E+06 |
| AAV-CAGG-Ranibizumab | | 1.77E+06 | 2.07E+06 | 2.23E+06 |
| | | 1.78E+06 | 2.05E+06 | 2.28E+06 |
| AAV-CAGG-Aflibercept | | 6.27E+04 | 1.72E+06 | 2.33E+06 |
| | | 7.46E+04 | 1.70E+06 | 2.35E+06 |
| Blank | N/A | 2.09E+06 | 2.27E+06 | 2.39E+06 |

The above results showed that the RLU was at the same level when AAV-CAGG-Fusion Protein 4 was diluted 4-fold, AAV-CAGG-Ranibizumab was the stock solution, and AAV-CAGG-aflibercept were diluted 2-fold. Therefore, it can be assumed that the ratio of the VEGFR binding activity levels of fusion protein 4: Ranibizumab: Aflibercept was 4:1:2. It can be seen that synergistic effects were achieved by transducing visual cells with an AAV vector containing construct 4 of the fusion protein 4 of VEGFR D2/3 and Ranibizumab.

### Example 9: Detection of protein expression level and activity for pAAV-CAGG-Fusion Protein 4 virus vector in animal experiment

Animal experiments were conducted on ordinary wild-type SD rats, and single monocular administration of subretinal or intravitreal injection of pAAV-CAGG-Fusion Protein 4 viral vector was performed (see Table 11 for details), among which an AAV dilution solution (composition: 180 mM NaCl, 10 mM PB, 0.001% F68) group was set as the control group. After completion of dosing, the animals were executed at 4 weeks and both eyes were collected and dissected to obtain vitreous humor for ELISA and cell line detection.

The animals were randomly divided into 3 groups and administered according to Table 11:

**Table 11 Grouping of animals and administration**

| Grouping | Test item | Mode and frequency of dosing | Dosage | Dosing volume (uL) | Number of animals |
|---|---|---|---|---|---|
| 1 | pAAV-CAGG-Fusion Protein 4 viral vector | vitreous injection single monocular dosing | 5E10Vg/eye | 5 | 3 |
| 2 | pAAV-CAGG-Aflibercept viral vector | | 5E10Vg/eye | 5 | 3 |
| 4 | AAV dilution | | NA | 5 | 1 |

During the entire experiment, ELISA and cell line tests were performed on the expression level of the target protein (see Figure 13, Table 12). The results showed that the expression level of AAV-CAGG-Fusion Protein 4 was higher than that of AAV-CAGG-Aflibercept, and both of them had high protein activity levels in cell line test when diluted to 100 ng/mL.

**Table 12 Detection of expression level and protein activity in rat vitreous**

| Groups | | Target protein detection results | | | | | |
|---|---|---|---|---|---|---|---|
| Vitreous injection of AAV dilution | Protein content ng/mL | < Minimum value | < Minimum value | | | | |
| | Cell line detection results RLU | 8.30E+04 | 7.69E+04 | | | | |
| Vitreous injection of AAV-CAGG-Fusion Protein 4 | Protein content ng/mL | 1641.6 | 1920.2 | 1529.2 | 2188 | 1549.2 | 1481 |
| | Cell line detection results RLU | 1.64E+03 | 1.64E+03 | 1.96E+0 3 | 1.79E+ 03 | 1.60E+ 03 | 1.73E+ 03 |
| Vitreous injection of AAV-CAGG-Afliberc ept | Protein content ng/mL | 1027.4 | 1216.8 | 1155.2 | 830.2 | 360.2 | 919.6 |
| | Cell line detection results RLU | 1.78E+03 | 2.54E+03 | 1.60E+0 3 | 2.27E+ 03 | 2.24E+ 03 | 1.47E+ 03 |

### Example 10: The therapeutic effect of pAAV-CAGG-Fusion Protein 4 viral vector on AMD model animals

4 w before dosing of pAAV-CAGG-Fusion Protein 4 viral vector (Week-4), DL-α-AAA was injected into the vitreous cavity of Dutch rabbits to induce a retinal neovascularization model. According to the modeling results during the adaptation period, animals successful modeled with retinal neovascularization modeling were screened and a single monocular administration of subretinal or intravitreal injection of pAAV-CAGG-Fusion Protein 4 viral vector, aflibercept intraocular injection solution (source: VeTTER Pharama-Fertigung GmbH & Co.KG, batch number: KT0A3F5, specification: >100 uL/bottle, expiration date: 2023.03.25), and AAV-CAGG-EGFP injection was performed. After completion of dosing, 50 uL of aqueous humor was collected from both eyes every week to test the protein content, 100 uL of vitreous was collected from both eyes every month to test the protein content, and FFA examination was performed every two weeks.

The specific protocol is as shown in Table 13.

**Table 13 Main research schedule**

| DL-α-AAA induced retinal neovascularization model | Test drug injection | Collection of aqueous humor | Collection of vitreous | FFA examination |
|---|---|---|---|---|
| Week-4 | Week 0 | Week 1~10 | Week 4, 10 | Week 2, 4, 6, 8, 10 |

The animals were given 15 mg/kg diluted cyclosporine A oral solution for 3 consecutive days before dosing, and intragastric administration of 15 mg/kg diluted cyclosporine A oral solution was given to the animals everyday until D57 after dosing. Considering that intravitreal injection and subretinal injection may cause ocular inflammation, care was provided for three consecutive days after dosing, and tobramycin dexamethasone eye drops (source: s.a. ALCON-COUVREUR n.v., batch number: 21I20GB, specification: 5 mL, expiration date: 2023.08.31), ofloxacin eye ointment (source: Shenyang Xingqi Eye Pharmaceutical Co., Ltd., batch number: 211201, specification: 3.2 g/tube, expiration date: 2023.11.01) and levofloxacin eye drops (source: SANTEN PHARMACEUTICAL CO., LTD. NOTO PLANT, batch number: CV2099, specification: 5 mL, expiration date: 2023.05.31) were given. Considering the drug expression, after the administration, 1 drop of tobramycin dexamethasone eye drops was dripped into each eye of the animal (starting from D16), and the drops were dripped every 4 hours for a total of 3 times/day until D56.

The animals were randomly divided into 3 groups and administered according to Table 14.

**Table 14 Grouping of animals and administration**

| Grouping | Test item | Mode and frequency of dosing | Dosage | Dosing volume (uL) | Number of animals and their numbers |
|---|---|---|---|---|---|
| 1 | pAAV-CAGG-Fusion Protein 4 viral vector | Subretinal cavity injection, single monocular administration | 5E11Vg /eye | 20 | 1 (1101#) |
| 2 | pAAV-CAGG-Fusion Protein 4 viral vector | Vitreous injection, single monocular administration | 5E11Vg /eye | 50 | 2(2101#, 2102#) |
| 3 | Afliberce pt intraocula r injection solution | Vitreous injection, single monocular administration | 500 ug/eye | 50 | 1 (3101#) |
| 4 | AAV-CAGG-EGFP group | Vitreous injection, single monocular administration | 5E11Vg /eye | 50 | 1 (4101#) |

Before administration, it was confirmed by FFA angiography that obvious retinal vascular dilation, tortuosity and leakage were observed in both eyes of all the animals in the group (see Table 15).

**Table 15 FFA Angiography Summary Score Sheet**

| Animal number | adaptation period | | W2 | | W4 | | W6 | | W8 | | W10 | |
|---|---|---|---|---|---|---|---|---|---|---|---|---|
| | OD | OS | OD | OS | OD | OS | OD | OS | OD | OS | OD | OS |
| 1101 | 6 | 2 | 0 | 3 | 0 | 3 | 0 | 3 | 0 | 3 | 0 | 8 |
| 2101 | | 6 | 0 | 8 | 0 | 8 | 0 | 8 | 0 | 8 | 0 | 8 |
| 2102 | 4 7 | 2 | 0 | 4 | 0 | 4 | 0 | | 0 | 4 | 0 | 6 |
| 3101 | 4 | 4 | 2 | 2 | 2 | 2 | 2 | 4 | 6 | 5 | - | 6 |
| 4101 | 2 | 2 | 2 | 2 | 2 | 3 | 4 | 4 | 6 | 4 | 6 | 8 |

| | | | | | | | | | | | | |
|---|---|---|---|---|---|---|---|---|---|---|---|---|
| Notes: 1. OS: left eye; OD: right eye. 2. -: No result was collected. 3. Scoring method: the eye was divided into 8 areas (see Figure 14 for specific divisions) and scored separately. The scoring criteria for each area were as follows: symptoms of straight large blood vessels, tortuous small blood vessels, and no vascular dilation, scored as 0; symptoms of increased tortuosity of large blood vessels and/or partial vascular dilation, scored as 1; symptoms of leakage between large blood vessels and obvious vascular dilation, scored as 2; symptoms of leakage between large and small blood vessels, with small blood vessels still visible, scored as 3; symptoms of leakage between large and small blood vessels, with poor/invisible small blood vessels, scored as 4. After scoring each area, the scores were added up to get the final score. | | | | | | | | | | | | |

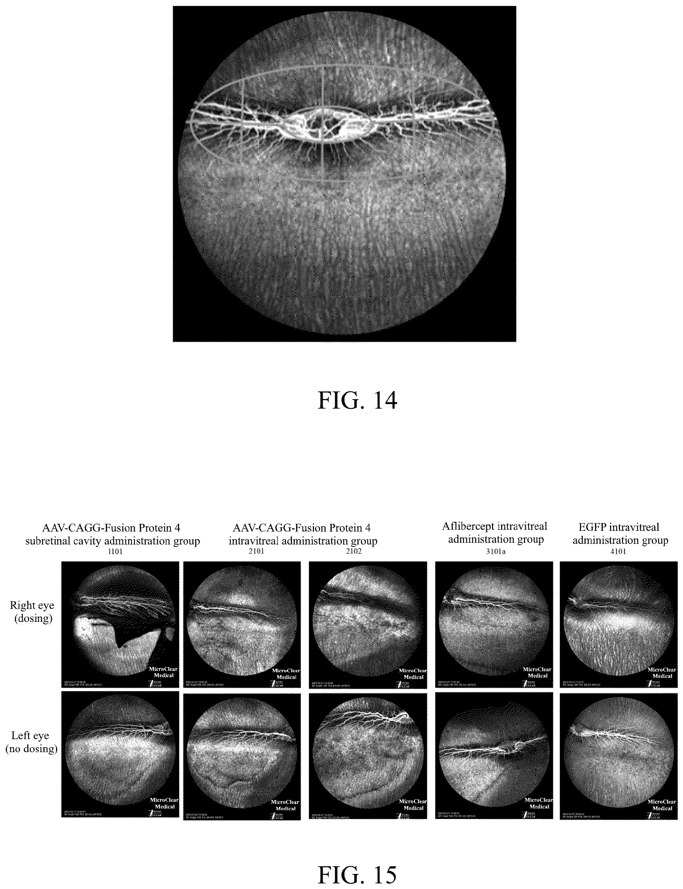

The experimental results (see Figure 15 and Table 15) showed that 2 weeks after administration, the retinal vascular dilation, tortuosity and leakage of the right eyes (drug-treated eyes) of 1101#, 2101# and 2102# disappeared, and the leakage level basically returned to the level before modeling, and this level was maintained until the end of the experimental period. In the right eye of 3101# (drug-treated eyes), retinal vascular dilation and tortuosity were still observed, but the degree of dilation and tortuosity was significantly reduced compared with that before drug administration. By the fourth week after drug administration, the degree of dilation, tortuosity and leakage had basically returned to the level before modeling. However, in the sixth week after administration, it was found that the retinal blood vessels were dilated, tortuous and leaky again, which was mainly related to the pharmacokinetics, absorption and distribution of aflibercept. In the right eye of 4101# (drug-treated eyes), retinal vascular dilation, tortuosity and leakage were observed throughout the experimental period. During the entire observation period, the degree of vascular dilation, tortuosity and leakage in the left eyes (non-drug-treated eyes) of the four groups of animals increased over time.

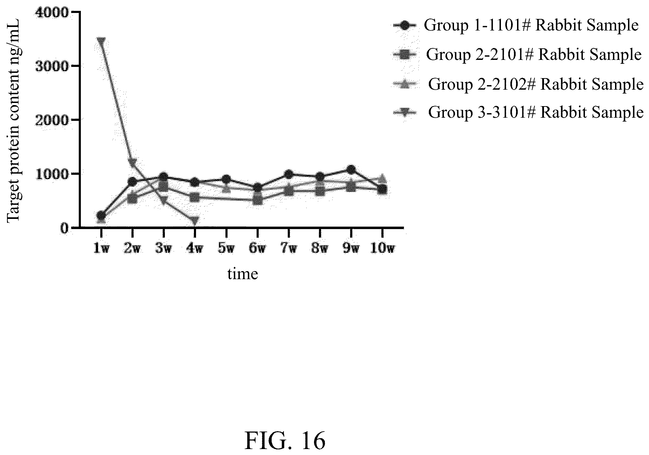

Throughout the experiment, the expression level of the target protein was detected by ELISA (see Figure 16, Table 16).

**Table 16 ELISA test results at different time points**

| Target protein content ng/mL | Group 1-1101# rabbit sample | Group 2-2101# rabbit sample | Group 2-2102# rabbit sample | Group 3-3101# rabbit sample | Group 4-4101# rabbit sample |
|---|---|---|---|---|---|
| 1w | 235.60 | / | 175.50 | 3448.50 | < Min |
| 2w | 860.10 | 549.30 | 626.00 | 1195.00 | < Min |
| 3w | 950.00 | 766.40 | 945.40 | 507.60 | < Min |
| 4w | 852.10 | 570.90 | 867.90 | 125.10 | < Min |
| 5w | 907.8 | 354.4 | 749.2 | < Min | < Min |
| 6w | 754.8 | 514.4 | 705.8 | < Min | < Min |
| 7w | 998.3 | 685.7 | 766.6 | < Min | < Min |
| 8w | 954 | 689.4 | 879.3 | < Min | < Min |
| 9w | 1084 | 761.9 | 849.9 | < Min | < Min |
| 10w | 731.2 | 710.7 | 928.7 | < Min | < Min |

The experimental results showed that the pAAV-CAGG-Fusion Protein 4 viral vector can maintain long-term high-level expression of the target protein in the AMD animal model, significantly inhibit the dilation, tortuosity and leakage of retinal blood vessels, which was consistent with the early effects of the finished drug aflibercept intraocular injection solution, indicating that the pAAV-CAGG-Fusion Protein 4 viral vector had a significant therapeutic effect on AMD.

### SEQUENCE LISTING

| S^{E}Q ID NO | Name | Sequence |
|---|---|---|
| 1 | Amino acid sequence of Brolucizumab | EIVMTQSPSTLSASVGDRVIITCQASEIIHSWLAWYQQKPG KAPKLLIYLASTLASGVPSRFSGSGSGAEFTLTISSLQPDDF ATYYCQNVYLASTNGANFGQGTKLTVLGGGGGSGGGGS GGGGSGGGGSEVQLVESGGGLVQPGGSLRLSCTASGFSL TDYYYMTWVRQAPGKGLEWVGFIDPDDDPYYATWAKG RFTISRDNSKNTLYLQMNSLRAEDTAVYYCAGGDHNSG WGLDIWGQGTLVTVSS |
| 2 | Nucleotide sequence encoding Brolucizumab | GAGATCGTGATGACACAGAGCCCCAGCACACTGTCCG CCAGCGTGGGCGACAGAGTGATCATCACCTGTCAGGCT TCTGAAATCATCCACTCTTGGCTGGCCTGGTACCAGCA GAAGCCCGGTAAAGCCCCTAAACTGCTGATCTACCTGG CTTCTACCCTGGCTAGCGGCGTTCCTAGCCGGTTTAGC GGATCCGGAAGCGGTGCTGAGTTCACACTGACCATTTC TAGCCTCCAGCCTGATGACTTCGCCACCTACTATTGCC AGAACGTGTACCTCGCCTCTACTAACGGCGCCAATTTC GGCCAAGGCACAAAGCTGACCGTGCTGGGCGGCGGCG GCGGCAGCGGCGGAGGCGGCAGCGGCGGAGGCGGCA GCGGCGGCGGCGGCTCCGAGGTGCAGCTGGTCGAGAG CGGCGGAGGGCTGGTGCAACCAGGAGGCTCTCTGAGA CTGAGCTGCACCGCCAGCGGATTCAGCCTGACAGATTA CTATTACATGACCTGGGTCAGACAGGCCCCTGGCAAGG GCCTGGAATGGGTGGGATTTATCGACCCCGACGACGAT CCTTACTACGCCACATGGGCCAAGGGCAGATTCACCAT CAGCAGAGATAACTCTAAGAACACCCTGTACCTGCAG ATGAACAGCCTGCGGGCCGAGGACACCGCCGTGTACT ACTGCGCCGGCGGCGACCACAATTCCGGCTGGGGCCTG GACATCTGGGGACAGGGCACCCTGGTGACCGTGTCCA |
| | | GC |
| 3 | Amino acid sequence of Ranibizumab | EVQLVESGGGLVQPGGSLRLSCAASGYDFTHYGMNWVR QAPGKGLEWVGWINTYTGEPTYAADFKRRFTFSLDTSKS TAYLQMNSLRAEDTAVYYCAKYPYYYGTSHWYFDVWG QGTLVTVSSASTKGPSVFPLAPSSKSTSGGTAALGCLVKD YFPEPVTVSWNSGALTSGVHTFPAVLQSSGLYSLSSVVTV PSSSLGTQTYICNVNHKPSNTKVDKKVEPKSCDKTHLRK RRAPVKQTLNFDLLKLAGDVESNPGPMDMRVPAQLLGL LLLWLSGARCDIQLTQSPSSLSASVGDRVTITCSASQDISN YLNWYQQKPGKAPKVLIYFTSSLHSGVPSRFSGSGSGTDF TLTISSLQPEDFATYYCQQYSTVPWTFGQGTKVEIKRTVA APSVFIFPPSDEQLKSGTASVVCLLNNFYPREAKVQWKVD NALQSGNSQESVTEQDSKDSTYSLSSTLTLSKADYEKHK VYACEVTHQGLSSPVTKSFNRGEC |
| 4 | Nucleotide sequence encoding Ranibizumab | GAGGTCCAGCTGGTGGAGTCCGGCGGCGGGCTCGTGC AGCCGGGGGGCTCCCTGCGCCTGTCCTGCGCCGCGAGC GGCTACGACTTCACGCACTACGGGATGAACTGGGTCCG CCAGGCGCCCGGCAAGGGCCTCGAGTGGGTCGGGTGG ATCAACACCTACACGGGCGAGCCCACGTACGCCGCGG ACTTCAAGAGGCGCTTCACCTTCAGCCTGGACACGAGC AAGTCGACGGCGTACCTTCAGATGAACTCCCTGCGGGC GGAGGACACCGCGGTCTACTACTGCGCGAAGTACCCGT ACTACTACGGCACCTCCCACTGGTACTTCGACGTGTGG GGCCAGGGCACCCTCGTCACGGTCAGCTCCGCGAGCAC AAAGGGTCCCAGCGTGTTCCCGCTGGCCCCCAGCTCGA AGAGCACCAGCGGGGGGACCGCCGCCCTCGGGTGCCT CGTGAAGGACTACTTCCCGGAACCCGTCACCGTCAGCT GGAACAGCGGCGCCCTCACCTCCGGCGTGCACACCTTC CCGGCCGTCCTGCAGAGCAGCGGGCTGTACAGCCTCAG CAGCGTCGTGACTGTGCCCTCCAGCTCGCTAGGGACGC AGACCTACATCTGCAACGTCAACCACAAGCCAAGCAA |
| | | CACCAAGGTCGACAAGAAGGTGGAGCCCAAGTCATGC GACAAGACCCACCTGCGGAAACGGCGGGCCCCCGTCA AGCAGACCCTGAACTTCGACCTGCTCAAGCTAGCGGGC GACGTAGAGAGCAACCCGGGCCCCATGGACATGCGGG TCCCCGCCCAGCTGCTCGGGCTGCTCCTACTCTGGCTC AGCGGGGCGCGTTGCGACATCCAGCTCACGCAGAGCC CCAGTAGCCTGAGCGCCAGCGTGGGCGACCGGGTGAC CATCACGTGCTCCGCCAGCCAGGACATCAGCAACTACC TCAACTGGTACCAGCAGAAGCCCGGGAAGGCCCCGAA GGTGCTCATCTACTTCACGAGCAGCCTGCACAGCGGGG TGCCCTCCCGCTTCAGCGGGAGCGGGTCCGGGACAGAC TTCACGCTCACCATCTCCAGCCTGCAGCCCGAGGACTT CGCGACCTACTACTGCCAGCAGTACAGCACGGTGCCCT GGACCTTCGGCCAGGGGACCAAGGTCGAGATCAAGCG CACGGTGGCGGCGCCCAGCGTCTTCATCTTCCCCCCGA GCGACGAGCAGCTCAAGAGCGGCACCGCGAGCGTCGT GTGCCTTCTCAACAACTTCTACCCCCGGGAGGCGAAGG TCCAGTGGAAGGTGGACAACGCCCTCCAGTCCGGGAA CAGCCAGGAGAGCGTGACCGAGCAGGACAGCAAGGAC AGCACGTACAGCCTCAGCTCCACGCTCACCCTCTCCAA GGCCGACTACGAGAAGCACAAGGTGTACGCGTGCGAG GTCACCCACCAGGGGCTGTCGAGCCCGGTGACGAAGA GCTTCAACCGAGGGGAGTGC |
| 5 | Amino acid sequence of D2/3 of VEGFR | SDTGRPFVEMYSEIPEIIHMTEGRELVIPCRVTSPNITVTLK KFPLDTLIPDGKRIIWDSRKGFIISNATYKEIGLLTCEATVN GHLYKTNYLTHRQTNTIIDVVLSPSHGIELSVGEKLVLNC TARTELNVGIDFNWEYPSSKHQHKKLVNRDLKTQSGSEM KKFLSTLTIDGVTRSDQGLYTCAASSGLMTKKNSTFVRV HEK |
| 6 | Nucleotide sequence encoding D2/3 of VEGFR | TCCGACACAGGCAGACCCTTCGTGGAGATGTATTCTGA GATCCCTGAGATCATCCACATGACCGAGGGCAGGGAG |
| | | CTGGTCATCCCATGTCGCGTGACAAGCCCCAACATCAC CGTGACACTGAAGAAGTTCCCTCTGGACACCCTGATCC CAGATGGCAAGCGGATCATCTGGGACAGCAGAAAGGG CTTTATCATCTCCAATGCCACATACAAGGAGATCGGCC TGCTGACCTGCGAGGCCACAGTGAACGGCCACCTGTAC AAGACCAATTATCTGACACACAGACAGACCAACACAA TCATCGATGTGGTGCTGAGCCCATCCCACGGCATCGAG CTGTCCGTGGGCGAGAAGCTGGTGCTGAATTGTACCGC CAGGACAGAGCTGAACGTGGGCATCGACTTCAATTGG GAGTACCCCTCTAGCAAGCACCAGCACAAGAAGCTGG TGAACAGGGATCTGAAGACCCAGTCTGGCAGCGAGAT GAAGAAGTTTCTGAGCACCCTGACAATCGACGGAGTG ACCCGCTCCGATCAGGGACTGTATACATGCGCCGCCTC CTCTGGCCTGATGACCAAGAAGAATTCCACATTCGTGC GGGTGCACGAGAAG |
| 7 | Amino acid sequence of fusion protein 1 | EIVMTQSPSTLSASVGDRVIITCQASEIIHSWLAWYQQKPG KAPKLLIYLASTLASGVPSRFSGSGSGAEFTLTISSLQPDDF ATYYCQNVYLASTNGANFGQGTKLTVLGGGGSGGGGS GGGGSGGGGSEVQLVESGGGLVQPGGSLRLSCTASGFSL TDYYYMTWVRQAPGKGLEWVGFIDPDDDPYYATWAKG RFTISRDNSKNTLYLQMNSLRAEDTAVYYCAGGDHNSG WGLDIWGQGTLVTVSSGGGGSGGGGSGGGGSGGGGSGG GGSDIQLTQSPSSLSASVGDRVTITCSASQDISNYLNWYQ QKPGKAPKVLIYFTSSLHSGVPSRFSGSGSGTDFTLTISSLQ PEDFATYYCQQYSTVPWTFGQGTKVEIKGGGGSGGGGSG GGGSGGGGSEVQLVESGGGLVQPGGSLRLSCAASGYDFT HYGMNWVRQAPGKGLEWVGWINTYTGEPTYAADFKRR FTFSLDTSKSTAYLQMNSLRAEDTAVYYCAKYPYYYGTS HWYFDVWGQGTLVTVSSEPKSCDKTHTCPPCPAPELLGG PSVFLFPPKPKDTLMISRTPEVTCVVVDVSHEDPEVKFNW YVDGVEVHNAKTKPREEQYNSTYRVVSVLTVLHQDWLN |
| | | GKEYKCKVSNKALPAPIEKTISKAKGQPREPQVYTLPPSR DELTKNQVSLTCLVKGFYPSDIAVEWESNGQPENNYKTT PPVLDSDGSFFLYSKLTVDKSRWQQGNVFSCSVMHEALH NHYTQKSLSLSPG |
| 8 | Nucleotide sequence encoding fusion protein 1 | GAGATCGTGATGACCCAGAGCCCTAGCACACTGAGCG CCTCTGTGGGAGATAGAGTGATCATCACCTGTCAGGCC TCTGAGATCATCCACAGCTGGCTGGCTTGGTACCAGCA AAAGCCTGGGAAGGCCCCTAAACTGCTGATCTATCTCG CTAGCACACTGGCCTCTGGAGTCCCCAGCCGGTTTAGC GGAAGCGGCTCTGGCGCCGAATTCACCCTGACAATCAG CAGCCTGCAACCCGATGACTTTGCCACATACTACTGTC AGAACGTGTACCTGGCTAGCACCAACGGCGCCAACTTC GGCCAGGGCACAAAGCTGACCGTGCTTGGAGGCGGCG GCGGCTCCGGCGGAGGCGGAAGCGGCGGCGGCAG CGGCGGGGGCGGCAGCGAGGTGCAGCTGGTGGAGTCG GGCGGCGGCCTGGTGCAGCCAGGAGGTTCTCTGAGGCT GAGCTGCACCGCAAGCGGCTTCAGCCTGACCGACTACT ACTATATGACATGGGTCAGACAGGCCCCTGGAAAGGG CCTGGAATGGGTCGGATTTATCGACCCTGACGACGATC CTTACTACGCCACTTGGGCCAAGGGCAGATTTACCATT TCCAGAGATAACTCTAAGAACACCCTGTACCTGCAAAT GAACAGCCTGCGCGCCGAGGATACAGCCGTGTACTACT GCGCAGGCGGCGACCATAATTCAGGATGGGGCCTGGA TATCTGGGGCCAGGGCACGCTGGTGACCGTGTCCTCCG GCGGAGGAGGCTCTGGCGGAGGCGGCAGCGGCGGCGG CGGCTCCGGCGGCGGCGGCAGCGGCGGTGGCGGCAGC GACATCCAGCTGACACAGTCCCCAAGCAGCCTGAGCG CTTCTGTGGGCGACAGAGTGACCATCACATGCAGCGCC TCTCAGGACATCAGTAACTACCTGAACTGGTACCAGCA GAAACCTGGTAAGGCCCCCAAGGTGCTGATCTACTTCA CCAGCAGCCTGCACTCTGGCGTGCCTAGCAGATTCAGC |
| | | GGCAGCGGATCTGGCACAGACTTCACCCTGACCATTAG CTCCCTGCAGCCTGAGGACTTCGCCACATACTACTGCC AGCAGTACTCCACCGTTCCCTGGACCTTCGGCCAGGGG ACCAAGGTGGAGATCAAGGGGGGCGGCGGCAGCGGAG GAGGCGGAAGCGGCGGGGGCGGATCTGGAGGAGGAG GTAGCGAAGTGCAGCTGGTGGAAAGCGGGGGCGGCCT GGTGCAACCTGGCGGCAGCCTCAGACTGAGCTGCGCC GCTAGCGGATATGATTTCACACACTACGGCATGAATTG GGTGCGGCAGGCTCCTGGCAAGGGCCTGGAATGGGTG GGCTGGATCAATACCTACACCGGCGAGCCTACATACGC CGCCGACTTCAAGCGGAGATTCACCTTCTCTCTGGACA CCAGCAAAAGCACCGCCTACCTGCAGATGAACAGTCT GCGGGCCGAGGACACCGCCGTGTACTACTGCGCCAAG TACCCCTACTATTACGGCACCAGCCACTGGTACTTCGA CGTGTGGGGGCAGGGCACCCTGGTGACAGTGAGTTCA GAGCCTAAATCTTGCGACAAGACCCACACATGCCCACC TTGTCCAGCACCAGAGCTGCTGGGAGGACCAAGCGTGT TCCTGTTTCCACCCAAGCCCAAGGATACCCTGATGATC TCCAGGACCCCAGAGGTGACATGCGTGGTGGTGGACG TGTCTCACGAGGATCCTGAGGTGAAGTTTAACTGGTAC GTGGACGGCGTGGAGGTGCACAATGCCAAGACCAAGC CTCGGGAGGAGCAGTACAATTCCACCTATAGAGTGGTG TCTGTGCTGACAGTGCTGCACCAGGATTGGCTGAACGG CAAGGAGTATAAGTGCAAGGTGAGCAATAAGGCCCTG CCTGCCCCAATCGAGAAGACCATCTCCAAGGCAAAGG GACAGCCAAGGGAGCCTCAGGTGTACACACTGCCTCC ATCTCGCGACGAGCTGACCAAGAACCAGGTGAGCCTG ACATGTCTGGTGAAGGGCTTCTATCCTTCCGATATCGC CGTGGAGTGGGAGTCTAATGGCCAGCCAGAGAACAAT TACAAGACCACACCCCCTGTGCTGGACAGCGATGGCTC CTTCTTTCTGTATTCTAAGCTGACCGTGGATAAGAGCC |
| | | GGTGGCAGCAGGGCAACGTGTTTTCCTGCTCTGTGATG CACGAGGCCCTGCACAATCACTACACCCAGAAGAGCC TGTCCCTGTCTCCCGGC |
| 9 | Amino acid sequence of fusion protein 2 | EIVMTQSPSTLSASVGDRVIITCQASEIIHSWLAWYQQKPG KAPKLLIYLASTLASGVPSRFSGSGSGAEFTLTISSLQPDDF ATYYCQNVYLASTNGANFGQGTKLTVLGGGGGSGGGGS GGGGSGGGGSEVQLVESGGGLVQPGGSLRLSCTASGFSL TDYYYMTWVRQAPGKGLEWVGFIDPDDDPYYATWAKG RFTISRDNSKNTLYLQMNSLRAEDTAVYYCAGGDHNSG WGLDIWGQGTLVTVSSGGGGSGGGGSGGGGSGGGGSGG GGSDIQLTQSPSSLSASVGDRVTITCSASQDISNYLNWYQ QKPGKAPKVLIYFTSSLHSGVPSRFSGSGSGTDFTLTISSLQ PEDFATYYCQQYSTVPWTFGQGTKVEIKGGGGSGGGGSG GGGSGGGGSEVQLVESGGGLVQPGGSLRLSCAASGYDFT HYGMNWVRQAPGKGLEWVGWINTYTGEPTYAADFKRR FTFSLDTSKSTAYLQMNSLRAEDTAVYYCAKYPYYYGTS HWYFDVWGQGTLVTVSS |
| 10 | Nucleotide sequence encoding fusion protein 2 | GAGATCGTGATGACCCAGAGCCCTAGCACACTGAGCG CCTCTGTGGGAGATAGAGTGATCATCACCTGTCAGGCC TCTGAGATCATCCACAGCTGGCTGGCTTGGTACCAGCA AAAGCCTGGGAAGGCCCCTAAACTGCTGATCTATCTCG CTAGCACACTGGCCTCTGGAGTCCCCAGCCGGTTTAGC GGAAGCGGCTCTGGCGCCGAATTCACCCTGACAATCAG CAGCCTGCAACCCGATGACTTTGCCACATACTACTGTC AGAACGTGTACCTGGCTAGCACCAACGGCGCCAACTTC GGCCAGGGCACAAAGCTGACCGTGCTTGGAGGCGGCG GCGGCTCCGGCGGAGGCGGAAGCGGCGGCGGCGGCAG CGGCGGGGGCGGCAGCGAGGTGCAGCTGGTGGAGTCG GGCGGCGGCCTGGTGCAGCCAGGAGGTTCTCTGAGGCT GAGCTGCACCGCAAGCGGCTTCAGCCTGACCGACTACT ACTATATGACATGGGTCAGACAGGCCCCTGGAAAGGG |
| | | CCTGGAATGGGTCGGATTTATCGACCCTGACGACGATC CTTACTACGCCACTTGGGCCAAGGGCAGATTTACCATT TCCAGAGATAACTCTAAGAACACCCTGTACCTGCAAAT GAACAGCCTGCGCGCCGAGGATACAGCCGTGTACTACT GCGCAGGCGGCGACCATAATTCAGGATGGGGCCTGGA TATCTGGGGCCAGGGCACGCTGGTGACCGTGTCCTCCG GCGGAGGAGGCTCTGGCGGAGGCGGCAGCGGCGGCGG CGGCTCCGGCGGCGGCGGCAGCGGCGGTGGCGGCAGC GACATCCAGCTGACACAGTCCCCAAGCAGCCTGAGCG CTTCTGTGGGCGACAGAGTGACCATCACATGCAGCGCC TCTCAGGACATCAGTAACTACCTGAACTGGTACCAGCA GAAACCTGGTAAGGCCCCCAAGGTGCTGATCTACTTCA CCAGCAGCCTGCACTCTGGCGTGCCTAGCAGATTCAGC GGCAGCGGATCTGGCACAGACTTCACCCTGACCATTAG CTCCCTGCAGCCTGAGGACTTCGCCACATACTACTGCC AGCAGTACTCCACCGTTCCCTGGACCTTCGGCCAGGGG ACCAAGGTGGAGATCAAGGGGGGCGGCGGCAGCGGAG GAGGCGGAAGCGGCGGGGGCGGATCTGGAGGAGGAG GTAGCGAAGTGCAGCTGGTGGAAAGCGGGGGCGGCCT GGTGCAACCTGGCGGCAGCCTCAGACTGAGCTGCGCC GCTAGCGGATATGATTTCACACACTACGGCATGAATTG GGTGCGGCAGGCTCCTGGCAAGGGCCTGGAATGGGTG GGCTGGATCAATACCTACACCGGCGAGCCTACATACGC CGCCGACTTCAAGCGGAGATTCACCTTCTCTCTGGACA CCAGCAAAAGCACCGCCTACCTGCAGATGAACAGTCT GCGGGCCGAGGACACCGCCGTGTACTACTGCGCCAAG TACCCCTACTATTACGGCACCAGCCACTGGTACTTCGA CGTGTGGGGGCAGGGCACCCTGGTGACAGTGAGTTCA |
| 11 | Amino acid sequence of fusion protein 3 | SDTGRPFVEMYSEIPEIIHMTEGRELVIPCRVTSPNITVTLK KFPLDTLIPDGKRIIWDSRKGFIISNATYKEIGLLTCEATVN GHLYKTNYLTHRQTNTIIDVVLSPSHGIELSVGEKLVLNC |
| | | TARTELNVGIDFNWEYPSSKHQHKKLVNRDLKTQSGSEM KKFLSTLTIDGVTRSDQGLYTCAASSGLMTKKNSTFVRV HEKGGGGSGGGGSGGGGSGGGGSGGGGSDIQLTQSPSSL SASVGDRVTITCSASQDISNYLNWYQQKPGKAPKVLIYFT SSLHSGVPSRFSGSGSGTDFTLTISSLQPEDFATYYCQQYS TVPWTFGQGTKVEIKGGGGSGGGGSGGGGSGGGGSEVQ LVESGGGLVQPGGSLRLSCAASGYDFTHYGMNWVRQAP GKGLEWVGWINTYTGEPTYAADFKRRFTFSLDTSKSTAY LQMNSLRAEDTAVYYCAKYPYYYGTSHWYFDVWGQGT LVTVSSEPKSCDKTHTCPPCPAPELLGGPSVFLFPPKPKDT LMISRTPEVTCVVVDVSHEDPEVKFNWYVDGVEVHNAK TKPREEQYNSTYRVVSVLTVLHQDWLNGKEYKCKVSNK ALPAPIEKTISKAKGQPREPQVYTLPPSRDELTKNQVSLTC LVKGFYPSDIAVEWESNGQPENNYKTTPPVLDSDGSFFLY SKLTVDKSRWQQGNVFSCSVMHEALHNHYTQKSLSLSP G |
| 12 | Nucleotide sequence encoding fusion protein 3 | AGCGACACCGGCAGACCTTTCGTGGAAATGTACAGCG AGATCCCAGAGATTATCCACATGACCGAGGGCCGGGA ACTGGTGATCCCCTGCAGAGTTACCAGCCCAAACATCA CCGTGACCCTCAAGAAGTTCCCCCTGGATACACTGATC CCTGACGGCAAAAGAATCATCTGGGACAGCAGAAAGG GCTTCATCATCAGCAACGCCACCTACAAGGAAATCGGC CTGCTGACATGCGAGGCCACCGTGAACGGCCACCTGTA CAAGACCAACTACCTGACCCATAGACAGACCAATACA ATCATCGATGTGGTGCTGAGCCCTTCCCACGGAATCGA GCTGTCCGTGGGAGAAAAGCTGGTGCTAAATTGCACCG CCAGAACAGAACTGAACGTGGGCATCGACTTCAACTG GGAGTACCCTTCTAGCAAGCACCAGCACAAGAAGCTG GTCAACCGGGACCTCAAGACACAGAGCGGCTCCGAGA TGAAAAAATTTCTGTCTACACTGACAATCGACGGCGTC ACAAGATCCGATCAGGGACTGTACACCTGTGCCGCTAG |
| | | CAGCGGACTGATGACCAAGAAGAACTCTACCTTCGTGC GGGTGCACGAGAAGGGCGGCGGCTCTGGCGGCGG CGGCAGCGGCGGAGGCGGCTCCGGCGGCGGCAGC GGCGGCGGAGGAAGCGACATCCAGCTGACACAAAGCC CCAGCAGCCTGTCAGCCTCTGTGGGCGACCGGGTGACC ATCACATGCAGCGCCAGCCAGGATATCAGCAATTACCT TAACTGGTATCAGCAGAAGCCCGGCAAGGCCCCTAAG GTGCTGATCTACTTCACCAGTAGCCTGCACAGCGGGGT GCCTTCTAGATTTAGCGGAAGCGGAAGCGGGACTGACT TTACCCTGACCATCTCTTCTCTGCAGCCTGAGGATTTTG CCACATACTACTGTCAGCAATACAGCACCGTCCCTTGG ACCTTCGGCCAGGGCACCAAGGTGGAAATAAAGGGCG GCGGCGGCTCTGGAGGCGGTGGCAGCGGGGGCGGCGG CAGCGGCGGAGGCGGATCCGAGGTGCAGCTGGTTGAA AGCGGCGGCGGCCTGGTGCAGCCCGGCGGAAGCCTGC GGCTGAGCTGTGCTGCTAGCGGCTACGACTTCACCCAC TACGGCATGAACTGGGTGCGCCAGGCTCCTGGAAAAG GCCTCGAATGGGTCGGATGGATCAACACATACACCGG CGAGCCTACATACGCCGCCGATTTCAAGCGAAGATTCA CCTTCTCTCTGGACACTAGCAAGTCCACCGCCTACCTG CAAATGAACAGCCTGAGGGCCGAGGATACAGCCGTGT ACTATTGCGCAAAGTACCCATACTATTACGGCACCAGC CACTGGTACTTCGACGTGTGGGGCCAGGGCACCCTGGT GACCGTGTCTTCTGAGCCTAAATCTTGCGACAAGACCC ACACATGCCCACCTTGTCCAGCACCAGAGCTGCTGGGA GGACCAAGCGTGTTCCTGTTTCCACCCAAGCCCAAGGA TACCCTGATGATCTCCAGGACCCCAGAGGTGACATGCG TGGTGGTGGACGTGTCTCACGAGGATCCTGAGGTGAAG TTTAACTGGTACGTGGACGGCGTGGAGGTGCACAATGC CAAGACCAAGCCTCGGGAGGAGCAGTACAATTCCACC TATAGAGTGGTGTCTGTGCTGACAGTGCTGCACCAGGA |
| | | TTGGCTGAACGGCAAGGAGTATAAGTGCAAGGTGAGC AATAAGGCCCTGCCTGCCCCAATCGAGAAGACCATCTC CAAGGCAAAGGGACAGCCAAGGGAGCCTCAGGTGTAC ACACTGCCTCCATCTCGCGACGAGCTGACCAAGAACCA GGTGAGCCTGACATGTCTGGTGAAGGGCTTCTATCCTT CCGATATCGCCGTGGAGTGGGAGTCTAATGGCCAGCCA GAGAACAATTACAAGACCACACCCCCTGTGCTGGACA GCGATGGCTCCTTCTTTCTGTATTCTAAGCTGACCGTGG ATAAGAGCCGGTGGCAGCAGGGCAACGTGTTTTCCTGC TCTGTGATGCACGAGGCCCTGCACAATCACTACACCCA GAAGAGCCTGTCCCTGTCTCCCGGC |
| 13 | Amino acid sequence of fusion protein 4 | SDTGRPFVEMYSEIPEIIHMTEGRELVIPCRVTSPNITVTLK KFPLDTLIPDGKRIIWDSRKGFIISNATYKEIGLLTCEATVN GHLYKTNYLTHRQTNTIIDVVLSPSHGIELSVGEKLVLNC TARTELNVGIDFNWEYPSSKHQHKKLVNRDLKTQSGSEM KKFLSTLTIDGVTRSDQGLYTCAASSGLMTKKNSTFVRV HEKGGGGSGGGGSGGGGSGGGGSGGGGSDIQLTQSPSSL SASVGDRVTITCSASQDISNYLNWYQQKPGKAPKVLIYFT SSLHSGVPSRFSGSGSGTDFTLTISSLQPEDFATYYCQQYS TVPWTFGQGTKVEIKGGGGSGGGGSGGGGSGGGGSEVQ LVESGGGLVQPGGSLRLSCAASGYDFTHYGMNWVRQAP GKGLEWVGWINTYTGEPTYAADFKRRFTFSLDTSKSTAY LQMNSLRAEDTAVYYCAKYPYYYGTSHWYFDVWGQGT LVTVSS |
| 14 | Nucleotide sequence encoding fusion protein 4 | AGCGACACCGGCAGACCTTTCGTGGAAATGTACAGCG AGATCCCAGAGATTATCCACATGACCGAGGGCCGGGA ACTGGTGATCCCCTGCAGAGTTACCAGCCCAAACATCA CCGTGACCCTCAAGAAGTTCCCCCTGGATACACTGATC CCTGACGGCAAAAGAATCATCTGGGACAGCAGAAAGG GCTTCATCATCAGCAACGCCACCTACAAGGAAATCGGC CTGCTGACATGCGAGGCCACCGTGAACGGCCACCTGTA |
| | | CAAGACCAACTACCTGACCCATAGACAGACCAATACA ATCATCGATGTGGTGCTGAGCCCTTCCCACGGAATCGA GCTGTCCGTGGGAGAAAAGCTGGTGCTAAATTGCACCG CCAGAACAGAACTGAACGTGGGCATCGACTTCAACTG GGAGTACCCTTCTAGCAAGCACCAGCACAAGAAGCTG GTCAACCGGGACCTCAAGACACAGAGCGGCTCCGAGA TGAAAAAATTTCTGTCTACACTGACGATCGACGGCGTC ACAAGATCCGATCAGGGACTGTACACCTGTGCCGCTAG CAGCGGACTGATGACCAAGAAGAACTCTACCTTCGTGC GGGTGCACGAGAAGGGCGGCGGCGGCTCTGGCGGCGG CGGCAGCGGCGGAGGCGGCTCCGGCGGCGGCGGCAGC GGCGGCGGAGGAAGCGACATCCAGCTGACACAAAGCC CCAGCAGCCTGTCAGCCTCTGTGGGCGACCGGGTGACC ATCACATGCAGCGCCAGCCAGGATATCAGCAATTACCT TAACTGGTATCAGCAGAAGCCCGGCAAGGCCCCTAAG GTGCTGATCTACTTCACCAGTAGCCTGCACAGCGGGGT GCCTTCTAGATTTAGCGGAAGCGGAAGCGGGACTGACT TTACCCTGACCATCTCTTCTCTGCAGCCTGAGGATTTTG CCACATACTACTGTCAGCAATACAGCACCGTCCCTTGG ACCTTCGGCCAGGGCACCAAGGTGGAAATAAAGGGCG GCGGCGGCTCTGGAGGCGGTGGCAGCGGGGGCGGCGG CAGCGGCGGAGGCGGATCCGAGGTGCAGCTGGTTGAA AGCGGCGGCGGCCTGGTGCAGCCCGGCGGAAGCCTGC GGCTGAGCTGTGCTGCTAGCGGCTACGACTTCACCCAC TACGGCATGAACTGGGTGCGCCAGGCTCCTGGAAAAG GCCTCGAATGGGTCGGATGGATCAACACATACACCGG CGAGCCTACATACGCCGCCGATTTCAAGCGAAGATTCA CCTTCTCTCTGGACACTAGCAAGTCCACCGCCTACCTG CAAATGAACAGCCTGAGGGCCGAGGATACAGCCGTGT ACTATTGCGCAAAGTACCCATACTATTACGGCACCAGC CACTGGTACTTCGACGTGTGGGGCCAGGGCACCCTGGT |
| | | GACCGTGTCTTCT |
| 15 | Amino acid sequence of fusion protein 5 | SDTGRPFVEMYSEIPEIIHMTEGRELVIPCRVTSPNITVTLK KFPLDTLIPDGKRIIWDSRKGFIISNATYKEIGLLTCEATVN GHLYKTNYLTHRQTNTIIDVVLSPSHGIELSVGEKLVLNC TARTELNVGIDFNWEYPSSKHQHKKLVNRDLKTQSGSEM KKFLSTLTIDGVTRSDQGLYTCAASSGLMTKKNSTFVRV HEKGGGGSGGGGSGGGGSGGGGSGGGGSEIVMTQSPSTL SASVGDRVIITCQASEIIHSWLAWYQQKPGKAPKLLIYLA STLASGVPSRFSGSGSGAEFTLTISSLQPDDFATYYCQNVY LASTNGANFGQGTKLTVLGGGGGSGGGGSGGGGSGGGG SEVQLVESGGGLVQPGGSLRLSCTASGFSLTDYYYMTWV RQAPGKGLEWVGFIDPDDDPYYATWAKGRFTISRDNSKN TLYLQMNSLRAEDTAVYYCAGGDHNSGWGLDIWGQGT LVTVSSGGGGSGGGGSGGGGSGGGGSGGGGSDIQLTQSP SSLSASVGDRVTITCSASQDISNYLNWYQQKPGKAPKVLI YFTSSLHSGVPSRFSGSGSGTDFTLTISSLQPEDFATYYCQ QYSTVPWTFGQGTKVEIKGGGGSGGGGSGGGGSGGGGS EVQLVESGGGLVQPGGSLRLSCAASGYDFTHYGMNWVR QAPGKGLEWVGWINTYTGEPTYAADFKRRFTFSLDTSKS TAYLQMNSLRAEDTAVYYCAKYPYYYGTSHWYFDVWG QGTLVTVSSEPKSCDKTHTCPPCPAPELLGGPSVFLFPPKP KDTLMISRTPEVTCVVVDVSHEDPEVKFNWYVDGVEVH NAKTKPREEQYNSTYRVVSVLTVLHQDWLNGKEYKCKV SNKALPAPIEKTISKAKGQPREPQVYTLPPSRDELTKNQVS LTCLVKGFYPSDIAVEWESNGQPENNYKTTPPVLDSDGSF FLYSKLTVDKSRWQQGNVFSCSVMHEALHNHYTQKSLS LSPG |
| 16 | Nucleotide sequence encoding fusion protein 5 | AGCGACACCGGCCGGCCTTTCGTGGAAATGTACAGCG AGATCCCCGAGATCATCCACATGACCGAGGGCAGAGA GCTGGTGATCCCCTGCCGGGTGACCAGCCCTAACATCA CCGTGACCCTGAAGAAATTCCCCCTGGACACACTGATC |
| | | CCCGATGGAAAAAGAATCATCTGGGACAGCAGAAAGG GCTTCATCATTTCTAATGCCACATACAAGGAAATCGGC CTGCTGACTTGTGAAGCCACCGTGAACGGCCACCTGTA CAAGACCAACTACCTGACTCATAGACAAACAAACACA ATCATCGACGTGGTGCTGAGCCCTTCTCATGGCATCGA GCTCTCTGTGGGCGAGAAGCTGGTGCTGAATTGCACCG CTCGCACCGAGCTGAACGTGGGAATCGACTTCAACTGG GAGTACCCTTCTTCTAAGCACCAGCACAAGAAGCTGGT GAACCGGGACCTGAAGACCCAGAGCGGCAGCGAAATG AAAAAGTTCCTGTCCACACTGACCATTGACGGCGTGAC AAGAAGCGACCAGGGCCTGTATACATGTGCCGCCTCTT CTGGGCTGATGACCAAGAAGAACAGCACCTTCGTGCG GGTGCACGAGAAGGGCGGCGGTGGCTCAGGAGGTGGC GGCAGCGGCGGCGGATCGGGCGGGGGCGGCTCTG GAGGCGGCGGTAGCGAGATCGTGATGACCCAGAGCCC CAGCACCCTGTCCGCCAGCGTGGGCGACAGAGTGATC ATCACTTGCCAGGCCTCCGAAATCATCCACAGCTGGCT GGCTTGGTACCAGCAGAAACCTGGCAAGGCTCCAAAG CTGCTGATCTACCTGGCCAGCACCCTCGCTAGCGGCGT TCCTTCCAGATTCAGCGGCAGCGGCTCCGGAGCTGAAT TCACCCTGACCATCAGCTCTCTGCAGCCTGACGACTTC GCCACATACTACTGTCAGAACGTGTACCTGGCTTCTAC AAACGGCGCCAATTTCGGCCAGGGCACAAAACTGACA GTGCTGGGCGGCGGTGGAGGATCGGGAGGCGGCGGCT CTGGAGGCGGCGGCAGCGGCGGCGGCGGCTCTGAAGT GCAACTGGTTGAGAGCGGCGGGGGCCTGGTGCAGCCC GGTGGCTCCCTGAGACTGAGCTGCACCGCCAGCGGATT TAGCCTGACAGATTACTATTACATGACATGGGTCAGAC AGGCCCCAGGCAAGGGCCTGGAATGGGTCGGCTTCAT CGATCCTGATGATGACCCCTACTACGCCACTTGGGCCA AGGGGAGGTTCACCATTTCTAGAGATAACAGCAAGAA |
| | | CACCCTGTACCTGCAGATGAACAGCCTGAGAGCCGAG GACACCGCCGTGTACTACTGCGCCGGCGGAGATCACA ACAGCGGCTGGGGCCTGGATATCTGGGGACAGGGCAC CCTGGTGACCGTGTCTAGCGGCGGCGGCGGCAGCGGC GGCGGCGGCAGCGGCGGCGGCGGCTCTGGCGGCGGCG GAAGCGGAGGTGGAGGTTCCGATATCCAGCTGACACA GAGTCCTAGCTCCCTTAGCGCTAGCGTGGGCGACAGAG TGACCATCACCTGCAGCGCCAGCCAGGACATTAGCAAT TACCTGAACTGGTACCAGCAAAAGCCTGGCAAAGCCC CTAAGGTACTGATCTACTTCACCAGCAGCCTGCACTCT GGGGTGCCTTCCCGGTTCAGCGGATCTGGCTCTGGTAC CGATTTTACCCTGACAATCTCCTCTCTGCAGCCAGAGG ATTTTGCCACCTACTACTGCCAGCAGTACAGCACCGTG CCCTGGACATTTGGACAAGGAACCAAGGTGGAAATCA AGGGAGGCGGCGGAAGCGGCGGCGGCGGTAGCGGCG GTGGCGGTAGCGGCGGTGGAGGCAGCGAGGTGCAGCT GGTGGAAAGCGGCGGAGGCCTCGTGCAACCTGGCGGC TCCCTGAGACTGAGTTGTGCCGCTTCCGGCTACGACTT CACACACTACGGCATGAACTGGGTGCGGCAGGCTCCA GGCAAGGGCCTGGAGTGGGTGGGATGGATCAACACCT ACACCGGAGAACCTACATATGCTGCCGACTTTAAGCGT AGGTTCACCTTCAGCCTGGACACCAGCAAGAGCACAG CCTACCTGCAGATGAATTCTCTGCGGGCCGAGGACACC GCCGTGTACTACTGCGCCAAGTACCCCTACTACTATGG CACCAGCCACTGGTATTTCGACGTGTGGGGCCAGGGCA CACTGGTTACCGTCAGCTCTGAGCCTAAATCTTGCGAC AAGACCCACACATGCCCACCTTGTCCAGCACCAGAGCT GCTGGGAGGACCAAGCGTGTTCCTGTTTCCACCCAAGC CCAAGGATACCCTGATGATCTCCAGGACCCCAGAGGTG ACATGCGTGGTGGTGGACGTGTCTCACGAGGATCCTGA GGTGAAGTTTAACTGGTACGTGGACGGCGTGGAGGTG |
| | | CACAATGCCAAGACCAAGCCTCGGGAGGAGCAGTACA ATTCCACCTATAGAGTGGTGTCTGTGCTGACAGTGCTG CACCAGGATTGGCTGAACGGCAAGGAGTATAAGTGCA AGGTGAGCAATAAGGCCCTGCCTGCCCCAATCGAGAA GACCATCTCCAAGGCAAAGGGACAGCCAAGGGAGCCT CAGGTGTACACACTGCCTCCATCTCGCGACGAGCTGAC CAAGAACCAGGTGAGCCTGACATGTCTGGTGAAGGGC TTCTATCCTTCCGATATCGCCGTGGAGTGGGAGTCTAA TGGCCAGCCAGAGAACAATTACAAGACCACACCCCCT GTGCTGGACAGCGATGGCTCCTTCTTTCTGTATTCTAAG CTGACCGTGGATAAGAGCCGGTGGCAGCAGGGCAACG TGTTTTCCTGCTCTGTGATGCACGAGGCCCTGCACAAT CACTACACCCAGAAGAGCCTGTCCCTGTCTCCCGGC |
| 17 | Amino acid sequence of fusion protein 6 | SDTGRPFVEMYSEIPEIIHMTEGRELVIPCRVTSPNITVTLK KFPLDTLIPDGKRIIWDSRKGFIISNATYKEIGLLTCEATVN GHLYKTNYLTHRQTNTIIDVVLSPSHGIELSVGEKLVLNC TARTELNVGIDFNWEYPSSKHQHKKLVNRDLKTQSGSEM KKFLSTLTIDGVTRSDQGLYTCAASSGLMTKKNSTFVRV HEKGGGGSGGGGSGGGGSGGGGSGGGGSEIVMTQSPSTL SASVGDRVIITCQASEIIHSWLAWYQQKPGKAPKLLIYLA STLASGVPSRFSGSGSGAEFTLTISSLQPDDFATYYCQNVY LASTNGANFGQGTKLTVLGGGGGSGGGGSGGGGSGGGG SEVQLVESGGGLVQPGGSLRLSCTASGFSLTDYYYMTWV RQAPGKGLEWVGFIDPDDDPYYATWAKGRFTISRDNSKN TLYLQMNSLRAEDTAVYYCAGGDHNSGWGLDIWGQGT LVTVSSGGGGSGGGGSGGGGSGGGGSGGGGSDIQLTQSP SSLSASVGDRVTITCSASQDISNYLNWYQQKPGKAPKVLI YFTSSLHSGVPSRFSGSGSGTDFTLTISSLQPEDFATYYCQ QYSTVPWTFGQGTKVEIKGGGGSGGGGSGGGGSGGGGS EVQLVESGGGLVQPGGSLRLSCAASGYDFTHYGMNWVR QAPGKGLEWVGWINTYTGEPTYAADFKRRFTFSLDTSKS |
| | | TAYLQMNSLRAEDTAVYYCAKYPYYYGTSHWYFDVWG QGTLVTVSS |
| 18 | Nucleotide sequence encoding fusion protein 6 | AGCGACACCGGCCGGCCTTTCGTGGAAATGTACAGCG AGATCCCCGAGATCATCCACATGACCGAGGGCAGAGA GCTGGTGATCCCCTGCCGGGTGACCAGCCCTAACATCA CCGTGACCCTGAAGAAATTCCCCCTGGACACACTGATC CCCGATGGAAAAAGAATCATCTGGGACAGCAGAAAGG GCTTCATCATTTCTAATGCCACATACAAGGAAATCGGC CTGCTGACTTGTGAAGCCACCGTGAACGGCCACCTGTA CAAGACCAACTACCTGACTCATAGACAAACAAACACA ATCATCGACGTGGTGCTGAGCCCTTCTCATGGCATCGA GCTCTCTGTGGGCGAGAAGCTGGTGCTGAATTGCACCG CTCGCACCGAGCTGAACGTGGGAATCGACTTCAACTGG GAGTACCCTTCTTCTAAGCACCAGCACAAGAAGCTGGT GAACCGGGACCTGAAGACCCAGAGCGGCAGCGAAATG AAAAAGTTCCTGTCCACACTGACCATTGACGGCGTGAC AAGAAGCGACCAGGGCCTGTATACATGTGCCGCCTCTT CTGGGCTGATGACCAAGAAGAACAGCACCTTCGTGCG GGTGCACGAGAAGGGCGGCGGTGGCTCAGGAGGTGGC GGCAGCGGCGGCGGATCGGGCGGGGGCGGCTCTG GAGGCGGCGGTAGCGAGATCGTGATGACCCAGAGCCC CAGCACCCTGTCCGCCAGCGTGGGCGACAGAGTGATC ATCACTTGCCAGGCCTCCGAAATCATCCACAGCTGGCT GGCTTGGTACCAGCAGAAACCTGGCAAGGCTCCAAAG CTGCTGATCTACCTGGCCAGCACCCTCGCTAGCGGCGT TCCTTCCAGATTCAGCGGCAGCGGCTCCGGAGCTGAAT TCACCCTGACCATCAGCTCTCTGCAGCCTGACGACTTC GCCACATACTACTGTCAGAACGTGTACCTGGCTTCTAC AAACGGCGCCAATTTCGGCCAGGGCACAAAACTGACA GTGCTGGGCGGCGGTGGAGGATCGGGAGGCGGCGGCT CTGGAGGCGGCGGCAGCGGCGGCGGCGGCTCTGAAGT |
| | | GCAACTGGTTGAGAGCGGCGGGGGCCTGGTGCAGCCC GGTGGCTCCCTGAGACTGAGCTGCACCGCCAGCGGATT TAGCCTGACAGATTACTATTACATGACATGGGTCAGAC AGGCCCCAGGCAAGGGCCTGGAATGGGTCGGCTTCAT CGATCCTGATGATGACCCCTACTACGCCACTTGGGCCA AGGGGAGGTTCACCATTTCTAGAGATAACAGCAAGAA CACCCTGTACCTGCAGATGAACAGCCTGAGAGCCGAG GACACCGCCGTGTACTACTGCGCCGGCGGAGATCACA ACAGCGGCTGGGGCCTGGATATCTGGGGACAGGGCAC CCTGGTGACCGTGTCTAGCGGCGGCGGCGGCAGCGGC GGCGGCGGCAGCGGCGGCGGCGGCTCTGGCGGCGGCG GAAGCGGAGGTGGAGGTTCCGATATCCAGCTGACACA GAGTCCTAGCTCCCTTAGCGCTAGCGTGGGCGACAGAG TGACCATCACCTGCAGCGCCAGCCAGGACATTAGCAAT TACCTGAACTGGTACCAGCAAAAGCCTGGCAAAGCCC CTAAGGTACTGATCTACTTCACCAGCAGCCTGCACTCT GGGGTGCCTTCCCGGTTCAGCGGATCTGGCTCTGGTAC CGATTTTACCCTGACAATCTCCTCTCTGCAGCCAGAGG ATTTTGCCACCTACTACTGCCAGCAGTACAGCACCGTG CCCTGGACATTTGGACAAGGAACCAAGGTGGAAATCA AGGGAGGCGGCGGAAGCGGCGGCGGTAGCGGCG GTGGCGGTAGCGGCGGTGGAGGCAGCGAGGTGCAGCT GGTGGAAAGCGGCGGAGGCCTCGTGCAACCTGGCGGC TCCCTGAGACTGAGTTGTGCCGCTTCCGGCTACGACTT CACACACTACGGCATGAACTGGGTGCGGCAGGCTCCA GGCAAGGGCCTGGAGTGGGTGGGATGGATCAACACCT ACACCGGAGAACCTACATATGCTGCCGACTTTAAGCGT AGGTTCACCTTCAGCCTGGACACCAGCAAGAGCACAG CCTACCTGCAGATGAATTCTCTGCGGGCCGAGGACACC GCCGTGTACTACTGCGCCAAGTACCCCTACTACTATGG CACCAGCCACTGGTATTTCGACGTGTGGGGCCAGGGCA |
| | | CACTGGTTACCGTCAGCTCT |
| 19 | Amino acid sequence of fusion protein 7 | SDTGRPFVEMYSEIPEIIHMTEGRELVIPCRVTSPNITVTLK KFPLDTLIPDGKRIIWDSRKGFIISNATYKEIGLLTCEATVN GHLYKTNYLTHRQTNTIIDVVLSPSHGIELSVGEKLVLNC TARTELNVGIDFNWEYPSSKHQHKKLVNRDLKTQSGSEM KKFLSTLTIDGVTRSDQGLYTCAASSGLMTKKNSTFVRV HEKGGGGSGGGGSGGGGSGGGGSGGGGSEIVMTQSPSTL SASVGDRVIITCQASEIIHSWLAWYQQKPGKAPKLLIYLA STLASGVPSRFSGSGSGAEFTLTISSLQPDDFATYYCQNVY LASTNGANFGQGTKLTVLGGGGGSGGGGSGGGGSGGGG SEVQLVESGGGLVQPGGSLRLSCTASGFSLTDYYYMTWV RQAPGKGLEWVGFIDPDDDPYYATWAKGRFTISRDNSKN TLYLQMNSLRAEDTAVYYCAGGDHNSGWGLDIWGQGT LVTVSS |
| 20 | Nucleotide sequence encoding fusion protein 7 | AGCGACACCGGCCGGCCTTTCGTGGAAATGTACAGCG AGATCCCCGAGATCATCCACATGACCGAGGGCAGAGA GCTGGTGATCCCCTGCCGGGTGACCAGCCCTAACATCA CCGTGACCCTGAAGAAATTCCCCCTGGACACACTGATC CCCGATGGAAAAAGAATCATCTGGGACAGCAGAAAGG GCTTCATCATTTCTAATGCCACATACAAGGAAATCGGC CTGCTGACTTGTGAAGCCACCGTGAACGGCCACCTGTA CAAGACCAACTACCTGACTCATAGACAAACAAACACA ATCATCGACGTGGTGCTGAGCCCTTCTCATGGCATCGA GCTCTCTGTGGGCGAGAAGCTGGTGCTGAATTGCACCG CTCGCACCGAGCTGAACGTGGGAATCGACTTCAACTGG GAGTACCCTTCTTCTAAGCACCAGCACAAGAAGCTGGT GAACCGGGACCTGAAGACCCAGAGCGGCAGCGAAATG AAAAAGTTCCTGTCCACACTGACCATTGACGGCGTGAC AAGAAGCGACCAGGGCCTGTATACATGTGCCGCCTCTT CTGGGCTGATGACCAAGAAGAACAGCACCTTCGTGCG GGTGCACGAGAAGGGCGGCGGTGGCTCAGGAGGTGGC |
| | | GGCAGCGGCGGCGGATCGGGCGGGGGCGGCTCTG GAGGCGGCGGTAGCGAGATCGTGATGACCCAGAGCCC CAGCACCCTGTCCGCCAGCGTGGGCGACAGAGTGATC ATCACTTGCCAGGCCTCCGAAATCATCCACAGCTGGCT GGCTTGGTACCAGCAGAAACCTGGCAAGGCTCCAAAG CTGCTGATCTACCTGGCCAGCACCCTCGCTAGCGGCGT TCCTTCCAGATTCAGCGGCAGCGGCTCCGGAGCTGAAT TCACCCTGACCATCAGCTCTCTGCAGCCTGACGACTTC GCCACATACTACTGTCAGAACGTGTACCTGGCTTCTAC AAACGGCGCCAATTTCGGCCAGGGCACAAAACTGACA GTGCTGGGCGGCGGTGGAGGATCGGGAGGCGGCGGCT CTGGAGGCGGCGGCAGCGGCGGCGGCGGCTCTGAAGT GCAACTGGTTGAGAGCGGCGGGGGCCTGGTGCAGCCC GGTGGCTCCCTGAGACTGAGCTGCACCGCCAGCGGATT TAGCCTGACAGATTACTATTACATGACATGGGTCAGAC AGGCCCCAGGCAAGGGCCTGGAATGGGTCGGCTTCAT CGATCCTGATGATGACCCCTACTACGCCACTTGGGCCA AGGGGAGGTTCACCATTTCTAGAGATAACAGCAAGAA CACCCTGTACCTGCAGATGAACAGCCTGAGAGCCGAG GACACCGCCGTGTACTACTGCGCCGGCGGAGATCACA ACAGCGGCTGGGGCCTGGATATCTGGGGACAGGGCAC CCTGGTGACCGTGTCTAGC |
| 21 | Amino acid sequence of fusion protein 8 | EIVMTQSPSTLSASVGDRVIITCQASEIIHSWLAWYQQKPG KAPKLLIYLASTLASGVPSRFSGSGSGAEFTLTISSLQPDDF ATYYCQNVYLASTNGANFGQGTKLTVLGGGGGSGGGGS GGGGSGGGGSEVQLVESGGGLVQPGGSLRLSCTASGFSL TDYYYMTWVRQAPGKGLEWVGFIDPDDDPYYATWAKG RFTISRDNSKNTLYLQMNSLRAEDTAVYYCAGGDHNSG WGLDIWGQGTLVTVSSGGGGSGGGGSGGGGSGGGGSGG GGSSDTGRPFVEMYSEIPEIIHMTEGRELVIPCRVTSPNITV TLKKFPLDTLIPDGKRIIWDSRKGFIISNATYKEIGLLTCEA |
| | | TVNGHLYKTNYLTHRQTNTIIDVVLSPSHGIELSVGEKLV LNCTARTELNVGIDFNWEYPSSKHQHKKLVNRDLKTQSG SEMKKFLSTLTIDGVTRSDQGLYTCAASSGLMTKKNSTF VRVHEK |
| 22 | Nucleotide sequence encoding fusion protein 8 | GAGATCGTGATGACCCAGAGCCCTAGCACACTGAGCG CCTCTGTGGGAGATAGAGTGATCATCACCTGTCAGGCC TCTGAGATCATCCACAGCTGGCTGGCTTGGTACCAGCA AAAGCCTGGGAAGGCCCCTAAACTGCTGATCTATCTCG CTAGCACACTGGCCTCTGGAGTCCCCAGCCGGTTTAGC GGAAGCGGCTCTGGCGCCGAATTCACCCTGACAATCAG CAGCCTGCAACCCGATGACTTTGCCACATACTACTGTC AGAACGTGTACCTGGCTAGCACCAACGGCGCCAACTTC GGCCAGGGCACAAAGCTGACCGTGCTTGGAGGCGGCG GCGGCTCCGGCGGAGGCGGAAGCGGCGGCGGCGGCAG CGGCGGGGGCGGCAGCGAGGTGCAGCTGGTGGAGTCG GGCGGCGGCCTGGTGCAGCCAGGAGGTTCTCTGAGGCT GAGCTGCACCGCAAGCGGCTTCAGCCTGACCGACTACT ACTATATGACATGGGTCAGACAGGCCCCTGGAAAGGG CCTGGAATGGGTCGGATTTATCGACCCTGACGACGATC CTTACTACGCCACTTGGGCCAAGGGCAGATTTACCATT TCCAGAGATAACTCTAAGAACACCCTGTACCTGCAAAT GAACAGCCTGCGCGCCGAGGATACAGCCGTGTACTACT GCGCAGGCGGCGACCATAATTCAGGATGGGGCCTGGA TATCTGGGGCCAGGGCACGCTGGTGACCGTGTCCTCCG GCGGAGGAGGCTCTGGCGGAGGCGGCAGCGGCGGCGG CGGCTCCGGCGGCGGCGGCAGCGGCGGTGGCGGCAGC AGCGACACCGGCCGGCCTTTCGTGGAAATGTACAGCG AGATCCCCGAGATCATCCACATGACCGAGGGCAGAGA GCTGGTGATCCCCTGCCGGGTGACCAGCCCTAACATCA CCGTGACCCTGAAGAAATTCCCCCTGGACACACTGATC CCCGATGGAAAAAGAATCATCTGGGACAGCAGAAAGG |
| | | GCTTCATCATTTCTAATGCCACATACAAGGAAATCGGC CTGCTGACTTGTGAAGCCACCGTGAACGGCCACCTGTA CAAGACCAACTACCTGACTCATAGACAAACAAACACA ATCATCGACGTGGTGCTGAGCCCTTCTCATGGCATCGA GCTCTCTGTGGGCGAGAAGCTGGTGCTGAATTGCACCG CTCGCACCGAGCTGAACGTGGGAATCGACTTCAACTGG GAGTACCCTTCTTCTAAGCACCAGCACAAGAAGCTGGT GAACCGGGACCTGAAGACCCAGAGCGGCAGCGAAATG AAAAAGTTCCTGTCCACACTGACCATTGACGGCGTGAC AAGAAGCGACCAGGGCCTGTATACATGTGCCGCCTCTT CTGGGCTGATGACCAAGAAGAACAGCACCTTCGTGCG GGTGCACGAGAAG |
| 23 | Nucleotide sequence of the lentiviral vector KL-Kan-BB | GGCCGCGTTGCTGGCGTTTTTCCATAGGCTCCGCCCCC CTGACGAGCATCACAAAAATCGACGCTCAAGTCAGAG GTGGCGAAACCCGACAGGACTATAAAGATACCAGGCG TTTCCCCCTGGAAGCTCCCTCGTGCGCTCTCCTGTTCCG ACCCTGCCGCTTACCGGATACCTGTCCGCCTTTCTCCCT TCGGGAAGCGTGGCGCTTTCTCATAGCTCACGCTGTAG GTATCTCAGTTCGGTGTAGGTCGTTCGCTCCAAGCTGG GCTGTGTGCACGAACCCCCCGTTCAGCCCGACCGCTGC GCCTTATCCGGTAACTATCGTCTTGAGTCCAACCCGGT AAGACACGACTTATCGCCACTGGCAGCAGCCACTGGTA ACAGGATTAGCAGAGCGAGGTATGTAGGCGGTGCTAC AGAGTTCTTGAAGTGGTGGCCTAACTACGGCTACACTA GAAGAACAGTATTTGGTATCTGCGCTCTGCTGAAGCCA GTTACCTTCGGAAAAAGAGTTGGTAGCTCTTGATCCGG CAAACAAACCACCGCTGGTAGCGGTGGTTTTTTTGTTT GCAAGCAGCAGATTACGCGCAGAAAAAAAGGATCTCA AGAAGATCCTTTGATCTTTTCTACGGGGTCTGACGCTC AGTGGAACGAAAACTCACGTTAAGGGATTTTGGTCATG Aagcgcttttgaagctcggatccgaacaaacgacccaacacccgtgcgttttattctgtcttt |
| | | ttattgccgatccccTCAGAAGAACTCGTCAAGAAGGCGATAG AAGGCGATGCGCTGCGAATCGGGAGCGGCGATACCGT AAAGCACGAGGAAGCGGTCAGCCCATTCGCCGCCAAG CTCTTCAGCAATATCACGGGTAGCCAACGCTATGTCCT GATAGCGGTCCGCCACACCCAGCCGGCCACAGTCGAT GAATCCAGAAAAGCGGCCATTTTCCACCATGATATTCG GCAAGCAGGCATCGCCATGGGTCACGACGAGATCCTC GCCGTCGGGCATGCTCGCCTTGAGCCTGGCGAACAGTT CGGCTGGCGCGAGCCCCTGATGCTCTTCGTCCAGATCA TCCTGATCGACAAGACCGGCTTCCATCCGAGTACGTGC TCGCTCGATGCGATGTTTCGCTTGGTGGTCGAATGGGC AGGTAGCCGGATCAAGCGTATGCAGCCGCCGCATTGC ATCAGCCATGATGGATACTTTCTCGGCAGGAGCAAGGT GAGATGACAGGAGATCCTGCCCCGGCACTTCGCCCAAT AGCAGCCAGTCCCTTCCCGCTTCAGTGACAACGTCGAG CACAGCTGCGCAAGGAACGCCCGTCGTGGCCAGCCAC GATAGCCGCGCTGCCTCGTCTTGCAGTTCATTCAGGGC ACCGGACAGGTCGGTCTTGACAAAAAGAACCGGGCGC CCCTGCGCTGACAGCCGGAACACGGCGGCATCAGAGC AGCCGATTGTCTGTTGTGCCCAGTCATAGCCGAATAGC CTCTCCACCCAAGCGGCCGGAGAACCTGCGTGCAATCC ATCTTGTTCAATCATGCGAAACGATCCTCATCCTGTCTC TTGATCAGAGCTTGATCCCCTGCGCCATCAGATCCTTG GCGGCAAGAAAGCCATCCAGTTTACTTTGCAGGGCTTC CCAACCTTACCAGAGGCCTGCGCCGCGGCCAGCTGGCT AGCAATTCCCGGGTTAACTCTAGAgacattgattattgactagttatta atagtaatcaattacggggtcattagttcatagcccatatatggagttccgcgttacataactta cggtaaatggcccgcctggctgaccgcccaacgacccccgcccattgacgtcaataatga cgtatgttcccatagtaacgccaatagggactttccattgacgtcaatgggtggagtatttac ggtaaactgcccacttggcagtacatcaagtgtatcatatgccaagtacgccccctattgac gtcaatgacggtaaatggcccgcctggcattatgcccagtacatgaccttatgggactttcct |
| | | acttggcagtacatctacgtattagtcatcgctattaccatggtgatgcggttttggcagtacat caatgggcgtggatagcggtttgactcacggggatttccaagtctccaccccattgacgtca atgggagtttgttttggcaccaaaatcaacgggactttccaaaatgtcgtaacaactccgccc cattgacgcaaatgggcggtaggcgtgtacggtgggaggtctatataagcagagctcgttt agtgaaccggggtctctctggttagaccagatctgagcctgggagctctctggctaactag ggaacccactgcttaagcctcaataaagcttgccttgagtgcttcaagtagtgtgtgcccgtc tgttgtgtgactctggtaactagagatccctcagacccttttagtcagtgtggaaaatctctag cagtggcgcccgaacagggacttgaaagcgaaagggaaaccagagGAGctctctcga cgcaggactcggcttgctgaagcgcgcacggcaagaggcgaggggcggcgactggtg agtacgccaaaaattttgactagcggaggctagaaggagagagatgggtgcgagagcgt cagtattaagcgggggagaattagatcgcgatgggaaaaaattcggttaaggccaggggg aaagaaaaaatataaattaaaacatatagtatgggcaagcagggagctagaacgattcgca gttaatcctggcctgttagaaacatcagaaggctgtagacaaatactgggacagctacaac catcccttcagacaggatcagaagaacttagatcattatataatacagtagcaaccctctattg tgtgcatcaaaggatagagataaaagacaccaaggaagctttagacaagatagaggaaga gcaaaacaaaagtaagaccaccgcacagcaagcggccgctgatcttcagacctggagga ggagatatgagggacaattggagaagtgaattatataaatataaagtagtaaaaattgaacc attaggagtagcacccaccaaggcaaagagaagagtggtgcagagagaaaaaagagca gtgggaataggagctttgttccttgggttcttgggagcagcaggaagcactatgggcgcag cGtcaatgacgctgacggtacaggccagacaattattgtctggtatagtgcagcagcagaa caatttgctgagggctattgaggcgcaacagcatctgttgcaactcacagtctggggcatca agcagctccaggcaagaatcctggctgtggaaagatacctaaaggatcaacagctcctgg ggatttggggttgctctggaaaactcatttgcaccactgctgtgccttggaatgctagttgga gtaataaatctctggaacagatttggaatcacacgacctggatggagtgggacagagaaat taacaattacacaagcttaatacactccttaattgaagaatcgcaaaaccagcaagaaaaga atgaacaagaattattggaattagataaatgggcaagtttgtggaattggtttaacataacaaa ttggctgtggtatataaaattattcataatgatagtaggaggcttggtaggtttaagaatagtttt tgctgtactttctatagtgaatagagttaggcagggatattcaccattatcgtttcagacccacc tcccaaccccgaggggacccgacaggcccgaaggaatagaagaagaaggtggagaga gagacagagacagatccattcgattagtgaacggatctcgacggtatcggttaacttttaaa agaaaaggggggattggggggtacagtgcaggggaaagaatagtagacataatagcaa |
| | | cagacatacaaactaaagaattacaaaaacaaattacaaaaattcaaaattttatcgatcacg agactagcctcgagaagcttgatatcCGTCGACACGCGTgacaatcaacctctg gattacaaaatttgtgaaagattgactggtattcttaactatgttgctccttttacgctatgtggat acgctgctttaatgcctttgtatcatgctattgcttcccgtatggctttcattttctcctccttgtata aatcctggttgctgtctctttatgaggagttgtggcccgttgtcaggcaacgtggcgtggtgt gcactgtgtttgctgacgcaacccccactggttggggcattgccaccacctgtcagctccttt ccgggactttcgctttccccctccctattgccacggcggaactcatcgccgcctgccttgcc cgctgctggacaggggctcggctgttgggcactgacaattccgtggtgttgtcggggaag ctgacgtcctttccatggctgctcgcctgtgttgccacctggattctgcgcgggacgtccttct gctacgtcccttcggccctcaatccagcggaccttccttcccgcggcctgctgccggctctg cggcctcttccgcgtcttcgccttcgccctcagacgagtcggatctccctttgggccgcctc cccgcctggaattcgagctcggtacctttaagaccaatgacttacaaggcagctgtagatctt agccactttttaaaagaaaaggggggactggaagggctaattcactcccaacgaagacaa gatctgctttttgcttgtactgggtctctctggttagaccagatctgagcctgggagctctctgg ctaactagggaacccactgcttaagcctcaataaagcttgccttgagtgcttcaagtagtgtg tgcccgtctgttgtgtgactctggtaactagagatccctcagacccttttagtcagtgtggaaa atctctagcagtagtagttcatgtcatcttattattcagtatttataacttgcaaagaaatgaatat cagagagtgagaggaacttgtttattgcagcttataatggttacaaataaagcaatagcatca caaatttcacaaataaagcatttttttcactgcattctagttgtggtttgtccaaactcatcaatgt atcttatcatgtctggctctagctatcccgcccctaactccgcccaTCCCGCCCCTA ACTCCGCCCAgttccgcccattctccgccccatggctgactaattttttttatttatgca gaggccgaggccgcctcggcctctgagctattccagaagtagtgaggaggcttttttggag gccgctagcgtcgacCATTACTTATTGTTTTAGCTGTCCTCATG AATGTCTTTTCACTACCCATTTGCTTATCCTGCATCTCT CAGCCTTGACTCCACTCAGTTCTCTTGCTTAGAGATACC ACCTTTCCCCTGAAGTGTTCCTTCCATGTTTTACGGCGA GATGGTTTCTCCTCGCCTGGCCACTCAGCCTTAGTTGTC TCTGTTGTCTTATAGAGGTCTACTTGAAGAAGGAAAAA CAGGGGGCATGGTTTGACTGTCCTGTGAGCCCTTCTTC CCTGCCTCCCCCACTCACAGTGACCCGGAATCCCTCGA CATGGCAGTCTAGCACTAGTGCGGCCGCAGATCTGCTT |
| | | CCTCGCTCACTGACTCGCTGCGCTCGGTCGTTCGGCTG CGGCGAGCGGTATCAGCTCACTCAAAGGCGGTAATAC GGTTATCCACAGAATCAGGGGATAACGCAGGAAAGAA CATGTGAGCAAAAGGCCAGCAAAAGGCCAGGAACCGT AAAAA |
| 24 | Nucleotide sequence of the adeno-associated virus vector pAAV-MCS backbone | ACATGTGAGCAAAAGGCCAGCAAAAGGCCAGGAACCG TAAAAAGGCCGCGTTGCTGGCGTTTTTCCATAGGCTCC GCCCCCCTGACGAGCATCACAAAAATCGACGCTCAAGT CAGAGGTGGCGAAACCCGACAGGACTATAAAGATACC AGGCGTTTCCCCCTGGAAGCTCCCTCGTGCGCTCTCCT GTTCCGACCCTGCCGCTTACCGGATACCTGTCCGCCTTT CTCCCTTCGGGAAGCGTGGCGCTTTCTCATAGCTCACG CTGTAGGTATCTCAGTTCGGTGTAGGTCGTTCGCTCCA AGCTGGGCTGTGTGCACGAACCCCCCGTTCAGCCCGAC CGCTGCGCCTTATCCGGTAACTATCGTCTTGAGTCCAA CCCGGTAAGACACGACTTATCGCCACTGGCAGCAGCCA CTGGTAACAGGATTAGCAGAGCGAGGTATGTAGGCGG TGCTACAGAGTTCTTGAAGTGGTGGCCTAACTACGGCT ACACTAGAAGGACAGTATTTGGTATCTGCGCTCTGCTG AAGCCAGTTACCTTCGGAAAAAGAGTTGGTAGCTCTTG ATCCGGCAAACAAACCACCGCTGGTAGCGGTGGTTTTT TTGTTTGCAAGCAGCAGATTACGCGCAGAAAAAAAGG ATCTCAAGAAGATCCTTTGATCTTTTCTACGGGGTCTG ACGCTCAGTGGAACGAAAACTCACGTTAAGGGATTTTG GTCATGAGATTATCAAAAAGGATCTTCACCTAGATCCT TTTAAATTAAAAATGAAGTTTTAAATCAATCTAAAGTA TATATGAGTAAACTTGGTCTGACAGTTACCAATGCTTA ATCAGTGAGGCACCTATCTCAGCGATCTGTCTATTTCG TTCATCCATAGTTGCCTGACTCCCCGTCGTGTAGATAA CTACGATACGGGAGGGCTTACCATCTGGCCCCAGTGCT GCAATGATACCGCGAGACCCACGCTCACCGGCTCCAG |
| | | ATTTATCAGCAATAAACCAGCCAGCCGGAAGGGCCGA GCGCAGAAGTGGTCCTGCAACTTTATCCGCCTCCATCC AGTCTATTAATTGTTGCCGGGAAGCTAGAGTAAGTAGT TCGCCAGTTAATAGTTTGCGCAACGTTGTTGCCATTGCT ACAGGCATCGTGGTGTCACGCTCGTCGTTTGGTATGGC TTCATTCAGCTCCGGTTCCCAACGATCAAGGCGAGTTA CATGATCCCCCATGTTGTGCAAAAAAGCGGTTAGCTCC TTCGGTCCTCCGATCGTTGTCAGAAGTAAGTTGGCCGC AGTGTTATCACTCATGGTTATGGCAGCACTGCATAATT CTCTTACTGTCATGCCATCCGTAAGATGCTTTTCTGTGA CTGGTGAGTACTCAACCAAGTCATTCTGAGAATAGTGT ATGCGGCGACCGAGTTGCTCTTGCCCGGCGTCAATACG GGATAATACCGCGCCACATAGCAGAACTTTAAAAGTG CTCATCATTGGAAAACGTTCTTCGGGGCGAAAACTCTC AAGGATCTTACCGCTGTTGAGATCCAGTTCGATGTAAC CCACTCGTGCACCCAACTGATCTTCAGCATCTTTTACTT TCACCAGCGTTTCTGGGTGAGCAAAAACAGGAAGGCA AAATGCCGCAAAAAAGGGAATAAGGGCGACACGGAA ATGTTGAATACTCATACTCTTCCTTTTTCAATATTATTG AAGCATTTATCAGGGTTATTGTCTCATGAGCGGATACA TATTTGAATGTATTTAGAAAAATAAACAAATAGGGGTT CCGCGCACATTTCCCCGAAAAGTGCCACCTGACGTCTA AGAAACCATTATTATCATGACATTAACCTATAAAAATA GGCGTATCACGAGGCCCTTTCGTCTCGCGCGTTTCGGT GATGACGGTGAAAACCTCTGACACATGCAGCTCCCGG AGACGGTCACAGCTTGTCTGTAAGCGGATGCCGGGAG CAGACAAGCCCGTCAGGGCGCGTCAGCGGGTGTTGGC GGGTGTCGGGGCTGGCTTAACTATGCGGCATCAGAGCA GATTGTACTGAGAGTGCACCATAAAATTGTAAACGTTA ATATTTTGTTAAAATTCGCGTTAAATTTTTGTTAAATCA GCTCATTTTTTAACCAATAGGCCGAAATCGGCAAAATC |
| | | CCTTATAAATCAAAAGAATAGCCCGAGATAGGGTTGA GTGTTGTTCCAGTTTGGAACAAGAGTCCACTATTAAAG AACGTGGACTCCAACGTCAAAGGGCGAAAAACCGTCT ATCAGGGCGATGGCCCACTACGTGAACCATCACCCAA ATCAAGTTTTTTGGGGTCGAGGTGCCGTAAAGCACTAA ATCGGAACCCTAAAGGGAGCCCCCGATTTAGAGCTTGA CGGGGAAAGCCGGCGAACGTGGCGAGAAAGGAAGGG AAGAAAGCGAAAGGAGCGGGCGCTAGGGCGCTGGCAA GTGTAGCGGTCACGCTGCGCGTAACCACCACACCCGCC GCGCTTAATGCGCCGCTACAGGGCGCGTACTATGGTTG CTTTGACGTATGCGGTGTGAAATACCGCACAGATGCGT AAGGAGAAAATACCGCATCAGGCGCCCCTGCAGGCAG CTGCGCGCTCGCTCGCTCACTGAGGCCGCCCGGGCAAA GCCCGGGCGTCGGGCGACCTTTGGTCGCCCGGCCTCAG TGAGCGAGCGAGCGCGCAGAGAGGGAGTGGCCAACTC CATCACTAGGGGTTCCTACGCGTGTCGACGTTTATTTGT GAAATTTGTGATGCTATTGCTTTATTTGTAACCATCTAG CTTTATTTGTGAAATTTGTGATGCTATTGCTTTATTTGT AACCATTATAAGCTGCAATAAACAAGTTAACAACAAC AATTGCATTCATTTTATGTTTCAGGTTCAGGGGGAGAT GTGGGAGGTTTTTTAAAGTTTAAACAGGAACCCCTAGT GATGGAGTTGGCCACTCCCTCTCTGCGCGCTCGCTCGC TCACTGAGGCCGGGCGACCAAAGGTCGCCCGACGCCC GGGCGGCCTCAGTGAGCGAGCGAGCGCGCAGCTGCCT GCAGG |
| 25 | Packaging plasmid pKL-Kan-pLP2 | GGCCGCGTTGCTGGCGTTTTTCCATAGGCTCCGCCCCC CTGACGAGCATCACAAAAATCGACGCTCAAGTCAGAG GTGGCGAAACCCGACAGGACTATAAAGATACCAGGCG TTTCCCCCTGGAAGCTCCCTCGTGCGCTCTCCTGTTCCG ACCCTGCCGCTTACCGGATACCTGTCCGCCTTTCTCCCT TCGGGAAGCGTGGCGCTTTCTCATAGCTCACGCTGTAG |
| | | GTATCTCAGTTCGGTGTAGGTCGTTCGCTCCAAGCTGG GCTGTGTGCACGAACCCCCCGTTCAGCCCGACCGCTGC GCCTTATCCGGTAACTATCGTCTTGAGTCCAACCCGGT AAGACACGACTTATCGCCACTGGCAGCAGCCACTGGTA ACAGGATTAGCAGAGCGAGGTATGTAGGCGGTGCTAC AGAGTTCTTGAAGTGGTGGCCTAACTACGGCTACACTA GAAGAACAGTATTTGGTATCTGCGCTCTGCTGAAGCCA GTTACCTTCGGAAAAAGAGTTGGTAGCTCTTGATCCGG CAAACAAACCACCGCTGGTAGCGGTGGTTTTTTTGTTT GCAAGCAGCAGATTACGCGCAGAAAAAAAGGATCTCA AGAAGATCCTTTGATCTTTTCTACGGGGTCTGACGCTC AGTGGAACGAAAACTCACGTTAAGGGATTTTGGTCATG Aagcgcttttgaagctcggatccgaacaaacgacccaacacccgtgcgttttattctgtcttt ttattgccgatccccTCAGAAGAACTCGTCAAGAAGGCGATAG AAGGCGATGCGCTGCGAATCGGGAGCGGCGATACCGT AAAGCACGAGGAAGCGGTCAGCCCATTCGCCGCCAAG CTCTTCAGCAATATCACGGGTAGCCAACGCTATGTCCT GATAGCGGTCCGCCACACCCAGCCGGCCACAGTCGAT GAATCCAGAAAAGCGGCCATTTTCCACCATGATATTCG GCAAGCAGGCATCGCCATGGGTCACGACGAGATCCTC GCCGTCGGGCATGCTCGCCTTGAGCCTGGCGAACAGTT CGGCTGGCGCGAGCCCCTGATGCTCTTCGTCCAGATCA TCCTGATCGACAAGACCGGCTTCCATCCGAGTACGTGC TCGCTCGATGCGATGTTTCGCTTGGTGGTCGAATGGGC AGGTAGCCGGATCAAGCGTATGCAGCCGCCGCATTGC ATCAGCCATGATGGATACTTTCTCGGCAGGAGCAAGGT GAGATGACAGGAGATCCTGCCCCGGCACTTCGCCCAAT AGCAGCCAGTCCCTTCCCGCTTCAGTGACAACGTCGAG CACAGCTGCGCAAGGAACGCCCGTCGTGGCCAGCCAC GATAGCCGCGCTGCCTCGTCTTGCAGTTCATTCAGGGC ACCGGACAGGTCGGTCTTGACAAAAAGAACCGGGCGC |
| | | CCCTGCGCTGACAGCCGGAACACGGCGGCATCAGAGC AGCCGATTGTCTGTTGTGCCCAGTCATAGCCGAATAGC CTCTCCACCCAAGCGGCCGGAGAACCTGCGTGCAATCC ATCTTGTTCAATCATGCGAAACGATCCTCATCCTGTCTC TTGATCAGAGCTTGATCCCCTGCGCCATCAGATCCTTG GCGGCAAGAAAGCCATCCAGTTTACTTTGCAGGGCTTC CCAACCTTACCAGAGGCCTGCGCCGCGGCCAGCTGGCT AGCAATTCCCGGGTTAACTCTAGAGaatgtagtcttatgcaatactct tgtagtcttgcaacatggtaacgatgagttagcaacatgccttacaaggagagaaaaagca ccgtgcatgccgattggtggaagtaaggtggtacgatcgtgccttattaggaaggcaacag acgggtctgacatggattggacgaaccactgaattccgcattgcagagatattgtatttaagt gcctagctcgatacaataaacgccatttgaccattcaccacattggtgtgcacctccaagctc gagctcgtttagtgaaccgtcagatcgcctggagacgccatccacgctgttttgacctccat agaagacaccgggaccgatccagcctcccctcgaagctagtcgattaggcatctcctatgg caggaagaagcggagacagcgacgaagacctcctcaaggcagtcagactcatcaagttt ctctatcaaagcaacccacctcccaatcccgaggggacccgacaggcccgaaggaatag aagaagaaggtggagagagagacagagacagatccattcgattagtgaacggatccttag cacttatctgggacgatctgcggagcctgtgcctcttcagctaccaccgcttgagagactta ctcttgattgtaacgaggattgtggaacttctgggacgcagggggtgggaagccctcaaat attggtggaatctcctacaatattggagtcaggagctaaagaatagtgctgttagcttgctca atgccacagctatagcagtagctgaggggacagatagggttatagaagtagtacaagaag cttggcactggccgtcgttttacaacgtcgtgatctgagcctgggagatctctggctaactag ggaacccactgcttaagcctcaataaagcttgccttgagtgcttcaagtagtgtgtgcccgtc tgttgtgtgactctggtaactagagatcaggaaaaccctggcgttacccaacttaatcgcctt gcagcacatccccctttcgccagctggcgtaatagcgaagaggcccgcaccgatcgccct tcccaacagttgcgcagcctgaatggcgaatggcgcctgatgcggtattttctccttacgcat ctgtgcggtatttcacaccgcatacgtcaaagcaaccatagtgtcgacCATTACTTA TTGTTTTAGCTGTCCTCATGAATGTCTTTTCACTACCCA TTTGCTTATCCTGCATCTCTCAGCCTTGACTCCACTCAG TTCTCTTGCTTAGAGATACCACCTTTCCCCTGAAGTGTT CCTTCCATGTTTTACGGCGAGATGGTTTCTCCTCGCCTG |
| | | GCCACTCAGCCTTAGTTGTCTCTGTTGTCTTATAGAGGT CTACTTGAAGAAGGAAAAACAGGGGGCATGGTTTGAC TGTCCTGTGAGCCCTTCTTCCCTGCCTCCCCCACTCACA GTGACCCGGAATCCCTCGACATGGCAGTCTAGCACTAG TGCGGCCGCAGATCTGCTTCCTCGCTCACTGACTCGCT GCGCTCGGTCGTTCGGCTGCGGCGAGCGGTATCAGCTC ACTCAAAGGCGGTAATACGGTTATCCACAGAATCAGG GGATAACGCAGGAAAGAACATGTGAGCAAAAGGCCAG CAAAAGGCCAGGAACCGTAAAAA |
| 26 | Packaging plasmid pKL-Kan- pLP1 | GGCCGCGTTGCTGGCGTTTTTCCATAGGCTCCGCCCCC CTGACGAGCATCACAAAAATCGACGCTCAAGTCAGAG GTGGCGAAACCCGACAGGACTATAAAGATACCAGGCG TTTCCCCCTGGAAGCTCCCTCGTGCGCTCTCCTGTTCCG ACCCTGCCGCTTACCGGATACCTGTCCGCCTTTCTCCCT TCGGGAAGCGTGGCGCTTTCTCATAGCTCACGCTGTAG GTATCTCAGTTCGGTGTAGGTCGTTCGCTCCAAGCTGG GCTGTGTGCACGAACCCCCCGTTCAGCCCGACCGCTGC GCCTTATCCGGTAACTATCGTCTTGAGTCCAACCCGGT AAGACACGACTTATCGCCACTGGCAGCAGCCACTGGTA ACAGGATTAGCAGAGCGAGGTATGTAGGCGGTGCTAC AGAGTTCTTGAAGTGGTGGCCTAACTACGGCTACACTA GAAGAACAGTATTTGGTATCTGCGCTCTGCTGAAGCCA GTTACCTTCGGAAAAAGAGTTGGTAGCTCTTGATCCGG CAAACAAACCACCGCTGGTAGCGGTGGTTTTTTTGTTT GCAAGCAGCAGATTACGCGCAGAAAAAAAGGATCTCA AGAAGATCCTTTGATCTTTTCTACGGGGTCTGACGCTC AGTGGAACGAAAACTCACGTTAAGGGATTTTGGTCATG Aagcgcttttgaagctcggatccgaacaaacgacccaacacccgtgcgttttattctgtcttt ttattgccgatccccTCAGAAGAACTCGTCAAGAAGGCGATAG AAGGCGATGCGCTGCGAATCGGGAGCGGCGATACCGT AAAGCACGAGGAAGCGGTCAGCCCATTCGCCGCCAAG |
| | | CTCTTCAGCAATATCACGGGTAGCCAACGCTATGTCCT GATAGCGGTCCGCCACACCCAGCCGGCCACAGTCGAT GAATCCAGAAAAGCGGCCATTTTCCACCATGATATTCG GCAAGCAGGCATCGCCATGGGTCACGACGAGATCCTC GCCGTCGGGCATGCTCGCCTTGAGCCTGGCGAACAGTT CGGCTGGCGCGAGCCCCTGATGCTCTTCGTCCAGATCA TCCTGATCGACAAGACCGGCTTCCATCCGAGTACGTGC TCGCTCGATGCGATGTTTCGCTTGGTGGTCGAATGGGC AGGTAGCCGGATCAAGCGTATGCAGCCGCCGCATTGC ATCAGCCATGATGGATACTTTCTCGGCAGGAGCAAGGT GAGATGACAGGAGATCCTGCCCCGGCACTTCGCCCAAT AGCAGCCAGTCCCTTCCCGCTTCAGTGACAACGTCGAG CACAGCTGCGCAAGGAACGCCCGTCGTGGCCAGCCAC GATAGCCGCGCTGCCTCGTCTTGCAGTTCATTCAGGGC ACCGGACAGGTCGGTCTTGACAAAAAGAACCGGGCGC CCCTGCGCTGACAGCCGGAACACGGCGGCATCAGAGC AGCCGATTGTCTGTTGTGCCCAGTCATAGCCGAATAGC CTCTCCACCCAAGCGGCCGGAGAACCTGCGTGCAATCC ATCTTGTTCAATCATGCGAAACGATCCTCATCCTGTCTC TTGATCAGAGCTTGATCCCCTGCGCCATCAGATCCTTG GCGGCAAGAAAGCCATCCAGTTTACTTTGCAGGGCTTC CCAACCTTACCAGAGGCCTGCGCCGCGGCCAGCTGGCT AGCAATTCCCGGGTTAACTCTAGAGACATTGATTATTG ACTAGTTATTAATAGTAATCAATTACGGGGTCATTAGT TCATAGCCCATATATGGAGTTCCGCGTTACATAACTTA CGGTAAATGGCCCGCCTGGCTGACCGCCCAACGACCCC CGCCCATTGACGTCAATAATGACGTATGTTCCCATAGT AACGCCAATAGGGACTTTCCATTGACGTCAATGGGTGG AGTATTTACGGTAAACTGCCCACTTGGCAGTACATCAA GTGTATCATATGCCAAGTACGCCCCCTATTGACGTCAA TGACGGTAAATGGCCCGCCTGGCATTATGCCCAGTACA |
| | | TGACCTTATGGGACTTTCCTACTTGGCAGTACATCTAC GTATTAGTCATCGCTATTACCATGGTGATGCGGTTTTG GCAGTACATCAATGGGCGTGGATAGCGGTTTGACTCAC GGGGATTTCCAAGTCTCCACCCCATTGACGTCAATGGG AGTTTGTTTTGGCACCAAAATCAACGGGACTTTCCAAA ATGTCGTAACAACTCCGCCCCATTGACGCAAATGGGCG GTAGGCGTGTACGGTGGGAGGTCTATATAAGCAGAGC TCGTTTAGTGAACCGTCAGATCGCCTGGAGACGCCATC CACGCTGTTTTGACCTCCATAGAAGACACCGGGACCGA TCCAGCCTCCCCTCGAAGCTTACATGTGGTACCGAGCT CGGATCCTGAGAACTTCAGGGTGAGTCTATGGGACCCT TGATGTTTTCTTTCCCCTTCTTTTCTATGGTTAAGTTCAT GTCATAGGAAGGGGAGAAGTAACAGGGTACACATATT GACCAAATCAGGGTAATTTTGCATTTGTAATTTTAAAA AATGCTTTCTTCTTTTAATATACTTTTTTGTTTATCTTAT TTCTAATACTTTCCCTAATCTCTTTCTTTCAGGGCAATA ATGATACAATGTATCATGCCTCTTTGCACCATTCTAAA GAATAACAGTGATAATTTCTGGGTTAAGGCAATAGCAA TATTTCTGCATATAAATATTTCTGCATATAAATTGTAAC TGATGTAAGAGGTTTCATATTGCTAATAGCAGCTACAA TCCAGCTACCATTCTGCTTTTATTTTATGGTTGGGATAA GGCTGGATTATTCTGAGTCCAAGCTAGGCCCTTTTGCT AATCATGTTCATACCTCTTATCTTCCTCCCACAGCTCCT GGGCAACGTGCTGGTCTGTGTGCTGGCCCATCACTTTG GCAAAGCACGTGAGATCTGAATTCGAGATCTGCCGCCG CCATGGGTGCGAGAGCGTCAGTATTAAGCGGGGGAGA ATTAGATCGATGGGAAAAAATTCGGTTAAGGCCAGGG GGAAAGAAAAAATATAAATTAAAACATATAGTATGGG CAAGCAGGGAGCTAGAACGATTCGCAGTTAATCCTGG CCTGTTAGAAACATCAGAAGGCTGTAGACAAATACTG GGACAGCTACAACCATCCCTTCAGACAGGATCAGAAG |
| | | AACTTAGATCATTATATAATACAGTAGCAACCCTCTAT TGTGTGCATCAAAGGATAGAGATAAAAGACACCAAGG AAGCTTTAGACAAGATAGAGGAAGAGCAAAACAAAAG TAAGAAAAAAGCACAGCAAGCAGCAGCTGACACAGGA CACAGCAATCAGGTCAGCCAAAATTACCCTATAGTGCA GAACATCCAGGGGCAAATGGTACATCAGGCCATATCA CCTAGAACTTTAAATGCATGGGTAAAAGTAGTAGAAG AGAAGGCTTTCAGCCCAGAAGTGATACCCATGTTTTCA GCATTATCAGAAGGAGCCACCCCACAAGATTTAAACA CCATGCTAAACACAGTGGGGGGACATCAAGCAGCCAT GCAAATGTTAAAAGAGACCATCAATGAGGAAGCTGCA GAATGGGATAGAGTGCATCCAGTGCATGCAGGGCCTA TTGCACCAGGCCAGATGAGAGAACCAAGGGGATCAGA CATCGCTGGAACTACTAGTACCCTTCAGGAACAAATAG GATGGATGACACATAATCCACCTATCCCAGTAGGAGA AATCTATAAAAGATGGATAATCCTGGGATTAAATAAA ATAGTAAGAATGTATAGCCCTACCAGCATTCTGGACAT AAGACAAGGACCAAAGGAACCCTTTAGAGACTATGTA GACCGATTCTATAAAACTCTAAGAGCCGAGCAAGCTTC ACAAGAGGTAAAAAATTGGATGACAGAAACCTTGTTG GTCCAAAATGCGAACCCAGATTGTAAGACTATTTTAAA AGCATTGGGACCAGGAGCGACACTAGAAGAAATGATG ACAGCATGTCAGGGAGTGGGGGGACCCGGCCATAAAG CAAGAGTTTTGGCTGAAGCAATGAGCCAAGTAACAAA TCCAGCTACCATAATGATACAGAAAGGCAATTTTAGGA ACCAAAGAAAGACTGTTAAGTGTTTCAATTGTGGCAAA GAAGGGCACATAGCCAAAAATTGCAGGGCCCCTAGGA AAAAGGGCTGTTGGAAATGTGGAAAGGAAGGACACCA AATGAAAGATTGTACTGAGAGACAGGCTAATTTTTTAG GGAAGATCTGGCCTTCCCACAAGGGAAGGCCAGGGAA TTTTCTTCAGAGCAGACCAGAGCCAACAGCCCCACCAG |
| | | AAGAGAGCTTCAGGTTTGGGGAAGAGACAACAACTCC CTCTCAGAAGCAGGAGCCGATAGACAAGGAACTGTAT CCTTTAGCTTCCCTCAGATCACTCTTTGGCAGCGACCCC TCGTCACAATAAAGATAGGGGGGCAATTAAAGGAAGC TCTATTAGATACTGGTGCTGACGACACAGTATTAGAAG AAATGAATTTGCCAGGAAGATGGAAACCAAAAATGAT AGGGGGAATTGGAGGTTTTATCAAAGTAAGACAGTAT GATCAGATACTCATAGAAATCTGCGGACATAAAGCTAT AGGTACAGTATTAGTAGGACCTACACCTGTCAACATAA TTGGAAGAAATCTGTTGACTCAGATTGGCTGCACTTTA AATTTTCCCATTAGTCCTATTGAGACTGTACCAGTAAA ATTAAAGCCAGGAATGGATGGCCCAAAAGTTAAACAA TGGCCATTGACAGAAGAAAAAATAAAAGCATTAGTAG AAATTTGTACAGAAATGGAAAAGGAAGGAAAAATTTC AAAAATTGGGCCTGAAAATCCATACAATACTCCAGTAT TTGCCATAAAGAAAAAAGACAGTACTAAATGGAGAAA ATTAGTAGATTTCAGAGAACTTAATAAGAGAACTCAAG ATTTCTGGGAAGTTCAATTAGGAATACCACATCCTGCA GGGTTAAAACAGAAAAAATCAGTAACAGTACTGGATG TGGGCGATGCATATTTTTCAGTTCCCTTAGATAAAGAC TTCAGGAAGTATACTGCATTTACCATACCTAGTATAAA CAATGAGACACCAGGGATTAGATATCAGTACAATGTG CTTCCACAGGGATGGAAAGGATCACCAGCAATATTCCA GTGTAGCATGACAAAAATCTTAGAGCCTTTTAGAAAAC AAAATCCAGACATAGTCATCTATCAATACATGGATGAT TTGTATGTAGGATCTGACTTAGAAATAGGGCAGCATAG AACAAAAATAGAGGAACTGAGACAACATCTGTTGAGG TGGGGATTTACCACACCAGACAAAAAACATCAGAAAG AACCTCCATTCCTTTGGATGGGTTATGAACTCCATCCTG ATAAATGGACAGTACAGCCTATAGTGCTGCCAGAAAA GGACAGCTGGACTGTCAATGACATACAGAAATTAGTG |
| | | GGAAAATTGAATTGGGCAAGTCAGATTTATGCAGGGA TTAAAGTAAGGCAATTATGTAAACTTCTTAGGGGAACC AAAGCACTAACAGAAGTAGTACCACTAACAGAAGAAG CAGAGCTAGAACTGGCAGAAAACAGGGAGATTCTAAA AGAACCGGTACATGGAGTGTATTATGACCCATCAAAA GACTTAATAGCAGAAATACAGAAGCAGGGGCAAGGCC AATGGACATATCAAATTTATCAAGAGCCATTTAAAAAT CTGAAAACAGGAAAGTATGCAAGAATGAAGGGTGCCC ACACTAATGATGTGAAACAATTAACAGAGGCAGTACA AAAAATAGCCACAGAAAGCATAGTAATATGGGGAAAG ACTCCTAAATTTAAATTACCCATACAAAAGGAAACATG GGAAGCATGGTGGACAGAGTATTGGCAAGCCACCTGG ATTCCTGAGTGGGAGTTTGTCAATACCCCTCCCTTAGT GAAGTTATGGTACCAGTTAGAGAAAGAACCCATAATA GGAGCAGAAACTTTCTATGTAGATGGGGCAGCCAATA GGGAAACTAAATTAGGAAAAGCAGGATATGTAACTGA CAGAGGAAGACAAAAAGTTGTCCCCCTAACGGACACA ACAAATCAGAAGACTGAGTTACAAGCAATTCATCTAGC TTTGCAGGATTCGGGATTAGAAGTAAACATAGTGACAG ACTCACAATATGCATTGGGAATCATTCAAGCACAACCA GATAAGAGTGAATCAGAGTTAGTCAGTCAAATAATAG AGCAGTTAATAAAAAAGGAAAAAGTCTACCTGGCATG GGTACCAGCACACAAAGGAATTGGAGGAAATGAACAA GTAGATAAATTGGTCAGTGCTGGAATCAGGAAAGTACT ATTTTTAGATGGAATAGATAAGGCCCAAGAAGAACAT GAGAAATATCACAGTAATTGGAGAGCAATGGCTAGTG ATTTTAACCTACCACCTGTAGTAGCAAAAGAAATAGTA GCCAGCTGTGATAAATGTCAGCTAAAAGGGGAAGCCA TGCATGGACAAGTAGACTGTAGCCCAGGAATATGGCA GCTAGATTGTACACATTTAGAAGGAAAAGTTATCTTGG TAGCAGTTCATGTAGCCAGTGGATATATAGAAGCAGA |
| | | AGTAATTCCAGCAGAGACAGGGCAAGAAACAGCATAC TTCCTCTTAAAATTAGCAGGAAGATGGCCAGTAAAAAC AGTACATACAGACAATGGCAGCAATTTCACCAGTACTA CAGTTAAGGCCGCCTGTTGGTGGGCGGGGATCAAGCA GGAATTTGGCATTCCCTACAATCCGCAGTCACAAGGAG TAATAGAATCTATGAATAAAGAATTAAAGAAAATTAT AGGACAGGTAAGAGATCAGGCTGAACATCTTAAAACA GCAGTACAAATGGCAGTATTCATCCACAATTTTAAAAG AAAAGGGGGGATTGGGGGGTACAGTGCAGGGGAAAG AATAGTAGACATAATAGCAACAGACATACAAACTAAA GAATTACAAAAACAAATTACAAAAATTCAAAATTTTCG GGTTTATTACAGGGACAGCAGAGATCCAGTTTGGAAA GGACCAGCAAAGCTCCTCTGGAAAGGTGAAGGGGCAG TAGTAATACAAGATAATAGTGACATAAAAGTAGTGCC AAGAAGAAAAGCAAAGATCATCAGGGATTATGGAAAA CAGATGGCAGGTGATGATTGTGTGGCAAGTAGACAGG ATGAGGATTAACACATGGAATTCCGGAGCGGCCGCAG GAGCTTTGTTCCTTGGGTTCTTGGGAGCAGCAGGAAGC ACTATGGGCGCAGCGTCAATGACGCTGACGGTACAGG CCAGACAATTATTGTCTGGTATAGTGCAGCAGCAGAAC AATTTGCTGAGGGCTATTGAGGCGCAACAGCATCTGTT GCAACTCACAGTCTGGGGCATCAAGCAGCTCCAGGCA AGAATCCTGGCTGTGGAAAGATACCTAAAGGATCAAC AGCTCCTGGGGATTTGGGGTTGCTCTGGAAAACTCATT TGCACCACTGCTGTGCCTTGGAATGCTAGTTGGAGTAA TAAATCTCTGGAACAGATTTGGAATCACACGACCTGGA TGGAGTGGGACAGAGAAATTAACAATTACACAAGCTT CCGCGGAATTCACCCCACCAGTGCAGGCTGCCTATCAG AAAGTGGTGGCTGGTGTGGCTAATGCCCTGGCCCACAA GTATCACTAAGCTCGCTTTCTTGCTGTCCAATTTCTATT AAAGGTTCCTTTGTTCCCTAAGTCCAACTACTAAACTG |
| | | GGGGATATTATGAAGGGCCTTGAGCATCTGGATTCTGC CTAATAAAAAACATTTATTTTCATTGCAATGATGTATTT AAATTATTTCTGAATATTTTACTAAAAAGGGAATGTGG GAGGTCAGTGCATTTAAAACATAAAGAAATGAAGAGC TAGTTCAAACCTTGGGAAAATACACTATATCTTAAACT CCATGAAAGAAGGTGAGGCTGCAAACAGCTAATGCAC ATTGGCAACAGCCCCTGATGCCTATGCCTTATTCATCC CTCAGAAAAGGATTCAAGTAGAGGCTTGATTTGGAGGT TAAAGTTTTGCTATGCTGTATTTTACATTACTTATTGTT TTAGCTGTCCTCATGAATGTCTTTTCACTACCCATTTGC TTATCCTGCATCTCTCAGCCTTGACTCCACTCAGTTCTC TTGCTTAGAGATACCACCTTTCCCCTGAAGTGTTCCTTC CATGTTTTACGGCGAGATGGTTTCTCCTCGCCTGGCCA CTCAGCCTTAGTTGTCTCTGTTGTCTTATAGAGGTCTAC TTGAAGAAGGAAAAACAGGGGGCATGGTTTGACTGTC CTGTGAGCCCTTCTTCCCTGCCTCCCCCACTCACAGTGA CCCGGAATCCCTCGACATGGCAGTCTAGCACTAGTGCG GCCGCAGATCTGCTTCCTCGCTCACTGACTCGCTGCGC TCGGTCGTTCGGCTGCGGCGAGCGGTATCAGCTCACTC AAAGGCGGTAATACGGTTATCCACAGAATCAGGGGAT AACGCAGGAAAGAACATGTGAGCAAAAGGCCAGCAAA AGGCCAGGAACCGTAAAAAgtcgacCATTACTTATTGTTT TAGCTGTCCTCATGAATGTCTTTTCACTACCCATTTGCT TATCCTGCATCTCTCAGCCTTGACTCCACTCAGTTCTCT TGCTTAGAGATACCACCTTTCCCCTGAAGTGTTCCTTCC ATGTTTTACGGCGAGATGGTTTCTCCTCGCCTGGCCACT CAGCCTTAGTTGTCTCTGTTGTCTTATAGAGGTCTACTT GAAGAAGGAAAAACAGGGGGCATGGTTTGACTGTCCT GTGAGCCCTTCTTCCCTGCCTCCCCCACTCACAGTGACC CGGAATCCCTCGACATGGCAGTCTAGCACTAGTGCGGC CGCAGATCTGCTTCCTCGCTCACTGACTCGCTGCGCTC |
| | | GGTCGTTCGGCTGCGGCGAGCGGTATCAGCTCACTCAA AGGCGGTAATACGGTTATCCACAGAATCAGGGGATAA CGCAGGAAAGAACATGTGAGCAAAAGGCCAGCAAAAG GCCAGGAACCGTAAAAA |
| 27 | Envelope plasmid pKL-Kan-Vsvg | GGCCGCGTTGCTGGCGTTTTTCCATAGGCTCCGCCCCC CTGACGAGCATCACAAAAATCGACGCTCAAGTCAGAG GTGGCGAAACCCGACAGGACTATAAAGATACCAGGCG TTTCCCCCTGGAAGCTCCCTCGTGCGCTCTCCTGTTCCG ACCCTGCCGCTTACCGGATACCTGTCCGCCTTTCTCCCT TCGGGAAGCGTGGCGCTTTCTCATAGCTCACGCTGTAG GTATCTCAGTTCGGTGTAGGTCGTTCGCTCCAAGCTGG GCTGTGTGCACGAACCCCCCGTTCAGCCCGACCGCTGC GCCTTATCCGGTAACTATCGTCTTGAGTCCAACCCGGT AAGACACGACTTATCGCCACTGGCAGCAGCCACTGGTA ACAGGATTAGCAGAGCGAGGTATGTAGGCGGTGCTAC AGAGTTCTTGAAGTGGTGGCCTAACTACGGCTACACTA GAAGAACAGTATTTGGTATCTGCGCTCTGCTGAAGCCA GTTACCTTCGGAAAAAGAGTTGGTAGCTCTTGATCCGG CAAACAAACCACCGCTGGTAGCGGTGGTTTTTTTGTTT GCAAGCAGCAGATTACGCGCAGAAAAAAAGGATCTCA AGAAGATCCTTTGATCTTTTCTACGGGGTCTGACGCTC AGTGGAACGAAAACTCACGTTAAGGGATTTTGGTCATG Aagcgcttttgaagctcggatccgaacaaacgacccaacacccgtgcgttttattctgtcttt ttattgccgatccccTCAGAAGAACTCGTCAAGAAGGCGATAG AAGGCGATGCGCTGCGAATCGGGAGCGGCGATACCGT AAAGCACGAGGAAGCGGTCAGCCCATTCGCCGCCAAG CTCTTCAGCAATATCACGGGTAGCCAACGCTATGTCCT GATAGCGGTCCGCCACACCCAGCCGGCCACAGTCGAT GAATCCAGAAAAGCGGCCATTTTCCACCATGATATTCG GCAAGCAGGCATCGCCATGGGTCACGACGAGATCCTC GCCGTCGGGCATGCTCGCCTTGAGCCTGGCGAACAGTT |
| | | CGGCTGGCGCGAGCCCCTGATGCTCTTCGTCCAGATCA TCCTGATCGACAAGACCGGCTTCCATCCGAGTACGTGC TCGCTCGATGCGATGTTTCGCTTGGTGGTCGAATGGGC AGGTAGCCGGATCAAGCGTATGCAGCCGCCGCATTGC ATCAGCCATGATGGATACTTTCTCGGCAGGAGCAAGGT GAGATGACAGGAGATCCTGCCCCGGCACTTCGCCCAAT AGCAGCCAGTCCCTTCCCGCTTCAGTGACAACGTCGAG CACAGCTGCGCAAGGAACGCCCGTCGTGGCCAGCCAC GATAGCCGCGCTGCCTCGTCTTGCAGTTCATTCAGGGC ACCGGACAGGTCGGTCTTGACAAAAAGAACCGGGCGC CCCTGCGCTGACAGCCGGAACACGGCGGCATCAGAGC AGCCGATTGTCTGTTGTGCCCAGTCATAGCCGAATAGC CTCTCCACCCAAGCGGCCGGAGAACCTGCGTGCAATCC ATCTTGTTCAATCATGCGAAACGATCCTCATCCTGTCTC TTGATCAGAGCTTGATCCCCTGCGCCATCAGATCCTTG GCGGCAAGAAAGCCATCCAGTTTACTTTGCAGGGCTTC CCAACCTTACCAGAGGCCTGCGCCGCGGCCAGCTGGCT AGCAATTCCCGGGTTAACTCTAGAGACATTGATTATTG ACTAGTTATTAATAGTAATCAATTACGGGGTCATTAGT TCATAGCCCATATATGGAGTTCCGCGTTACATAACTTA CGGTAAATGGCCCGCCTGGCTGACCGCCCAACGACCCC CGCCCATTGACGTCAATAATGACGTATGTTCCCATAGT AACGCCAATAGGGACTTTCCATTGACGTCAATGGGTGG AGTATTTACGGTAAACTGCCCACTTGGCAGTACATCAA GTGTATCATATGCCAAGTACGCCCCCTATTGACGTCAA TGACGGTAAATGGCCCGCCTGGCATTATGCCCAGTACA TGACCTTATGGGACTTTCCTACTTGGCAGTACATCTAC GTATTAGTCATCGCTATTACCATGGTGATGCGGTTTTG GCAGTACATCAATGGGCGTGGATAGCGGTTTGACTCAC GGGGATTTCCAAGTCTCCACCCCATTGACGTCAATGGG AGTTTGTTTTGGCACCAAAATCAACGGGACTTTCCAAA |
| | | ATGTCGTAACAACTCCGCCCCATTGACGCAAATGGGCG GTAGGCGTGTACGGTGGGAGGTCTATATAAGCAGAGC TCGTTTAGTGAACCGTCAGATCGCCTGGAGACGCCATC CACGCTGTTTTGACCTCCATAGAAGACACCGGGACCGA TCCAGCCTCCCCTCGAAGCTTACATGTGGTACCGAGCT CGGATCCTGAGAACTTCAGGGTGAGTCTATGGGACCCT TGATGTTTTCTTTCCCCTTCTTTTCTATGGTTAAGTTCAT GTCATAGGAAGGGGAGAAGTAACAGGGTACACATATT GACCAAATCAGGGTAATTTTGCATTTGTAATTTTAAAA AATGCTTTCTTCTTTTAATATACTTTTTTGTTTATCTTAT TTCTAATACTTTCCCTAATCTCTTTCTTTCAGGGCAATA ATGATACAATGTATCATGCCTCTTTGCACCATTCTAAA GAATAACAGTGATAATTTCTGGGTTAAGGCAATAGCAA TATTTCTGCATATAAATATTTCTGCATATAAATTGTAAC TGATGTAAGAGGTTTCATATTGCTAATAGCAGCTACAA TCCAGCTACCATTCTGCTTTTATTTTATGGTTGGGATAA GGCTGGATTATTCTGAGTCCAAGCTAGGCCCTTTTGCT AATCATGTTCATACCTCTTATCTTCCTCCCACAGCTCCT GGGCAACGTGCTGGTCTGTGTGCTGGCCCATCACTTTG GCAAAGCACGTGAGATCTGAATTCTGACACTATGAAGT GCCTTTTGTACTTAGCCTTTTTATTCATTGGGGTGAATT GCAAGTTCACCATAGTTTTTCCACACAACCAAAAAGGA AACTGGAAAAATGTTCCTTCTAATTACCATTATTGCCC GTCAAGCTCAGATTTAAATTGGCATAATGACTTAATAG GCACAGCCTTACAAGTCAAAATGCCCAAGAGTCACAA GGCTATTCAAGCAGACGGTTGGATGTGTCATGCTTCCA AATGGGTCACTACTTGTGATTTCCGCTGGTATGGACCG AAGTATATAACACATTCCATCCGATCCTTCACTCCATCT GTAGAACAATGCAAGGAAAGCATTGAACAAACGAAAC AAGGAACTTGGCTGAATCCAGGCTTCCCTCCTCAAAGT TGTGGATATGCAACTGTGACGGATGCCGAAGCAGTGAT |
| | | TGTCCAGGTGACTCCTCACCATGTGCTGGTTGATGAAT ACACAGGAGAATGGGTTGATTCACAGTTCATCAACGG AAAATGCAGCAATTACATATGCCCCACTGTCCATAACT CTACAACCTGGCATTCTGACTATAAGGTCAAAGGGCTA TGTGATTCTAACCTCATTTCCATGGACATCACCTTCTTC TCAGAGGACGGAGAGCTATCATCCCTGGGAAAGGAGG GCACAGGGTTCAGAAGTAACTACTTTGCTTATGAAACT GGAGGCAAGGCCTGCAAAATGCAATACTGCAAGCATT GGGGAGTCAGACTCCCATCAGGTGTCTGGTTCGAGATG GCTGATAAGGATCTCTTTGCTGCAGCCAGATTCCCTGA ATGCCCAGAAGGGTCAAGTATCTCTGCTCCATCTCAGA CCTCAGTGGATGTAAGTCTAATTCAGGACGTTGAGAGG ATCTTGGATTATTCCCTCTGCCAAGAAACCTGGAGCAA AATCAGAGCGGGTCTTCCAATCTCTCCAGTGGATCTCA GCTATCTTGCTCCTAAAAACCCAGGAACCGGTCCTGCT TTCACCATAATCAATGGTACCCTAAAATACTTTGAGAC CAGATACATCAGAGTCGATATTGCTGCTCCAATCCTCT CAAGAATGGTCGGAATGATCAGTGGAACTACCACAGA AAGGGAACTGTGGGATGACTGGGCACCATATGAAGAC GTGGAAATTGGACCCAATGGAGTTCTGAGGACCAGTTC AGGATATAAGTTTCCTTTATACATGATTGGACATGGTA TGTTGGACTCCGATCTTCATCTTAGCTCAAAGGCTCAG GTGTTCGAACATCCTCACATTCAAGACGCTGCTTCGCA ACTTCCTGATGATGAGAGTTTATTTTTTGGTGATACTGG GCTATCCAAAAATCCAATCGAGCTTGTAGAAGGTTGGT TCAGTAGTTGGAAAAGCTCTATTGCCTCTTTTTTCTTTA TCATAGGGTTAATCATTGGACTATTCTTGGTTCTCCGAG TTGGTATCCATCTTTGCATTAAATTAAAGCACACCAAG AAAAGACAGATTTATACAGACATAGAGATGAACCGAC TTGGAAAGTAACTCAAATCCTGCACAACAGATTCTTCA TGTTTGGACCAAATCAACTTGTGATACCATGCTCAAAG |
| | | AGGCCTCAATTATATTTGAGTTTTTAATTTTTATGAAAA AAAAAAAAAAAAACGGAATTCACCCCACCAGTGCAGG CTGCCTATCAGAAAGTGGTGGCTGGTGTGGCTAATGCC CTGGCCCACAAGTATCACTAAGCTCGCTTTCTTGCTGTC CAATTTCTATTAAAGGTTCCTTTGTTCCCTAAGTCCAAC TACTAAACTGGGGGATATTATGAAGGGCCTTGAGCATC TGGATTCTGCCTAATAAAAAACATTTATTTTCATTGCA ATGATGTATTTAAATTATTTCTGAATATTTTACTAAAAA GGGAATGTGGGAGGTCAGTGCATTTAAAACATAAAGA AATGAAGAGCTAGTTCAAACCTTGGGAAAATACACTAT ATCTTAAACTCCATGAAAGAAGGTGAGGCTGCAAACA GCTAATGCACATTGGCAACAGCCCCTGATGCCTATGCC TTATTCATCCCTCAGAAAAGGATTCAAGTAGAGGCTTG ATTTGGAGGTTAAAGTTTTGCTATGCTGTATTTTAgtcgac CATTACTTATTGTTTTAGCTGTCCTCATGAATGTCTTTT CACTACCCATTTGCTTATCCTGCATCTCTCAGCCTTGAC TCCACTCAGTTCTCTTGCTTAGAGATACCACCTTTCCCC TGAAGTGTTCCTTCCATGTTTTACGGCGAGATGGTTTCT CCTCGCCTGGCCACTCAGCCTTAGTTGTCTCTGTTGTCT TATAGAGGTCTACTTGAAGAAGGAAAAACAGGGGGCA TGGTTTGACTGTCCTGTGAGCCCTTCTTCCCTGCCTCCC CCACTCACAGTGACCCGGAATCCCTCGACATGGCAGTC TAGCACTAGTGCGGCCGCAGATCTGCTTCCTCGCTCAC TGACTCGCTGCGCTCGGTCGTTCGGCTGCGGCGAGCGG TATCAGCTCACTCAAAGGCGGTAATACGGTTATCCACA GAATCAGGGGATAACGCAGGAAAGAACATGTGAGCAA AAGGCCAGCAAAAGGCCAGGAACCGTAAAAA |
| 28 | Nucleotide sequence of the CBH promoter | CGTTACATAACTTACGGTAAATGGCCCGCCTGGCTGAC CGCCCAACGACCCCCGCCCATTGACGTCAATAATGACG TATGTTCCCATAGTAACGCCAATAGGGACTTTCCATTG ACGTCAATGGGTGGAGTATTTACGGTAAACTGCCCACT |
| | | TGGCAGTACATCAAGTGTATCATATGCCAAGTACGCCC CCTATTGACGTCAATGACGGTAAATGGCCCGCCTGGCA TTATGCCCAGTACATGACCTTATGGGACTTTCCTACTTG GCAGTACATCTACGTATTAGTCATCGCTATTACCATGG TCGAGGTGAGCCCCACGTTCTGCTTCACTCTCCCCATCT CCCCCCCCTCCCCACCCCCAATTTTGTATTTATTTATTT TTTAATTATTTTGTGCAGCGATGGGGGCGGGGGGGGGG GGGGGGCGCGCGCCAGGCGGGGCGGGGCGGGGCGAG GGGCGGGGCGGGGCGAGGCGGAGAGGTGCGGCGGCA GCCAATCAGAGCGGCGCGCTCCGAAAGTTTCCTTTTAT GGCGAGGCGGCGGCGGCGGCGGCCCTATAAAAAGCGA AGCGCGCGGCGGGCGGGAGTCGCTGCGACGCTGCCTT CGCCCCGTGCCCCGCTCCGCCGCCGCCTCGCGCCGCCC GCCCCGGCTCTGACTGACCGCGTTACTCCCACAGGTGA GCGGGCGGGACGGCCCTTCTCCTCCGGGCTGTAATTAG CTGAGCAAGAGGTAAGGGTTTAAGGGATGGTTGGTTG GTGGGGTATTAATGTTTAATTACCTGGAGCACCTGCCT GAAATCACTTTTTTTCAG |
| 29 | Nucleotide sequence of the CMV promoter | TAGTTATTAATAGTAATCAATTACGGGGTCATTAGTTC ATAGCCCATATATGGAGTTCCGCGTTACATAACTTACG GTAAATGGCCCGCCTGGCTGACCGCCCAACGACCCCCG CCCATTGACGTCAATAATGACGTATGTTCCCATAGTAA CGCCAATAGGGACTTTCCATTGACGTCAATGGGTGGAG TATTTACGGTAAACTGCCCACTTGGCAGTACATCAAGT GTATCATATGCCAAGTACGCCCCCTATTGACGTCAATG ACGGTAAATGGCCCGCCTGGCATTATGCCCAGTACATG ACCTTATGGGACTTTCCTACTTGGCAGTACATCTACGT ATTAGTCATCGCTATTACCATGGTGATGCGGTTTTGGC AGTACATCAATGGGCGTGGATAGCGGTTTGACTCACGG GGATTTCCAAGTCTCCACCCCATTGACGTCAATGGGAG TTTGTTTTGGCACCAAAATCAACGGGACTTTCCAAAAT |
| | | GTCGTAACAACTCCGCCCCATTGACGCAAATGGGCGGT AGGCGTGTACGGTGGGAGGTCTATATAAGCAGAGCT |
| 30 | Nucleotide sequence of the CAGG promoter | TAGTTATTAATAGTAATCAATTACGGGGTCATTAGTTC ATAGCCCATATATGGAGTTCCGCGTTACATAACTTACG GTAAATGGCCCGCCTGGCTGACCGCCCAACGACCCCCG CCCATTGACGTCAATAATGACGTATGTTCCCATAGTAA CGCCAATAGGGACTTTCCATTGACGTCAATGGGTGGAG TATTTACGGTAAACTGCCCACTTGGCAGTACATCAAGT GTATCATATGCCAAGTACGCCCCCTATTGACGTCAATG ACGGTAAATGGCCCGCCTGGCATTATGCCCAGTACATG ACCTTATGGGACTTTCCTACTTGGCAGTACATCTACGT ATTAGTCATCGCTATTACCATGGTCGAGGTGAGCCCCA CGTTCTGCTTCACTCTCCCCATCTCCCCCCCCTCCCCAC CCCCAATTTTGTATTTATTTATTTTTTAATTATTTTGTGC AGCGATGGGGGCGGGGGGGGGGGGGGGGCGCGCGCC AGGCGGGGCGGGGCGGGGCGAGGGGCGGGGCGGGGC GAGGCGGAGAGGTGCGGCGGCAGCCAATCAGAGCGGC GCGCTCCGAAAGTTTCCTTTTATGGCGAGGCGGCGGCG GCGGCGGCCCTATAAAAAGCGAAGCGCGCGGCGGGCG GGGAGTCGCTGCGACGCTGCCTTCGCCCCGTGCCCCGC TCCGCCGCCGCCTCGCGCCGCCCGCCCCGGCTCTGACT GACCGCGTTACTCCCACAGGTGAGCGGGCGGGACGGC CCTTCTCCTCCGGGCTGTAATTAGCGCTTGGTTTAATGA CGGCTTGTTTCTTTTCTGTGGCTGCGTGAAAGCCTTGAG GGGCTCCGGGAGGGCCCTTTGTGCGGGGGGAGCGGCT CGGGGGGTGCGTGCGTGTGTGTGTGCGTGGGGAGCGC CGCGTGCGGCTCCGCGCTGCCCGGCGGCTGTGAGCGCT GCGGGCGCGGCGCGGGGCTTTGTGCGCTCCGCAGTGTG CGCGAGGGGAGCGCGGCCGGGGGCGGTGCCCCGCGGT GCGGGGGGGGCTGCGAGGGGAACAAAGGCTGCGTGCG GGGTGTGTGCGTGGGGGGGTGAGCAGGGGGTGTGGGC |
| | | GCGTCGGTCGGGCTGCAACCCCCCCTGCACCCCCCTCC CCGAGTTGCTGAGCACGGCCCGGCTTCGGGTGCGGGGC TCCGTACGGGGCGTGGCGCGGGGCTCGCCGTGCCGGG CGGGGGGTGGCGGCAGGTGGGGGTGCCGGGCGGGGCG GGGCCGCCTCGGGCCGGGGAGGGCTCGGGGGAGGGGC GCGGCGGCCCCCGGAGCGCCGGCGGCTGTCGAGGCGC GGCGAGCCGCAGCCATTGCCTTTTATGGTAATCGTGCG AGAGGGCGCAGGGACTTCCTTTGTCCCAAATCTGTGCG GAGCCGAAATCTGGGAGGCGCCGCCGCACCCCCTCTA GCGGGCGCGGGGCGAAGCGGTGCGGCGCCGGCAGGAA GGAAATGGGCGGGGAGGGCCTTCGTGCGTCGCCGCGC CGCCGTCCCCTTCTCCCTCTCCAGCCTCGGGGCTGTCCG CGGGGGGACGGCTGCCTTCGGGGGGGACGGGGCAGGG CGGGGTTCGGCTTCTGGCGTGTGACCGGCGGCTCTAGA GCCTCTGCTAACCATGTTCATGCCTTCTTCTTTTTCCTA CAG |
| 31 | Nucleotide sequence of the CAG promoter | CCATTGACGTCAATAATGACGTATGTTCCCATAGTAAC GCCAATAGGGACTTTCCATTGACGTCAATGGGTGGAGT ATTTACGGTAAACTGCCCACTTGGCAGTACATCAAGTG TATCATATGCCAAGTACGCCCCCTATTGACGTCAATGA CGGTAAATGGCCCGCCTGGCATTATGCCCAGTACATGA CCTTATGGGACTTTCCTACTTGGCAGTACATCTACGTAT TAGTCATCGCTATTACCATGGTCGAGGTGAGCCCCACG TTCTGCTTCACTCTCCCCATCTCCCCCCCCTCCCCACCC CCAATTTTGTATTTATTTATTTTTTAATTATTTTGTGCAG CGATGGGGGCGGGGGGGGGGGGGGGGCGCGCGCCAG GCGGGGCGGGGCGGGGCGAGGGGCGGGGCGGGGCGA GGCGGAGAGGTGCGGCGGCAGCCAATCAGAGCGGCGC GCTCCGAAAGTTTCCTTTTATGGCGAGGCGGCGGCGGC GGCGGCCCTATAAAAAGCGAAGCGCGCGGCGGGCGGG AGTCGCTGCGCGCTGCCTTCGCCCCGTGCCCCGCTCCG |
| | | CCGCCGCCTCGCGCCGCCCGCCCCGGCTCTGACTGACC GCGTTACTCCCACAGGTGAGCGGGCGGGACGGCCCTTC TCCTCCGGGCTGTAATTAGCGCTTGGTTTAATGACGGC TTGTTTCTTTTCTGTGGCTGCGTGAAAGCCTTGAGGGGC TCCGGGAGGGCCCTTTGTGCGGGGGGAGCGGCTCGGG GCTGTCCGCGGGGGGACGGCTGCCTTCGGGGGGGACG GGGCAGGGCGGGGTTCGGCTTCTGGCGTGTGACCGGC GGCTCTAGAGCCTCTGCTAACCATGTTCATGCCTTCTTC TTTTTCCTACAGCTCCTGGGCAACGT |
| 32 | Nucleotide sequence of the RPE65 promoter | AGATCTTCGAAATACTCTCAGAGTGCCAAACATATACC AATGGACAAGAAGGTGAGGCAGAGAGCAGACAGGCAT TAGTGACAAGCAAAGATATGCAGAATTTCATTCTCAGC AAATCAAAAGTCCTCAACCTGGTTGGAAGAATATTGGC ACTGAATGGTATCAATAAGGTTGCTAGAGAGGGTTAG AGGTGCACAATGTGCTTCCATAACATTTTATACTTCTCC AATCTTAGCACTAATCAAACATGGTTGAATACTTTGTT TACTATAACTCTTACAGAGTTATAAGATCTGTGAAGAC AGGGACAGGGACAATACCCATCTCTGTCTGGTTCATAG GTGGTATGTAATAGATATTTTTAAAAATAAGTGAGTTA ATGAATGAGGGTGAGAATGAAGGCACAGAGGTATTAG GGGGAGGTGGGCCCCAGAGAATGGTGCCAAGGTCCAG TGGGGTGACTGGGATCAGCTCAGGCCTGACGCTGGCCA CTCCCACCTAGCTCCTTTCTTTCTAATCTGTTCTCATTCT CCTTGGGAAGGATTGAGGTCTCTGGAAAACAGCCAAA CAACTGTTATGGGAACAGCAAGCCCAAATAAAGCCAA GCATCAGGGGGATCTGAGAGCTGAAAGCAACTTCTGTT CCCCCTCCCTCAGCTGAAGGGGTGGGGAAGGGCTCCCA AAAAAGCCATAACTCCTTTTAAGGGATTTAGAAGGCAT AAAAAGGCCCCTGGCTGAGAACTTCCTTCTTCATTCTG CAGTTGG |
| 33 | Nucleotide sequence of the VMD2 promoter | GAATTCTGTCATTTTACTAGGGTGATGAAATTCCCAAG |
| | | CAACACCATCCTTTTCAGATAAGGGCACTGAGGCTGAG AGAGGAGCTGAAACCTACCCGGGGTCACCACACACAG GTGGCAAGGCTGGGACCAGAAACCAGGACTGTTGACT GCAGCCCGGTATTCATTCTTTCCATAGCCCACAGGGCT GTCAAAGACCCCAGGGCCTAGTCAGAGGCTCCTCCTTC CTGGAGAGTTCCTGGCACAGAAGTTGAAGCTCAGCAC AGCCCCCTAACCCCCAACTCTCTCTGCAAGGCCTCAGG GGTCAGAACACTGGTGGAGCAGATCCTTTAGCCTCTGG ATTTTAGGGCCATGGTAGAGGGGGTGTTGCCCTAAATT CCAGCCCTGGTCTCAGCCCAACACCCTCCAAGAAGAAA TTAGAGGGGCCATGGCCAGGCTGTGCTAGCCGTTGCTT CTGAGCAGATTACAAGAAGGGACTAAGACAAGGACTC CTTTGTGGAGGTCCTGGCTTAGGGAGTCAAGTGACGGC GGCTCAGCACTCACGTGGGCAGTGCCAGCCTCTAAGAG TGGGCAGGGGCACTGGCCACAGAGTCCCAGGGAGTCC CACCAGCCTAGTCGCCAGACC |
| 34 | Amino acid sequence of Fc | LGGPSVFLFPPKPKDTLMISRTPEVTCVVVDVSHEDPEVK FNWYVDGVEVHNAKTKPREEQYNSTYRVVSVLTVLHQD WLNGKEYKCKVSNKALPAPIEKTISKAKGQPREPQVYTL PPSRDELTKNQVSLTCLVKGFYPSDIAVEWESNGQPENNY KTTPPVLDSDGSFFLYSKLTVDKSRWQQGNVFSCSVMHE ALHNHYTQKSLSLSPG |
| 35 | Nucleotide sequence of Fc | CTGGGAGGACCAAGCGTGTTCCTGTTTCCACCCAAGCC CAAGGATACCCTGATGATCTCCAGGACCCCAGAGGTG ACATGCGTGGTGGTGGACGTGTCTCACGAGGATCCTGA GGTGAAGTTTAACTGGTACGTGGACGGCGTGGAGGTG CACAATGCCAAGACCAAGCCTCGGGAGGAGCAGTACA ATTCCACCTATAGAGTGGTGTCTGTGCTGACAGTGCTG CACCAGGATTGGCTGAACGGCAAGGAGTATAAGTGCA AGGTGAGCAATAAGGCCCTGCCTGCCCCAATCGAGAA GACCATCTCCAAGGCAAAGGGACAGCCAAGGGAGCCT |
| | | CAGGTGTACACACTGCCTCCATCTCGCGACGAGCTGAC CAAGAACCAGGTGAGCCTGACATGTCTGGTGAAGGGC TTCTATCCTTCCGATATCGCCGTGGAGTGGGAGTCTAA TGGCCAGCCAGAGAACAATTACAAGACCACACCCCCT GTGCTGGACAGCGATGGCTCCTTCTTTCTGTATTCTAAG CTGACCGTGGATAAGAGCCGGTGGCAGCAGGGCAACG TGTTTTCCTGCTCTGTGATGCACGAGGCCCTGCACAAT CACTACACCCAGAAGAGCCTGTCCCTGTCTCCCGGC |
| 36 | LTR Forward primer | CTGTTGTGTGACTCTGGTAACT |
| 37 | LTR Probe | AAATCTCTAGCAGTGGCGCCCG |
| 38 | LTR Reverse primer | TTCGCTTTCAAGTCCCTGTT |
| 39 | HK Forward primer | GCTGTCATCTCTTGTGGGCTGT |
| 40 | HK Probe | CCTGTCATGCCCACACAAATCTCTCC |
| 41 | HK Reverse primer | ACTCATGGGAGCTGCTGGTTC |
| 42 | ITR Forward primer | GGAACCCCTAGTGATGGAGTT |
| 43 | ITR Probe | CACTCCCTCTCTGCGCGC |
| 44 | ITR Reverse primer | CGGCCTCAGTGAGCGA |
| 45 | Amino acid sequence of N-terminal signal sequence | MDAMKRGLCCVLLLCGAVFVSP |
| 46 | Nucleotide sequence encoding N-terminal signal sequence | ATGGACGCCATGAAAAGAGGCCTGTGCTGCGTGCTGCT GCTGTGCGGCGCCGTGTTCGTGTCCCCT |
| 47 | Amino acid sequence of the Aflibercept fusion protein | MYRMQLLSCIALSLALVTNSSDTGRPFVEMYSEIPEIIHMT EGRELVIPCRVTSPNITVTLKKFPLDTLIPDGKRIIWDSRKG FIISNATYKEIGLLTCEATVNGHLYKTNYLTHRQTNTIIDV VLSPSHGIELSVGEKLVLNCTARTELNVGIDFNWEYPSSK HQHKKLVNRDLKTQSGSEMKKFLSTLTIDGVTRSDQGLY TCAASSGLMTKKNSTFVRVHEKDKTHTCPPCPAPELLGG PSVFLFPPKPKDTLMISRTPEVTCVVVDVSHEDPEVKFNW YVDGVEVHNAKTKPREEQYNSTYRVVSVLTVLHQDWLN GKEYKCKVSNKALPAPIEKTISKAKGQPREPQVYTLPPSR DELTKNQVSLTCLVKGFYPSDIAVEWESNGQPENNYKTT PPVLDSDGSFFLYSKLTVDKSRWQQGNVFSCSVMHEALH NHYTQKSLSLSPG |
| 48 | Nucleotide sequence encoding the Aflibercept fusion protein | ATGTACCGGATGCAGCTGCTGTCTTGCATCGCCCTGAG CCTGGCCCTGGTGACCAACAGCTCCGACACAGGCAGA CCCTTCGTGGAGATGTATTCTGAGATCCCTGAGATCAT CCACATGACCGAGGGCAGGGAGCTGGTCATCCCATGTC GCGTGACAAGCCCCAACATCACCGTGACACTGAAGAA GTTCCCTCTGGACACCCTGATCCCAGATGGCAAGCGGA TCATCTGGGACAGCAGAAAGGGCTTTATCATCTCCAAT GCCACATACAAGGAGATCGGCCTGCTGACCTGCGAGG CCACAGTGAACGGCCACCTGTACAAGACCAATTATCTG ACACACAGACAGACCAACACAATCATCGATGTGGTGC TGAGCCCATCCCACGGCATCGAGCTGTCCGTGGGCGAG AAGCTGGTGCTGAATTGTACCGCCAGGACAGAGCTGA ACGTGGGCATCGACTTCAATTGGGAGTACCCCTCTAGC AAGCACCAGCACAAGAAGCTGGTGAACAGGGATCTGA AGACCCAGTCTGGCAGCGAGATGAAGAAGTTTCTGAG CACCCTGACAATCGACGGAGTGACCCGCTCCGATCAGG GACTGTATACATGCGCCGCCTCCTCTGGCCTGATGACC AAGAAGAATTCCACATTCGTGCGGGTGCACGAGAAGG |
| | | ACAAGACCCACACATGCCCACCTTGTCCAGCACCAGAG CTGCTGGGAGGACCAAGCGTGTTCCTGTTTCCACCCAA GCCCAAGGATACCCTGATGATCTCCAGGACCCCAGAG GTGACATGCGTGGTGGTGGACGTGTCTCACGAGGATCC TGAGGTGAAGTTTAACTGGTACGTGGACGGCGTGGAG GTGCACAATGCCAAGACCAAGCCTCGGGAGGAGCAGT ACAATTCCACCTATAGAGTGGTGTCTGTGCTGACAGTG CTGCACCAGGATTGGCTGAACGGCAAGGAGTATAAGT GCAAGGTGAGCAATAAGGCCCTGCCTGCCCCAATCGA GAAGACCATCTCCAAGGCAAAGGGACAGCCAAGGGAG CCTCAGGTGTACACACTGCCTCCATCTCGCGACGAGCT GACCAAGAACCAGGTGAGCCTGACATGTCTGGTGAAG GGCTTCTATCCTTCCGATATCGCCGTGGAGTGGGAGTC TAATGGCCAGCCAGAGAACAATTACAAGACCACACCC CCTGTGCTGGACAGCGATGGCTCCTTCTTTCTGTATTCT AAGCTGACCGTGGATAAGAGCCGGTGGCAGCAGGGCA ACGTGTTTTCCTGCTCTGTGATGCACGAGGCCCTGCAC AATCACTACACCCAGAAGAGCCTGTCCCTGTCTCCCGG CTAG |

## Claims

1. A nucleic acid construct such as an expression vector, wherein the nucleic acid construct comprises one or more nucleotide sequence(s) encoding a VEGF-binding region, such as a first nucleotide sequence encoding a first VEGF-binding region and/or a second nucleotide sequence encoding a second VEGF-binding region, wherein the first VEGF-binding region and/or the second VEGF-binding region are each independently selected from an antigen-binding fragment derived from an anti-VEGF antibody such as scFv and a VEGF-binding fragment derived from a VEGF receptor (VEGFR) which comprises domain 2 and domain 3 (D2/3) of the VEGFR, wherein the first nucleotide sequence and the second nucleotide sequence are connected by a nucleotide sequence encoding a linker.

2. The nucleic acid construct of claim 1, wherein:
the first VEGF-binding region is the antigen-binding fragment derived from the anti-VEGF antibody, and the second VEGF-binding region is an antigen-binding fragment derived from another different anti-VEGF antibody; or
the first VEGF-binding region is the VEGF-binding fragment derived from the VEGFR, and the second VEGF-binding region is the antigen-binding fragment derived from the anti-VEGF antibody.

3. The nucleic acid construct of claim 1, wherein the nucleic acid construct further comprises a third nucleotide sequence encoding a third VEGF-binding region, wherein the third VEGF-binding region is selected from the antigen-binding fragment derived from the anti-VEGF antibody and/or the VEGF-binding fragment derived from the VEGFR which comprises the domain 2 and domain 3 (D2/3) of the VEGFR , wherein the first nucleotide sequence, the second nucleotide sequence and the third nucleotide sequence are connected to each other by a nucleotide sequence encoding a linker.

4. The nucleic acid construct of claim 3, wherein:
the first VEGF-binding region is the antigen-binding fragment derived from the anti-VEGF antibody, the second VEGF-binding region is the antigen-binding fragment derived from another different anti-VEGF antibody, and the third VEGF-binding region is the VEGF-binding fragment derived from the VEGFR.

5. The nucleic acid construct of any one of claims 1-4, wherein the nucleic acid construct comprises, from the 5' end to the 3' end, the following structures:
(1) scFv1;
(2) D2/3;
(3) D2/3-linker-scFv1;
(4) scFv1-linker-scFv2;
(5) scFv1-linker-D2/3;
(6) scFv1-linker-scFv2-linker-D2/3;
(7) scFv1-linker-D2/3-linker-scFv2; or
(8) D2/3-linker-scFv1-linker-scFv2,
wherein, the scFv1 is a nucleotide sequence encoding the scFv derived from the anti-VEGF antibody,
the scFv2 is a nucleotide sequence encoding the scFv derived from another different anti-VEGF antibody,
the D2/3 is a nucleotide sequence encoding the VEGF-binding fragment derived from the VEGFR which comprises the D2/3 of the VEGFR,
the linker is a nucleotide sequence encoding a linker, wherein each of the linkers may be same or different.

6. The nucleic acid construct of any one of claims 1-4, wherein the nucleic acid construct further comprises a nucleotide sequence encoding the Fc region of an antibody, preferably the Fc region of human IgG1.

7. The nucleic acid construct of claim 6, wherein the nucleic acid construct comprises, from the 5' end to the 3' end, the following structures:
(1) scFv1-linker-Fc;
(2) D2/3-linker-Fc;
(3) D2/3-linker-scFv1-Fc;
(4) scFv1-linker-D2/3-Fc;
(5) scFv1-linker-scFv2-Fc;
(6) scFv1-linker-scFv2-linker-D2/3-Fc;
(7) scFv1-linker-D2/3-linker-scFv2-Fc; or
(8) D2/3-linker-scFv1-linker-scFv2-Fc,
wherein, the scFv1 is a nucleotide sequence encoding the scFv derived from the anti-VEGF antibody,
the scFv2 is a nucleotide sequence encoding the scFv derived from another different anti-VEGF antibody,
the D2/3 is a nucleotide sequence encoding the VEGF-binding fragment derived from the VEGFR which comprises the D2/3 of the VEGFR,
the linker is a nucleotide sequence encoding a linker, wherein each of the linkers may be same or different,
the Fc is a nucleotide sequence encoding the Fc region of the antibody.

8. The nucleic acid construct of any one of claims 1-7, wherein the anti-VEGF antibody is selected from Brolucizumab, Ranibizumab or Aflibercept;
preferably, the scFv derived from Brolucizumab has the amino acid sequence as set forthin SEQ ID NO: 1, the scFv derived from Ranibizumab has the amino acid sequence as set forth in SEQ ID NO: 3, and/or the scFv derived from Aflibercept has the amino acid sequence as set forth in SEQ ID NO: 47.

9. The nucleic acid construct of claim 8, wherein the nucleic acid construct comprises, from the 5' end to the 3' end, the following structures:
(1) D2/3;
(2) D2/3-Fc;
(3) BrolscFv;
(4) BrolscFv-Fc;
(5) RaniScFv;
(6) RaniScFv-Fc;
(7) D2/3-linker-RaniScFv;
(8) RaniScFv-linker-D2/3;
(9) BrolscFv-linker-RaniScFv-Fc;
(10) BrolscFv-linker-RaniScFv;
(11) RaniScFv-linker-BrolscFv;
(12) RaniScFv-linker-BrolscFv-Fc;
(13) D2/3-linker-RaniScFv-Fc;
(14) RaniScFv-linker-D2/3-Fc;
(15) D2/3-linker-BrolscFv-linker-RaniScFv-Fc;
(16) D2/3-linker-BrolscFv-linker-RaniScFv;
(17) BrolscFv-linker-D2/3-linker-RaniScFv;
(18) BrolscFv-linker-D2/3-linker-RaniScFv-Fc;
(19) RaniScFv-linker-BrolscFv-linker-D2/3;
(20) RaniScFv-linker-BrolscFv-linker-D2/3-Fc;
(21) BrolscFv-linker-D2/3;
(22) BrolscFv-linker-D2/3-Fc;
(23) D2/3-linker-BrolscFv; or
(24) BrolscFv-linker-D2/3,
wherein, the BrolscFv is a nucleotide sequence encoding the scFv derived from Brolucizumab,
the RaniScFv is a nucleotide sequence encoding the scFv derived from Ranibizumab.
the D2/3 is a nucleotide sequence encoding the VEGF-binding fragment derived from the VEGFR which comprises the D2/3 of the VEGFR,
the linker is a nucleotide sequence encoding a linker, wherein each of the linkers may be same or different,
the Fc is a nucleotide sequence encoding the Fc region of the antibody.

10. The claim of any one of claims 1-9, wherein the VEGF-binding fragment derived from the VEGFR has the amino acid sequence as set forth in SEQ ID NO: 5.

11. The nucleic acid construct of any one of claims 1-10, wherein the linker is non-cleavable linker.

12. The nucleic acid construct of claim 11, wherein the linker each independently is (G₁S)ₙ, (G₂S)ₙ, (G₃S)ₙ or (G₄S)ₙ, wherein n is from 1 to 10, preferably n is from 2 to 8, more preferably n is 4 or 5.

13. The nucleic acid construct of any one of claims 1-12, further comprising a nucleotide sequence encoding an N-terminal signal sequence at the 5' end of the coding sequence, preferably, the N-terminal signal sequence has the amino acid sequence as set forth in SEQ ID NO: 45.

14. The nucleic acid construct of any one of claims 1-13, wherein the nucleic acid construct encodes a fusion protein comprising the amino acid sequence selected from SEQ ID NO: 13, SEQ ID NO: 7, SEQ ID NO: 9, SEQ ID NO: 11, SEQ ID NO: 15, SEQ ID NO: 17, SEQ ID NO: 19 and SEQ ID NO: 21.

15. The nucleic acid construct of any one of claims 1-14, wherein the nucleic acid construct comprises the nucleotide sequence selected from SEQ ID NO: 14, SEQ ID NO: 8, SEQ ID NO: 10, SEQ ID NO: 12, SEQ ID NO: 16, SEQ ID NO: 18, SEQ ID NO: 20 and SEQ ID NO: 22.

16. The nucleic acid construct of any one of claims 1-15, wherein the nucleic acid construct further comprises a promoter sequence for the expression of the nucleotide sequence, preferably the promoter is a CAGG promoter.

17. The nucleic acid construct of any one of claims 1-16, wherein the nucleic acid construct is a viral vector.

18. The nucleic acid construct of claim 17, wherein the nucleic acid construct is a lentiviral vector.

19. The nucleic acid construct of claim 18, wherein the lentiviral vector further comprises one or more elements selected from a chimeric LTR promoter, an HIV-1 packaging signal (ψ), a central polypurine tract (cPPT), a Rev response element (RRE), a polypurine tract (PPT), a woodchuck hepatitis B virus post-transcriptional regulatory element (WPRE), a polyadenylation signal of SV40 virus (SV40 pA signal), a replication origin of SV40 virus (SV40 ori), and a self-inactivating long terminal repeats.

20. The nucleic acid construct of claim 17, wherein the nucleic acid construct is an adeno-associated viral vector.

21. The nucleic acid construct according to claim 20, wherein the adeno-associated viral vector further comprises one or more elements selected from a polyadenylation signal of SV40 virus (SV40SL), a woodchuck hepatitis B virus post-transcriptional regulatory element (WPRE) and an inverted terminal repeats (ITR).

22. A viral particle comprising the nucleic acid construct of any one of claims 1-21, preferably the viral particle is a lentiviral particle or an adeno-associated viral particle.

23. A cell comprising the nucleic acid construct according to any one of claims 1-21.

24. The cell of claim 23, wherein the nucleic acid construct is an adeno-associated viral vector, wherein the cell further comprises a serotyped plasmid and/or a helper plasmid for assembling the nucleic acid construct into the viral particle.

25. The cell of claim 23, wherein the nucleic acid construct is a lentiviral vector, wherein the cell further comprises an envelope plasmid and/or a packaging plasmid for assembling the nucleic acid construct into the viral particle.

26. A vector system comprising the nucleic acid construct of claims 1-21, and optionally one or more additional vectors such as plasmids.

27. The vector system of claim 26, wherein the nucleic acid construct is an adeno-associated viral vector, wherein the vector system further comprises a serotyped plasmid and/or a helper plasmid for assembling the nucleic acid construct into the viral particle.

28. The vector system of claim 27, wherein the nucleic acid construct is a lentiviral vector, wherein the vector system further comprising an envelope plasmid and/or a packaging plasmid for assembling the nucleic acid construct into the viral particle.

29. The vector system according to any one of claims 26-28, further comprising a host cell, preferably the host cell is a 293T cell.

30. A composition comprising the nucleic acid construct according to any one of claims 1-21, the viral particle according to claim 22, the cell according to any one of claims 23-25 or the vector system according to any one of claims 26-29, and optionally a pharmaceutically acceptable carrier and/or excipient.

31. A system for intraocular delivery comprising:
a) the nucleic acid construct according to any one of claims 1-21, the viral particle according to claim 22, the cell according to any one of claims 23-25, the vector system according to any one of claims 26-29 or the composition according to claim 30; and
b) a device for intraocular delivery.

32. The system according to claim 31, wherein the device for intraocular delivery is selected from a device for intravitreal delivery, a device for subretinal cavity delivery and a device for suprachoroidal cavity delivery.

33. A method for preventing, ameliorating or treating age-related macular degeneration in a subject, which comprises the step of administering into the eye of the subject the nucleic acid construct according to any one of claims 1-21, the viral particle according to claim 22, the cell according to any one of claims 23-25, the vector system according to any one of claims 26-29 or the composition according to claim 30.

34. The method according to claim 33, wherein the age-related macular degeneration is wet macular degeneration.

35. The method according to claim 33 or 34, wherein the nucleic acid construct, the viral particle, the cell, the vector system, or the composition are administered to the intravitreal and/or subretinal cavity and/or suprachoroidal cavity of the subject.
